# EUROPÄISCHE PATENTSCHRIFT

(11) **EP 1 871 242 B1**
(45) Veröffentlichungstag und Bekanntmachung des Hinweises auf die Patenterteilung: **27.09.2017**
(21) Anmeldenummer: 05735007.6
(22) Anmeldetag: 16.04.2005
(51) Int. Cl.: A61B 90/00, A61B 17/16

(54) **CHIRURGISCHE MASCHINE UND VERFAHREN ZUM STEUERN UND/ODER REGELN EINER CHIRURGISCHEN MASCHINE**
SURGICAL MACHINE AND METHOD FOR CONTROLLING AND/OR REGULATING THE SAME
MACHINE CHIRURGICALE ET PROCEDE DE COMMANDE ET/OU DE REGULATION DE CELLE-CI

(43) Veröffentlichungstag der Anmeldung: 02.01.2008
(73) Patentinhaber: Aesculap AG, 78532 Tuttlingen (DE)
(72) Erfinder: HÖGERLE, Roland, Alois, 78432 Tuttlingen (DE); KAHLER, Thomas, 78606 Seitingen-Oberflacht (DE); KLEINWÄCHTER, Timo, 78532 Tuttlingen (DE); MACHILL, Martin, 78604 Rietheim-Weilheim (DE); SCHAZ, Uwe, 78579 Neuhausen (DE); SCHNEIDER, Jürgen, 78532 Tuttlingen (DE); KONRATH, Harald, 72108 Rottenburg-Hailfingen (DE)
(74) Vertreter: Hoeger, Stellrecht & Partner Patentanwälte mbB
(86) Internationale Anmeldenummer: PCT/EP2005/004059
(87) Internationale Veröffentlichungsnummer: WO 2006/111173

(56) Entgegenhaltungen:
- DE-U1- 8 000 592
- DE-U1- 20 202 724
- US-A- 2 244 683
- US-A- 4 091 880
- US-A- 6 013 991
- US-A- 6 059 806

## Beschreibung

Die vorliegende Erfindung betrifft eine chirurgische Maschine mit einem eine Längsachse definierenden Motorträger umfassenden Gehäuse, einem im wesentlichem quer vom Motorträger abstehenden Griff und einem chirurgischen Antrieb, wobei der Antrieb und das Gehäuse direkt oder indirekt lösbar verbindbar sind, wobei der Griff einstückig an den Motorträger angeformt ist, wobei der Motorträger und der Griff stirnseitig eine schlüssellochartig geformte Öffnung im Übergangsbereich vom Motorträger zum Griff aufweisen, welche mit einer als Rahmen dienenden, im wesentlichen schlüssellochartig geformten Platte verschlossen ist, wobei der Antrieb in den im wesentlichen hohlzylindrischen Motorträger von vorne kommend, parallel zur Längsachse eingeführt ist und wobei der Antrieb ein Elektromotor ist.

Ferner wird nachfolgend ein Verfahren zum Steuern und/oder Regeln einer chirurgischen Maschine mit einem Antrieb, vorzugsweise in Form eines Elektromotors, welcher einen Rotor und mindestens zwei Motorwicklungen aufweist, und mit einer Motorsteuerung zum Steuern und/oder Regeln des Antriebs, beschrieben.

Maschinen der eingangs beschriebenen Art kommen in unterschiedlichen Varianten und Ausführungsformen in der Chirurgie zum Einsatz. Beispiele derartiger Maschinen sind Bohrmaschinen, Fräsmaschinen sowie Sägen, insbesondere Stichsägen und oszillierende Sägen. Sowohl der Aufbau derartiger Maschinen als auch ihr Betrieb sind teilweise sehr aufwendig. Auch gibt es immer wieder Probleme bei der Wartung und Reinigung der Maschinen.

Eine Maschine der eingangs beschriebenen Art ist beispielsweise aus der US 6,013,991 bekannt. Ein K-Draht-Applikationsgerät ist ferner in der US 4,091,880 beschrieben. In der US 2,244,683 ist eine chirurgische Säge offenbart.

Es ist daher Aufgabe der vorliegenden Erfindung, eine Maschine der eingangs beschriebenen Art so zu verbessern, daß sich deren Aufbau insgesamt vereinfacht.

Diese Aufgabe wird bei einer chirurgischen Maschine der eingangs beschrieben Art erfindungsgemäß dadurch gelöst, dass der Elektromotor ein sensorloser Motor ist. Die erfindungsgemäße chirurgische Maschine ist im Anspruch 1 definiert.

Die chirurgische Maschine läßt sich so besonders einfach zerlegen, beispielsweise durch Trennen des Antriebs und des Gehäuses. Der Antrieb kann direkt mit dem Gehäuse verbunden werden, beispielsweise mit entsprechenden Befestigungsmitteln oder indirekt, beispielsweise durch Klemmen zwischen zwei direkt mit dem Gehäuse verbindbaren Elementen. So läßt sich der Antrieb vom Gehäuse auf einfache Weise lösen, beispielsweise zu Reinigungszwecken oder um den Motor auszuwechseln, insbesondere infolge eines Defekt oder zu Wartungszwecken. Der Antrieb der Maschine ist besonders robust ausgeführt, weil der Antrieb ein Elektromotor ist. Zudem kann die Maschine mit einer entsprechenden Energieversorgung so auch netzunabhängig eingesetzt werden. Grundsätzlich wäre es denkbar, einen Elektromotor zu verwenden, der ein Hall-System zur Drehzahlerfassung aufweist. Allerdings wären dann zusätzliche Kontakte erforderlich um die Motorsteuerung mit Hall-Sensoren des Hall-Systems zur Drehzahlerfassung zu verbinden. Daher ist es besonders günstig, dass der Elektromotor ein sensorloser Motor ist. Eine Erfassung der Motordrehzahl kann dann beispielsweise über die Bestimmung einer Gegen-EMK (Elektro-Motorische-Kraft) ermittelt werden. Unter einem sensorlosen Elektromotor ist insbesondere zu verstehen, daß keine Drehzahlerfassungssensoren zur Bestimmung einer Ist-Drehzahl des Elektromotors vorgesehen oder am Elektromotor angeordnet sind. Derartige Elektromotoren sind wesentlich kostengünstiger als Motoren, die Sensoren umfassen, und zudem vereinfacht sich der Aufbau der chirurgischen Maschine insgesamt. Grund hierfür ist, daß weniger Anschlüsse für den Motor vorgesehen werden müssen. Dies hat zudem den Vorteil, daß bei einer zerlegbaren chirurgischen Maschine keine Korrosionsprobleme bei Kontakten auftreten können, über welche eine Verbindung der Motorsteuerung zu Drehzahlerfassungs- und/oder Positionssensoren hergestellt werden kann. Derartige Kontakte werden üblicherweise mit kleinen Spannungen beziehungsweise Strömen beaufschlagt, so daß schon eine geringe Korrosion der Kontakte zu Fehlern bei der Ermittlung der Ist-Drehzahl des Elektromotors führen kann. Genau dies kann bei einer vorgeschlagenen Maschine nicht passieren.

Vorteilhaft ist es, wenn das Gehäuse mindestens zwei Öffnungen aufweist. Beispielsweise kann durch eine Öffnung des Gehäuses der Antrieb eingeführt werden und durch eine andere oder dieselbe Öffnung eine Energieversorgungseinheit der Maschine. Es können jedoch auch mehr als zwei Öffnungen vorgesehen sein, beispielsweise drei oder vier, um weitere Baugruppen oder Bauelemente auf einfache Weise in das Gehäuse zu Montage- oder Wartungszwecken ein- und/oder ausführen zu können.

Vorzugsweise umfaßt die Maschine eine in das Gehäuse einführbare Energieversorgungseinheit. Dadurch wird es möglich, die Maschine netzunabhängig zu betreiben und damit auf bei einem Netzbetrieb erforderliche Kabelverbindungen ganz zu verzichten.

Günstigerweise ist die Maschine ohne die Energieversorgungseinheit vollständig wasch- und/oder sterilisierbar. Dadurch kann zum einen sichergestellt werden, daß die Maschine auch in sterilen Bereichen, wie zum Beispiel Operationssälen, eingesetzt werden kann, ohne daß die Gefahr besteht, daß Keime in den sterilen Bereich gelangen können. Zum anderen wird so auch vermieden, daß die Maschine beim Reinigen, insbesondere in einer Wasch- oder Spülmaschine, beschädigt werden kann.

Grundsätzlich wäre es denkbar, eine netzabhängige Gleichstromspannungsquelle als Energieversorgungeinheit für die chirurgische Maschine zu wählen. Besonders vorteilhaft ist es jedoch, wenn eine netzunabhängige Energieversorgungseinheit zur Energieversorgung der Maschine vorgesehen ist. Insbesondere vorteilhaft ist der Einsatz einer Batterie oder eines wiederaufladbaren Akkumulators. Denkbar wäre auch die Verwendung einer Brennstoffzelle. Die Maschine läßt sich so ohne störende Kabelverbindungen in gewünschter Weise bei chirurgischen Eingriffen verwenden.

Um den Antrieb mit einer gewünschten Drehzahl oder Drehrichtung betreiben zu können, ist es vorteilhaft, wenn die Maschine eine Steuer- und/oder Regelungseinheit zum Steuern und/oder Regeln des Antriebs umfaßt.

Vorzugsweise ist die Steuer- und/oder Regelungseinheit eine Motorsteuerung. Diese kann beispielsweise im Gehäuse oder direkt am Antrieb angeordnet sein.

Gemäß einer weiteren bevorzugten Ausführungsform der Erfindung kann vorgesehen sein, daß die Energieversorgungseinheit und die Steuer- und/oder Regelungseinheit eine als Ganzes in das Gehäuse einführbare Energie- und Steuereinheit bilden. Auf diese Weise ist es möglich, die chirurgische Maschine von der Energieversorgung und der Steuer- und/oder Regelungseinheit getrennt zu reinigen. Eine Energie- und Steuereinheit vorzusehen erleichtert zudem das Zusammenfügen und Vorbereiten der Maschine für einen chirurgischen Einsatz.

Vorzugsweise bildet ein Teil des Gehäuses einen Aufnahmeraum für den Antrieb. Vorteilhaft ist es ferner, wenn ein Teil des Gehäuses einen Handgriff der Maschine bildet und wenn die Energie- und Steuereinheit in den Handgriff einführbar ist. Diese Ausgestaltung gestattet eine besonders gute Handhabung der Maschine, da die in der Regel schwere Energieversorgungseinheit dann direkt in der Hand einer Bedienperson liegt. Zudem kann die Energie- und Steuereinheit auf einfache Weise ausgetauscht werden.

Vorteilhaft ist es, wenn die Energie- und Steuereinheit Ladekontakte zum Verbinden der Energieversorgungseinheit mit einem Ladegerät und Verbindungskontakte zum Verbinden mit dem Antrieb aufweist. Dies ermöglicht es prinzipiell, die mit dem Antrieb verbundene Energie- und Steuereinheit, ohne sie vom Antrieb zu trennen, aufzuladen. Außerdem wird eine Beschädigung der mit den Verbindungskontakten verbundenen Motorsteuerung verhindert. Bei herkömmlichen Energieversorgungseinheiten die ohne integrierte Motorsteuerung angeboten werden, also nicht als Energie- und Steuereinheit, können die Verbindungskontakte im Prinzip auch als Ladekontakte dienen. Bei der vorgeschlagenen Ausführungsform sind zwar zusätzliche Ladekontakte erforderlich, doch ist durch das Vorsehen derselben insgesamt eine einfachere Handhabung der Maschine möglich.

Für ein einfaches und schnelles Laden der Energieversorgungseinheit ist es günstig, wenn vier Ladekontakte vorgesehen sind.

Um die Zahl der Motorkontakte zu minimieren, ist es vorteilhaft, wenn der Antrieb mindestens zwei Motorwicklungen und mindestens zwei mit jeweils zwei Motorwicklungen verbundene Motorkontakte aufweist.

Vorzugsweise umfaßt der Antrieb drei Motorwicklungen und drei Motorkontakte. Insbesondere kann jeder Motorkontakt mit zwei über diesen verbundene Motorwicklungen verbunden sein. Vorteilhafterweise stehen die Motorkontakte in Form parallel zueinander abstehender Kontaktstifte vom Antrieb ab. Dadurch können die Motorkontakte auf einfache Weise mit den Verbindungskontakten der Energie- und Steuereinheit verbunden werden, beispielsweise durch einfaches Zusammenschieben.

Um die Stabilität der Motorkontakte zu erhöhen, ist es günstig, wenn die Kontaktstifte massiv ausgebildet sind. Zudem wird so eine Korrosionsgefahr der Motorkontakte minimiert. Im Vergleich zu aus einer Vielzahl von Federelementen bestehenden Kontaktstiften kann ein massiver Kontaktstift oder aber auch ein nach außen verschlossener, hülsenartiger Kontaktstift besser gereinigt werden, da dieser keine Zwischen- oder Hohlräume aufweist, in denen sich Keime ablagern können. Des weiteren können sowohl die Motorkontakte als auch die Ladekontakte vergoldet sein, wodurch eine Korrosionsbeständigkeit der Kontakte verbessert wird und dadurch auch nach häufigem Reinigen, insbesondere Sterilisieren, der Maschinen stets ein einwandfreier Kontakt zwischen dem Antrieb und der Energie- und Steuereinheit hergestellt werden kann.

Um eine sichere Verbindung zwischen dem Antrieb und der Energie- und Steuereinheit erreichen zu können, ist es günstig, wenn die Energie- und Steuereinheit zu den Kontaktstiften korrespondierende Kontaktbuchsen aufweist. Durch einfaches Zusammenschieben kann so die Energie- und Steuereinheit mit dem Antrieb verbunden werden.

Im Falle von massiv oder in Form verschlossener Hülsen ausgebildeter Kontaktstifte ist es besonders günstig, wenn die Kontaktbuchsen im wesentlichen hohlzylindrisch ausgebildet sind und federnde Wandabschnitte zum klemmenden Halten der Kontaktstifte aufweisen. Durch diese Ausgestaltung kann auch nach häufigem Wechseln der Energie- und Steuereinheit ein sicherer Kontakt zwischen den Kontaktbuchsen und den Motorkontakten erreicht werden. Obwohl es nicht Teil der Erfindung ist, wäre es grundsätzlich denkbar, daß die Energie- und Steuereinheit eine Drehzahlvorgabeeinheit mit einem Drehzahlvorgabesensor umfaßt, der von einem Betätigungsglied berührungslos betätigbar ist. Auf diese Weise werden elektrische Verbindungen zwischen dem Betätigungsglied und der Drehzahlvorgabeeinheit überflüssig. So kann beispielsweise das Betätigungsglied mit dem Gehäuse verbunden sein und so zusammen mit der Maschine gereinigt werden, wogegen die Energie- und Steuereinheit vor dem Reinigen der Maschine entfernt werden kann. Korrosions- und Kontaktprobleme können auf diese Weise sicher vermieden werden.

Damit die Maschine in unterschiedlichen Betriebsmodi betrieben werden kann, ist es vorteilhaft, wenn die Energie- und Steuereinheit mindestens einen Betriebsmodusumschaltsensor (nicht Teil der Erfindung) trägt, der von einem Betätigungsglied berührungslos betätigbar ist. Durch die berührungslose Betätigbarkeit des Betriebsmodusumschaltsensors kann auf elektrische Verbindungen zwischen dem Betätigungsglied und der Motorsteuerung ganz verzichtet werden. Korrosionsprobleme werden so vermieden und eine optimierte Reinigbarkeit des Betätigungsglieds gewährleistet.

Grundsätzlich wäre es denkbar, daß der mindestens eine Betriebsmodusumschaltsensor ein Hall-Sensor (nicht Teil der Erfindung) ist, der von einem Magneten oder einem Weicheisenelement berührungslos betätigbar ist. Vorzugsweise ist der mindestens eine Betriebsmodusumschaltsensor eine Lichtschranke, die von einem Betätigungslied berührungslos betätigbar ist. Beispielsweise kann ein relativ zum Gehäuse an diesem gelagertes bewegliches Betätigungsglied die an der Energie- und Steuereinheit vorgesehene Lichtschranke unterbrechen oder freigeben, um beispielsweise von einem ersten in einen zweiten Betriebsmodus umzuschalten.

Günstigerweise ist mindestens ein Betriebsmodusaktivierungssensor (nicht Teil der Erfindung) vorgesehen zum Aktivieren oder Deaktivieren eines bestimmten Betriebsmodus der Maschine. Beispielsweise kann dadurch ein bestimmter Betriebsmodus der Maschine, beispielsweise ein Oszillationsbetrieb oder ein Pilgerschrittbetrieb, in gewünschter Weise aktiviert oder deaktiviert werden, insbesondere auch dauerhaft.

Grundsätzlich wäre es denkbar, daß der Betriebsmodusaktivierungssensor (nicht Teil der Erfindung) ein manuell betätigbares Schaltelement ist. Günstig ist es jedoch, wenn der Betriebsmodusaktivierungssensor eine Lichtschranke ist und wenn die Lichtschranke von einem Betriebsmodusaktivierungsbetätigungsglied berührungslos betätigbar ist. Denkbar wäre es auch, den Betriebsmodusaktivierungssensor (nicht Teil der Erfindung) in Form eines induktiven oder kapazitiven Sensors oder Schaltelements auszubilden.

Günstigerweise ist die Lichtschranke eine Infrarotlichtschranke. Dies gestattet es, den Betriebsmodusaktivierungssensor und/oder den Betriebsmodusumschaltsensor vorteilhafterweise als Infrarotlichtschranke auszubilden. (nicht Teil der Erfindung) Die beiden Sensoren können so auch in einem insbesondere für optisches Licht nicht durchlässigen Gehäuse geschützt angeordnet werden. Dadurch wird sowohl die Gefahr einer Beschädigung der Sensoren beim Reinigen sowie unbeabsichtigtes Berühren vermieden.

Vorteilhafterweise trägt der Antrieb das Betriebsmodusaktivierungsbetätigungsglied. Dies gestattet es, beim Einbau eines bestimmten Antriebs in die Maschine bereits vorzusehen, ob ein oder mehrere Betriebsmodi dauerhaft aktiviert oder deaktiviert werden sollen. Das mindestens eine Betriebsmodusaktivierungsbetätigungsglied ist dazu beispielsweise derart angeordnet, daß es mit dem Betriebsmodusaktivierungssensor in Wechselwirkung treten kann. Durch das Betriebsmodusaktivierungsbetätigungsglied läßt sich auf einfache Weise eine Codierung des Antriebs für bestimmte Betriebsmodi erreichen.

Für einen besonders kompakten Aufbau der Maschine ist es vorteilhaft, wenn die Energie- und Steuereinheit den Betriebsmodusaktivierungssensor trägt. So kann mit der Energie- und Steuereinheit ein Betriebsmodusaktivierungssensor auf einfache Weise ausgetauscht werden, falls er defekt sein sollte. Die Austauschbarkeit der Energie- und Steuereinheit hat zudem den Vorteil, daß bei einem Defekt eines Teils der Energie- und Steuereinheit, dieser Defekt von einer Bedienperson besonders einfach behoben werden soll, und zwar durch Austausch der defekten Energie- und Steuereinheit durch eine funktionsfähige Energie- und Steuereinheit. Eine Wartung der Energie- und Steuereinheit kann dann separat von der Maschine erfolgen, die mit einer weiteren Energie- und Steuereinheit weiter betrieben werden kann.

Besonders robust und korrosionsbeständig wird das Gehäuse, wenn es aus Titan hergestellt ist. Ferner wird es so auch besonders leicht.

Um eine optimierte Zerlegbarkeit der Maschine zu erreichen, ist es vorteilhaft, wenn ein mit dem Gehäuse verbindbarer Rahmen vorgesehen ist und wenn der Antrieb mit dem Rahmen lösbar verbindbar ist. Dies ermöglicht es, den Antrieb vom Gehäuse durch Lösen des Rahmens vom Gehäuse zu lösen. Insbesondere dann, wenn der Rahmen weitere Bauelemente oder Baugruppen der Maschine trägt, kann so die Maschine auf einfache Weise zerlegt werden, beispielsweise zu Wartungs- oder Reinigungszwecken.

Gemäß einer bevorzugten Ausführungsform der Erfindung kann vorgesehen sein, daß mindestens ein Betätigungsglied zum Vorgeben einer Drehzahl und/oder einer Drehrichtung und/oder eines Betriebsmodus des Antriebs vorgesehen ist. Das Betätigungsglied kann beispielsweise ein beweglich gelagertes Betätigungsglied sein oder aber auch ein auf Druck empfindliches Betätigungsglied, beispielsweise ein Drucksensor.

Um den Aufbau der Maschine weiter zu vereinfachen, ist es günstig, wenn das mindestens eine Betätigungsglied mit dem Rahmen zur Ausbildung einer Drückereinheit lösbar verbindbar ist. Das mindestens eine Betätigungsglied wird so Teil einer Drückereinheit, die auch als Ganzes vom Gehäuse lösbar sein kann. Das mindestens eine Betätigungsglied kann insbesondere zur Aktivierung des Betriebsmodusumschaltsensors und/oder Betriebsmodusaktivierungssenors dienen.

Gemäß einer weiteren bevorzugten Ausführungsform der Erfindung kann eine Kupplungsvorrichtung zum Verbinden mit einem chirurgischen Werkzeug vorgesehen sein. Über die Kupplungsvorrichtung läßt sich der Antrieb der Maschine insbesondere mit einem chirurgischen Werkzeug oder auch einer Getriebeeinheit verbinden, um das Werkzeug in gewünschter Weise zu bewegen, beispielsweise in Rotation oder in oszillierende Schwingungen zu versetzen, wie dies beispielsweise bei einer Säge der Fall ist.

Um die Zerlegbarkeit der Maschine zu verbessern, ist es vorteilhaft, wenn die Kupplungsvorrichtung mit dem Rahmen lösbar verbindbar ist. Dadurch kann es beispielsweise möglich werden, einen Rotor des Antriebs aus diesem und aus dem Gehäuse zu entfernen, wenn die Kupplungsvorrichtung vom Rahmen gelöst wird. Die Kupplungsvorrichtung kann bei einer solchen Ausgestaltung gleichzeitig als Gegenlager für einen Rotor dienen, kann, muß jedoch nicht zwingend Lagerungselemente für diesen aufweisen.

Günstigerweise umfaßt die Kupplungsvorrichtung mindestens ein Verriegelungselement zum Sichern eines Kupplungselements des chirurgischen Werkzeugs oder Instruments in einer Kupplungsaufnahme. Durch das Verriegelungselement kann verhindert werden, daß sich das Kupplungselement des Werkzeugs, einer Getriebeeinheit oder des Instruments von der Maschine löst, wenn es in die Kupplungsaufnahme eingreift.

Um auf einfache Weise das Kupplungselement des Werkzeugs, der Getriebeeinheit oder des Instruments in der Kupplungsaufnahme zu sichern, ist es günstig, wenn das mindestens eine Verriegelungselement quer zur Längsachse der Kupplungsvorrichtung bewegbar ist, in einer Einführstellung die Kupplungsaufnahme zum Einführen des Kupplungselements freigibt und in einer Kupplungsstellung die Kupplungsaufnahme verschließt.

Damit sich ein mit der Maschine verbundenes Werkzeug oder Instrument nicht in unbeabsichtigter Weise lösen kann, ist es vorteilhaft, wenn das mindestens eine Verriegelungselement in einer Grundstellung die Kupplungsstellung einnimmt.

Vorzugsweise ist das mindestens eine Verriegelungselement federnd vorgespannt in der Grundstellung gehalten. Wird das Verriegelungselement aus der Grundstellung in die Einführstellung überführt oder in eine andere gewünschte Stellung, dann wird aufgrund der federnden Vorspannung das Verriegelungselement nach Freigabe wieder in die Grundstellung überführt.

Besonders vorteilhaft ist es, wenn das mindestens eine Verriegelungselement durch Einführen des Kupplungselements in die Kupplungsaufnahme von der Kupplungsstellung in die Einführstellung überführbar ist. Eine Bedienperson muß bei einer derart ausgebildeten Kupplungsvorrichtung keine Betätigungselemente betätigen, es genügt vielmehr, das Kupplungselement gegen das Verriegelungselement zu bewegen, wodurch dieses automatisch in die Einführstellung überführt wird, sodaß das Kupplungselement vollständig in die Kupplungsaufnahme eingeführt werden kann zur Verbindung des Instruments oder Werkzeugs mit der Maschine.

Besonders einfach kann das Verriegelungselement durch Einführen des Kupplungselements in die Kupplungsaufnahme von der Kupplungsstellung in die Einführstellung überführt werden, wenn das mindestens eine Verriegelungselement eine erste Aufgleitfläche aufweist, an welcher das Kupplungselement aufgleiten kann zum Überführen des Verriegelungselements von der Kupplungsstellung in die Einführstellung. Beispielsweise durch Bewegen des Kupplungselements parallel zur Längsachse der Kupplungsvorrichtung kann bei einer entsprechend vorgesehenen Aufgleitfläche das Verriegelungselement quer zur Längsrichtung der Kupplungsvorrichtung bewegt werden.

Eine Verriegelung des Kupplungselements in der Kupplungsaufnahme wird besonders einfach, wenn das Verriegelungselement eine zweite Aufgleitfläche aufweist und wenn das Verriegelungselement derart gelagert ist, daß die zweite Aufgleitfläche nach dem Einführen des Verriegelungselements in die Kupplungsaufnahme am Kupplungselement aufgleiten kann und das Kupplungselement spielfrei in der Kupplungsaufnahme hält. Beispielsweise kann das Verriegelungselement federnd vorgespannt sein, sodaß es nach Einführen des Kupplungselements in die Kupplungsaufnahme in die Grundstellung zurückbewegt wird, wobei dann die zweite Aufgleitfläche am Kupplungselement aufgleitet und dieses in der Kupplungsaufnahme sichert, und zwar spielfrei, da aufgrund der federnden Vorspannung das Verriegelungselement genau so weit bewegt werden kann, bis kein Spiel mehr quer zur Bewegungsrichtung des Verriegelungselements zwischen dem Kupplungselement und der Kupplungsaufnahme verbleibt.

Besonders einfach wird eine Verbindung der Maschine mit einem Werkzeug, einer Getriebeeinheit oder Instrument, wenn die mindestens eine Kupplungsaufnahme eine sich in Richtung der Längsachse der Kupplungsvorrichtung erstreckende Ausnehmung ist, die an eine koaxial zur Längsachse angeordnete Werkzeugaufnahme der Kupplungsvorrichtung angrenzt und wenn das mindestens eine Kupplungselement ein vom Instrument oder Werkzeug in radialer Richtung abstehender, in die Kupplungsaufnahme einführbarer Vorsprung ist. Das mit der Maschine zu verbindende Instrument oder Werkzeug kann mit einem Kupplungsteil parallel zur Längsachse in die Werkzeugaufnahme der Kupplungsvorrichtung eingeführt werden, wobei gleichzeitig das Kupplungselement in die Kupplungsaufnahme eingreift oder eintaucht.

Der Aufbau sowohl des Werkzeugs oder Instruments als auch der Kupplungsvorrichtung und damit der Maschine wird besonders einfach, wenn der Vorsprung ein zylindrischer Stift ist. Insbesondere kann ein Verriegelungselement an einem solchen Stift besonders gut aufgleiten.

Um eine sichere Verbindung der Maschine mit einem Instrument oder Zubehörteil sicherzustellen, ist es günstig, wenn drei Kupplungsaufnahmen mit jeweils einem Verriegelungselement für jeweils ein Kupplungselement vorgesehen sind.

Um die Maschine universell einsetzen zu können, ist es vorteilhaft, wenn ein mit dem Antrieb lösbar verbindbares Getriebe vorgesehen ist. Beispielsweise kann dann mit dem Getriebe ein Instrument oder Werkzeug in gewünschter Weise verbunden werden, beispielsweise ein Bohrer oder ein Sägeblatt. So kann aufgrund der lösbaren Verbindbarkeit des Getriebes mit dem Antrieb die Maschine in vorteilhafter Weise in einzelne Baugruppen zerlegt werden.

Damit größere Baugruppen gemeinsam vom Gehäuse gelöst werden können, ist es vorteilhaft, wenn das Getriebe mit dem Rahmen lösbar verbindbar ist. Das Getriebe kann dann zusammen mit dem Rahmen vom Gehäuse gelöst werden.

Je nachdem, ob ein vom Antrieb bereitgestelltes Drehmoment oder eine Drehzahl für bestimmte chirurgische Zwecke erhöht oder vermindert werden sollen, kann es günstig sein, wenn das Getriebe ein Unter- oder ein Übersetzungsgetriebe ist.

Gemäß einer bevorzugten Ausführungsform der Erfindung kann vorgesehen sein, daß mit dem Getriebe eine Rotationsbewegung einer Antriebswelle des Antriebs in eine Oszillationsbewegung eines mit einem Werkzeug verbindbaren Kupplungsteils umsetzbar ist. Diese Ausgestaltung ermöglicht es, die Maschine zum Beispiel als Säge einzusetzen. Ein Sägeblatt kann mit dem Kupplungsteil verbunden werden und durch die Oszillationsbewegung, beispielsweise um eine Drehachse oder in einer Längsrichtung, kann sowohl eine Stichsäge als auch eine oszillierende Pendelsäge bereitgestellt werden.

Der Aufbau der Maschine wird besonders kompakt, wenn das Kupplungsteil derart gelagert ist, daß eine oszillierende Bewegung des Kupplungsteils in Verlängerung einer Längsachse der Antriebswelle ausführbar ist. Beispielsweise kann so eine Stichsäge ausgebildet werden, wobei das Sägeblatt direkt in Verlängerung einer Längsachse des Antriebs mit dem Kupplungsteil verbunden werden kann, was einen besonders schlanken Aufbau der Maschine ermöglicht.

Der Aufbau des Getriebes zum Ermöglichen einer oszillierenden Bewegung des Kupplungsteils wird besonders einfach, wenn das Getriebe einen von einem Stirnradgetriebe angetriebenen Exzenter umfaßt. Eine Rotationsbewegung eines Rotors des Antriebs kann so in eine oszillierende Bewegung des Exzenters umgesetzt werden.

Damit eine oszillierende Bewegung des Kupplungsteils in Verlängerung einer Längsachse der Antriebswelle ausführbar ist, ist es günstig, wenn das Stirnradgetriebe ein um eine Achse quer zur Längsachse der Antriebswelle rotierbar gelagertes Kegelrad umfaßt, wenn das Kegelrad den Exzenter trägt und wenn der Exzenter parallel zur Längsachse des Kegelrads absteht.

Gemäß einer bevorzugten Ausführungsform der Erfindung kann vorgesehen sein, daß zum Verschließen der mindestens zwei Öffnungen des Gehäuses mindestens zwei Verschlußelemente vorgesehen sind. Dadurch kann zum einen verhindert werden, daß innerhalb des Gehäuses angeordnete Teile aus diesem unbeabsichtigt heraustreten können. Zum anderen sind die im Gehäuse angeordneten Teile, Elemente oder Baugruppen der Maschine gegen äußere Einflüsse geschützt.

Besonders einfach wird der Aufbau der Maschine, wenn der Rahmen ein Verschlußelement bildet.

Um das Eindringen von Keimen in das Innere der Maschine beziehungsweise in das Innere des Gehäuses zu verhindern, ist es günstig, wenn die mindestens zwei Öffnungen fluiddicht abgedichtet sind. Dies ermöglicht es unter anderem, die Maschine ohne die Energie- und Steuereinheit zu reinigen, insbesondere zu sterilisieren. Anschließend kann die Energie- und Steuereinheit, die nicht zwingend keimfrei sein oder gemacht werden muß, in das Gehäuse eingeführt und die dafür vorgesehene Öffnung fluiddicht abgedichtet werden.

Der Aufbau der Maschine vereinfacht sich weiter, wenn die mindestens zwei Verschlußelemente die mindestens zwei Öffnungen fluiddicht verschließen. Hierfür können beispielsweise entsprechend einer Kontur der jeweiligen Öffnung geformte Dichtelemente eingesetzt werden, bei denen es sich beispielsweise um im Querschnitt kreisförmige Dichtungen oder auch Lippendichtungen handeln kann. Vorzugsweise werden O-Ringe verwendet, wenn es sich bei den Öffnungen um in sich geschlossene ringförmige Öffnungen handelt.

Eine Standzeit der Maschine erhöht sich, wenn der Elektromotor ein bürstenloser Motor ist. Ausfälle aufgrund von Reparatur und Wartung der Maschine werden so deutlich verringert.

Um einen Schaltungsaufwand zu minimieren und damit den Motor besonders kompakt aufbauen zu können, ist es günstig, wenn der Elektromotor ein elektronisch kommutierter Gleichstrommotor ist.

Eine baugruppenweise Zerlegung der Maschine wird weiter verbessert, wenn eine den Antrieb umfassende Antriebseinheit vorgesehen ist und wenn die Antriebseinheit mit dem Rahmen lösbar verbindbar ist. Beispielsweise können an einem Elektromotor noch weitere Elemente vorgesehen sein, beispielsweise besondere Motorkontakte oder Befestigungselemente zum Festlegen am Rahmen oder am Gehäuse, sodaß die den Antrieb umfassende Antriebseinheit dann als Ganzes mit dem Rahmen vom Gehäuse entfernt werden kann.

Um die Wartungsfreundlichkeit der Maschine weiter zu erhöhen, ist es günstig, wenn der Elektromotor einen Rotor umfaßt und wenn der Rotor parallel zu seiner Längsachse aus der Antriebseinheit entfernbar ist nach Entfernen der Kupplungsvorrichtung und/oder des an dem Rahmen angeordneten Getriebes.

Insbesondere dann, wenn der Rotor eine Welle umfaßt, auf welcher Lager, Wuchtelemente und ein Dauermagnet befestigt sind, kann durch den vorgeschlagenen Aufbau der Rotor einfach und schnell ausgewechselt werden, ohne das Gehäuse vollständig öffnen zu müssen. Der auf der Welle vorgesehene Magnet kann ein Dauermagnet sein, insbesondere ein einteiliger Magnet oder ein aus dünnen Einzelscheiben zusammengesetzter Magnet. Der Vorteil hierbei ist, daß Wirbelstromverluste verringert werden. Wird ein Dauermagnet verwendet, so ist dieser vorzugsweise durchbohrt und kann dadurch auf die Rotorwelle aufgefädelt werden. Dies erhöht die Festigkeit und die Biegesteifigkeit des Rotors. Um den Rotor vor äußeren Einflüssen bei der Reinigung, insbesondere bei der Sterilisierung zu schützen, wird vorzugsweise eine Umhüllung in Form einer dünnen Hülse aus nichtrostendem, amagnetischen Material über dem auf der Welle gelagerten Magnet vorgesehen. Dadurch wird die mechanische Festigkeit des Rotors insbesondere bei hohen Drehzahlen erhöht. Ferner wird so ein Korrosionsschutz für den Magnet gebildet, sodaß auch Neodym-Eisen-Bor-Magnete eingesetzt werden können.

Um den Antrieb in definierter Weise und sicher am Gehäuse zu halten, ist es günstig, wenn mindestens ein Befestigungselement vorgesehen ist. Beispielsweise kann das Befestigungselement eine Schraube oder eine Schraubhülse oder aber auch ein Bauteil sein, mit dem der Antrieb über eine Bajonettverbindung mit dem Gehäuse verbunden werden kann.

Um den Antrieb besonders einfach mit dem Gehäuse verbinden zu können, ist es vorteilhaft, wenn das Befestigungselement in Verlängerung einer Längsachse des Antriebs zum festlegenden Verbinden des Antriebs am Gehäuse vorgesehen ist. Insbesondere kann das Befestigungselement auch dazu dienen, eine Verlängerungshülse, die vom Gehäuse in Verlängerung der Längsachse des Rotors absteht, festzulegen, beispielsweise wenn mit der Maschine ein K-Draht gesetzt werden soll, der sich durch den gesamten Antrieb hindurcherstreckt.

Der Aufbau der Maschine wird weiter vereinfacht, wenn das Befestigungselement ein Verschlusselement bildet. Somit dient das Befestigungselement sowohl zum Verschließen einer Öffnung als auch zum Festlegen des Antriebs am Gehäuse.

Die Maschine kann besonders einfach zerlegt werden, wenn der Rahmen vom Gehäuse lösbar ist nach Entfernen des Befestigungselements. Beispielsweise kann der Rahmen in einer entsprechenden Ausnehmung gehalten werden durch von dem Befestigungselement aufgebrachte Zugkräfte, die den Rahmen in die entsprechende Ausnehmung ziehen. Ein Lösen des Befestigungselements gibt dann auch den Rahmen relativ zum Gehäuse frei.

Um möglichst vielfältige chirurgische Eingriffe mit der Maschine ausführen zu können, ist es vorteilhaft, wenn die Maschine ein Bohr- oder Fräsmaschine, eine Stichsäge oder eine oszillierende Säge ist.

Gemäß einer bevorzugten Ausführungsform der Erfindung kann vorgesehen sein, daß das mindestens eine am Rahmen oder am Gehäuse beweglich gelagerte Betätigungsglied relativ zum Rahmen und/oder zum Gehäuse fluiddicht abgedichtet ist. Dadurch wird verhindert, daß Keime im Bereich des Betätigungsglieds ins Innere des Gehäuses eindringen können.

Eine besonders einfache und dauerhaft dichte Verbindung zwischen dem Betätigungsglied und dem Rahmen und/oder dem Gehäuse wird erreicht, indem für das mindestens eine Betätigungsglied eine Faltenbalgdichtung vorgesehen ist.

Um die Zahl der Betätigungsglieder zu minimieren und damit auch die Zahl der abzudichtenden Öffnungen oder Durchbrechungen ins Innere des Gehäuses, ist es günstig, wenn die Stichsäge und die oszillierende Säge jeweils nur ein einziges beweglich gelagertes Betätigungsglied zum Vorgeben einer Drehzahl des Antriebs aufweisen. Eine Bohrmaschine dagegen kann beispielsweise zwei Betätigungsglieder aufweisen, eines zum Vorgeben einer Drehzahl, ein anderes zum Um- oder Einstellen eines Betriebsmodus der Maschine.

Um die Zahl der erforderlichen Lager für einen Rotor des Antriebs zu minimieren und dessen Lagerung zusätzlich zu verbessern, ist es günstig, wenn die Stichsäge und die oszillierende Säge einen Antrieb aufweisen, der nur an einem Ende ein Wellenlager für einen Rotor des Antriebs umfaßt und wenn ein zweites Wellenlager für den Rotor in einer Getriebeeinheit der Maschine vorgesehen ist. Der Rotor kann dann mit der Getriebeeinheit verbunden werden, wobei er einerseits ein Wellenlager tragen kann, andererseits ein Wellenlager für den Rotor im Getriebe vorgesehen ist. Man könnte eine derartige Welle des Rotors auch als zweiteilige Welle bezeichnen, wobei ein Teil im Antrieb, ein anderer Teil im Getriebe vorgesehen ist.

Günstig ist es, wenn die Bohr- oder Fräsmaschine zwei beweglich gelagerte Betätigungsglieder aufweist zum Vorgeben einer Drehzahl des Antriebs und zum Umschalten des Antriebs von einem ersten in mindestens einen zweiten Betriebsmodus und umgekehrt.

Vorteilhaft ist es, wenn eine chirurgische Drückereinheit zum Vorgeben einer Drehzahl und/oder einer Drehrichtung der chirurgischen Maschine mit mindestens einem in einer Betätigungsrichtung beweglich gelagertes Betätigungsglied vorgesehen ist. Der Vorteil einer solchen Drückereinheit liegt insbesondere darin, daß diese als Ganzes von der Maschine, insbesondere vom Gehäuse entfernt werden kann, beispielsweise wenn sie beschädigt ist oder separat gereinigt werden soll.

Günstig ist es ferner, wenn die Drückereinheit den Rahmen umfaßt. Ist der Rahmen vom Gehäuse entfernbar, dann bedeutet dies, daß auch die Drückereinheit als Ganzes vom Gehäuse entfernbar ist. Dadurch wird eine Zerlegbarkeit der Maschine weiter vereinfacht. Denkbar wäre es eine Felderzeugungseinheit zum Erzeugen eines magnetischen oder elektrischen Feldes und mindestens ein Betätigungssensor (nicht Teil der Erfindung) zum Erzeugen eines Betätigungssignals als Reaktion auf eine Bewegung und/eine Stellung des Betätigungsglieds vorgesehen sein, wobei das erzeugte Betätigungssignal mit einer Feldstärke und/oder einer Änderung des von der Felderzeugungseinheit erzeugten Feldes in Folge einer Bewegung des Betätigungsgliedes korreliert ist. Diese Ausgestaltung kann insbesondere durch den Betätigungssensor berührungslos betätigt werden, beispielsweise indem durch ein entsprechendes Betätigungselement oder Betätigungsglied eine Feldstärke und/oder eine Änderung des von der Felderzeugungseinheit erzeugten Feldes bewirkt werden kann.

Aus der US 5,747,953 ist beispielsweise eine batteriebetriebene chirurgische Antriebseinheit mit einer Drückereinheit in Form einer Akkumaschine bekannt, welche als Felderzeugungseinheit einen Magneten aufweist, welcher mit einem Drücker der Drückereinheit verbunden ist und relativ zu einem als Betätigungssensor dienenden Hallsensor bewegt werden kann. Durch Verändern der räumlichen Distanz des Magneten zum Sensor wird ein Betätigungssignal erzeugt. Eine derartige Anordnung ermöglicht es zwar, zu Reinigungszwecken, beispielsweise zum Sterilisieren der Antriebseinheit, eine in der Antriebseinheit angeordnete Elektronik vor dem Sterilisieren von der Drückereinheit zu trennen. Allerdings weist die bekannte Konstruktion den Nachteil auf, daß der Magnet einen Reinigungszyklus ebenfalls mitmachen muß. Insbesondere dann, wenn er nicht vollständig gekapselt ist, kann er sich unter Umständen von dem Drücker lösen. Ferner stellt sich bei jeder Verbindung der Elektronik, welche den Hallsensor umfaßt, und den Drückern der Drückereinheit das Problem, daß Magnet und Sensor relativ zueinander wieder richtig justiert werden. Wird die Antriebseinheit häufig zerlegt, dann kann es im Laufe der Zeit zu einer Dejustage kommen, die die Funktionsfähigkeit der Antriebseinheit negativ beeinflussen kann. Um daher die Betriebssicherheit der Maschine möglichst lange zu gewährleisten, ist es vorteilhaft, wenn der Betätigungssensor (nicht Teil der Erfindung) mit der Felderzeugungseinheit gekoppelt ist und daß ein Feldänderungsglied vorgesehen ist zum Erzeugen einer Änderung des am Ort des Betätigungssensors wirkenden, von der Felderzeugungseinheit erzeugten Feldes infolge einer Bewegung und/oder einer geänderten Stellung des Betätigungsgliedes. Diese Ausgestaltung macht es insbesondere möglich, den Betätigungssensor zusammen mit der Felderzeugungseinheit in einer festen räumlichen Korrelation zu Reinigungszwecken aus der Antriebseinheit zu entnehmen, so daß keine Toleranzprobleme beim Zusammensetzen von Antriebseinheit und Steuerelektronik mit Felderzeugungseinheit und Betätigungssensor auftreten können. Insbesondere dann, wenn bei mehreren verwendeten Antriebseinheiten deren Steuerelektroniken vertauscht werden, wirkt sich dies nicht nachteilig aus. Zudem läßt sich so vermeiden, daß die Felderzeugungseinheit einer Aufbereitung der Antriebseinheit unterzogen wird. Ferner wird die Betriebssicherheit erhöht, da die Felderzeugungseinheit nicht mehr zwingend relativ zum Sensor bewegt werden muß.

Vorzugsweise sind der Betätigungssensor (nicht Teil der Erfindung) und die Felderzeugungseinheit relativ zueinander feststehend angeordnet. So lassen sich alle möglichen Nachteile einer relativ zueinander beweglichen Anordnung von Betätigungssensor und Felderzeugungseinheit gänzlich ausschließen. Eine Funktionsfähigkeit der Drückereinheit bleibt gewährleistet, da das Feldänderungsglied eine Änderung des von der Felderzeugungseinheit erzeugten Feldes am Ort des Betätigungssensors bewirken kann, zur Verwendung mit der Antriebseinheit.

Grundsätzlich könnte der Betätigungssensor (nicht Teil der Erfindung) ein elektrischer oder ein elektromagnetischer Betätigungssensor sein. Vorzugsweise ist er jedoch ein Magnetfeldsensor. Damit eignet sich jede Felderzeugungseinheit, welche ein magnetisches Feld erzeugen kann, zur Verwendung mit der Antriebseinheit.

Vorzugsweise ist der Magnetfeldsensor ein Hallsensor. (nicht Teil der Erfindung) Mit einem solchen Sensor lassen sich auf einfache Weise Betätigungssignale in Form von elektrischen Spannungen erzeugen.

Vorteilhaft ist es, wenn das Feldänderungsglied mindestens teilweise magnetisch polarisierbar und eine von Null verschiedene magnetische Suszeptibilität χₘ aufweist. Dies ermöglicht es, bei Einführen des Feldänderungsglieds in das von der Felderzeugungseinheit erzeugte Feld, daß eine Flußdichte am Ort des Betätigungssensors verändert wird, wodurch ein Betätigungssignal erzeugt werden kann.

Besonders günstig ist es, wenn das Feldänderungsglied mindestens teilweise diamagnetisch, paramagnetisch, ferromagnetisch, antiferromagnetisch oder ferrimagnetisch ist. Materialien mit den genannten magnetischen Eigenschaften können eine gewünschte Änderung eines Magnetfeldes, insbesondere am Ort eines Magnetfeldsensors, bewirken.

Ein besonders einfacher Aufbau der Drückereinheit ergibt sich, wenn das Feldänderungsglied ein Weicheisenelement ist. Es läßt sich auf einfache Weise herstellen und ist widerstandsfähig gegen herkömmliche Reinigungsmittel, welche zur Reinigung der Antriebseinheit eingesetzt werden.

Besonders einfach wird der Aufbau der Drückereinheit, wenn die Felderzeugungseinheit ein Magnet ist.

Grundsätzlich wäre es denkbar, als Magnet einen Elektromagnet einzusetzen. Besonders sicher wird ein Betrieb der Drückereinheit jedoch dann, wenn der Magnet ein Permanentmagnet ist. Wartungsintervalle der Drückereinheit werden dadurch deutlich verlängert.

Vorteilhaft ist es aber auch, wenn der Magnet durch eine Spule gebildet wird.

Um eine möglichst hohe Flußdichte am Ort des Betätigungssensors (nicht Teil der Erfindung) zu erhalten, ist es vorteilhaft, wenn der Betätigungssensor zwischen Polen der Felderzeugungseinheit angeordnet ist.

Insbesondere kann es gemäß einer bevorzugten Ausführungsform der Erfindung vorteilhaft sein, wenn der Betätigungssensor in einem Spalt einer Ringspule angeordnet ist.

Um zusätzlich eine Verstärkung des Betätigungssignals zu erhalten, ist es günstig, wenn ein Querschnitt des Feldänderungsglieds in einer Betätigungsrichtung des Betätigungsglieds variiert. Dadurch wird erreicht, daß das von einem Fluß des Feldes durchsetzte Volumen des Feldänderungsglieds in Folge einer Betätigung des mindestens einen Betätigungsglieds vergrößert wird, nämlich dann, wenn das Feldänderungsglied in das Feld der Felderzeugungseinheit hinein- oder aus dem Feld herausbewegt wird.

Günstigerweise nimmt der Querschnitt zu. Ein Feldänderungsglied mit einem zunehmenden Querschnitt läßt sich besonders leicht ausbilden.

Eine besonders gute Kopplung zwischen der Felderzeugungseinheit und dem Betätigungssensor (nicht Teil der Erfindung) kann erreicht werden, wenn die Felderzeugungseinheit über ein Rückschlußsystem mit dem Betätigungssensor gekoppelt ist. Das Rückschlußsystem eignet sich in vorteilhafter Weise dazu, einen Fluß des von der Felderzeugungseinheit erzeugten Feldes direkt von der Felderzeugungseinheit zum Betätigungssensor zu führen. Dadurch werden unerwünschte Einflüsse des von der Felderzeugungseinheit erzeugten Feldes auf eine Steuerelektronik der Antriebseinheit vermieden.

Insbesondere bei einer magnetischen Felderzeugungseinheit ist es günstig, wenn das Rückschlußsystem ein magnetisches Rückschlußsystem ist. Beispielsweise kann hier ein magnetisierbares Material zur Herstellung des Rückschlußsystems, beispielsweise einer mechanischen Kopplung, verwendet werden. Denkbar wäre es, daß die Felderzeugungseinheit, das Rückschlußsystem und der Betätigungssensor eine Ausnehmung definieren und daß das Feldänderungsglied derart angeordnet ist, daß es in Folge einer Bewegung des Betätigungsglieds in die Ausnehmung mindestens teilweise einführbar ist. So läßt sich einfach und sicher eine Änderung des am Ort des Betätigungssensors wirkenden Feldes bewirken.

Der Aufbau der Drückereinheit wird besonders einfach, wenn die Ausnehmung einen im wesentlichen rechteckigen Querschnitt aufweist.

Grundsätzlich wäre es denkbar, daß das Betätigungsglied das Feldänderungsglied antreibt oder mit diesem indirekt verbunden ist. Der Aufbau der Drückereinheit wird jedoch noch einfacher, wenn das Betätigungsglied das Feldänderungsglied trägt.

Besonders vorteilhaft ist es, wenn die Felderzeugungseinheit an der Energieversorgungseinheit angeordnet ist. Insbesondere kann die Felderzeugungseinheit an der Energie- und Steuereinheit angeordnet sein.Damit kann erreicht werden, daß nur das Feldänderungsglied am Betätigungsglied anzuordnen ist, alle anderen Elemente, insbesondere die Felderzeugungseinheit, an der Energie- und Steuereinheit angeordnet werden können. Es sind somit keine elektrischen Kontakte zwischen dem Betätigungsglied und der Energie- und Steuereinheit erforderlich, um zum Beispiel eine Drehzahl mit dem Betätigungsglied vorzugeben. Ferner können dadurch Fertigungstoleranzen einfach ausgeglichen werden, beispielsweise wenn die Position der Energie- und Steuereinheit nach Auswechseln derselben durch Austausch gegen eine andere Energie- und Steuereinheit nicht mehr identisch ist, das heißt, wenn das Feldänderungsglied nicht mehr die ursprüngliche Position relativ zur Felderzeugungseinheit einnimmt.

Vorteilhaft ist es, wenn die Maschine mindestens zwei verschiedene Betriebsmodi aufweist, wobei einem ersten Betriebsmodus eine erste Betriebsmodistellung des mindestens einen Betätigungsglieds zugeordnet ist und wenn das mindestens eine Betätigungsglied von der ersten Betriebsmodistellung in eine zweite Betriebsmodistellung, welche einem zweiten Betriebsmodus der Maschine zugeordnet ist, um eine Drehachse verdrehbar ist zum Umschalten der Antriebseinheit vom ersten Betriebsmodus in den zweiten Betriebsmodus. Eine solche Ausgestaltung der Drückereinheit gestattet es, dem Betätigungsglied mehrere Funktionen zuzuweisen. Beispielsweise kann es als Drehzahlvorgabeglied zum Vorgeben einer Drehzahl der Antriebseinheit dienen und gleichzeitig als Umschalter von einem ersten in einen zweiten Betriebsmodus.

Günstig kann es sein, wenn zwei Betätigungssensoren (nicht Teil der Erfindung) vorgesehen und derart angeordnet sind, daß mit dem einen Betätigungssensor eine Stellung des mindestens einen Betätigungsglieds in der ersten Betriebsmodistellung und/oder eine Bewegung des mindestens einen Betätigungsglieds in ersten Betriebsmodistellung und daß mit dem zweiten Betätigungssensor eine Stellung des mindestens einen Betätigungsglieds in der zweiten Betriebsmodistellung und/oder eine Bewegung des mindestens einen Betätigungsglieds in der zweiten Betriebsmodistellung detektierbar ist. Man kann auf diese Weise in Abhängigkeit von einer erforderlichen Empfindlichkeit unterschiedliche Betätigungssensoren für unterschiedliche Zwecke einsetzen. So läßt sich der Aufbau der Drückereinheit an besondere Erfordernisse einer verwendeten Schaltung oder deren Bauelemente anpassen.

Vorteilhafterweise weist der Elektromotor einen Rotor und mindestens zwei Motorwicklungen auf.

In der Chirurgie werden zunehmend Maschinen mit einer netzunabhängigen Energieversorgung eingesetzt. Dies hat zur Folge, daß bei üblicherweise als Energieversorgungen eingesetzten Batterien oder Akkumulatoren Umrichterschaltungen vorgesehen werden müssen, um aus von den Energieversorgungen bereitgestellten Gleichspannungen zum Betreiben eines Elektromotors mit mehreren, in der Regel drei Motorwicklungen erforderliche zeitabhängige Spannungs- und Stromverläufe bereitzustellen.

Aufgrund der netzunabhängigen Energieversorgung muß der Elektromotor elektronisch kommutiert werden. Allerdings ergeben sich insbesondere bei kleinen Motordrehzahlen, das heißt bei Drehzahlen von weniger als 1000 Umdrehungen pro Minute, erhöhte Anforderungen an die Motorsteuer- und/oder -regelung. Da ferner hohe Ansprüche an ein optimales Anlaufverhalten des Motors unter Last sowie an dessen Dynamik gestellt werden und gleichzeitig in jedem Arbeitspunkt der bestmögliche Wirkungsgrad erreicht werden soll, ist es erforderlich, die Position oder Lage des üblicherweise durch einen Magneten gebildeten Rotors des Motors zu bestimmen. Erst die genaue Rotorposition ermöglicht es, die als Motor- oder Statorwicklungen bezeichneten Spulen zum geforderten Kommutierungszeitpunkt bestimmungsgemäß bestromen zu können.

Es ist bekannt, zur Positionserkennung Sensorsysteme einzusetzen, beispielsweise digitale oder analoge Hall-Systeme. Nachteilig bei diesen Ausführungen ist, daß Positionssensoren in den Motor integriert und mit der Motorsteuerung verbunden werden müssen. Es müssen folglich für jeden Positionssensor entsprechende Kontakte vorgesehen werden, wenn die Motorsteuerung nicht fest mit dem Elektromotor verbunden ist. Dies kann zu Kontaktkorrosion beim Reinigen, insbesondere beim Sterilisieren der Maschine führen, und im schlimmsten Fall die Maschine außer Betrieb setzen.

Ferner ist es bekannt, für Anwendungen, bei denen keine hohen Anforderungen an die Dynamik, das Anlaufmoment und die Motorqualität im Bereich kleiner Motordrehzahlen gestellt werden, sensorlose Rotorpositionserkennungsverfahren zu nutzen. Da bei herkömmlichen elektronischen Kommutierungsverfahren für Elektromotoren stets eine Motorwicklung nicht bestromt wird, wird zur Ermittlung einer Ist-Motordrehzahl, die Gegen-EMK (Elektro-Motorische-Kraft) an der nicht bestromten Motorwicklung gemessen und ausgewertet.

Die oben beschriebenen, bekannten Steuer- und Regelungsverfahren für chirurgische Maschinen erfordern entweder einen erhöhten schaltungstechnischen Aufwand und zusätzliche Bauteile, insbesondere Sensorsysteme mit Positionssensoren, oder sind nicht geeignet, den Elektromotor aus dem Stillstand unter Last gezielt anlaufen zu lassen und auch bei sehr niedrigen Drehzahlen mit hoher Laufruhe zu betreiben.

Es ist daher vorteilhaft, eine chirurgische Maschine insbesondere so zu verbessern, daß der Elektromotor mit optimalem Wirkungsgrad bei kleinen Drehzahlen betreibbar ist sowie ein bestimmungsgemäßer Motoranlauf auch unter Last ermöglicht wird. Dies wird gemäß einer bevorzugten Ausführungsform der Erfindung beispielsweise dadurch erreicht, daß mit der Motorsteuerung ein Space-Vektor-Puls-Weiten-Modulations-(SVPWM)-Verfahren zum Steuern und/oder Regeln des Elektromotors durchführbar ist, bei welchem alle Motorwicklungen gleichzeitig bestrombar sind.

Die Motorsteuerung so auszubilden, daß die chirurgische Maschine mittels Space-Vektor-Puls-Weiten-Modulations-(SVPWM)-Verfahren gesteuert und/oder geregelt werden kann, verbessert insbesondere den Motoranlauf und dessen Betrieb bei kleinen Drehzahlen. Grund hierfür ist insbesondere, daß, anders als bei einem herkömmlichen Puls-Weiten-Modulations-(PWM)-Verfahren, alle Motorwicklungen gleichzeitig bestromt werden. Dies bedeutet insbesondere bei einem Elektromotor mit drei Motorwicklungen, daß nicht nur zwei, sondern alle drei Motorleitungen bestromt werden. Bei drei Motorwicklungen können so jeweils 60°-Phasen einer Rotorbewegung des Elektromotors relativ zu den Motorwicklungen stufenlos variiert werden. Bei herkömmlicher beziehungsweise bislang angewandten Puls-Weiten-Modulations-(PWM)-Verfahren konnte ein Feldwinkel des Statorfeldes nicht stufenlos, sondern nur in 60°-Schritten geändert werden. Vor allem bei niedrigen Drehzahlen kann so ein wesentlich ruhigerer Motorlauf erreicht werden. Ferner läßt sich das Anlaufen des Motors unabhängig von einer Rotorposition des Elektromotors ganz gezielt vorgeben.

Ein optimaler Aufbau der Maschine ergibt sich, wenn die Motorsteuerung eine Steuerungseinheit und eine Leistungseinheit umfaßt. Auf diese Weise läßt sich insbesondere eine Leistungsaufnahme der Maschine minimieren, wenn der Elektromotor stillsteht.

Eine elektronische Kommutierung läßt sich auf einfache Weise dadurch erreichen, daß die Leistungseinheit jeweils zwei Leistungstransistoren für jede der mindestens zwei Motorwicklungen umfaßt. Es lassen sich so auf einfache Weise positive und negative Spannungen bezogen auf ein Referenzpotential an die mindestens zwei Motorwicklungen anlegen, auch wenn nur eine Gleichspannungsquelle als Energieversorgung zur Verfügung steht.

Besonders wartungsfreundlich wird die Maschine, wenn der Elektromotor ein bürstenloser Gleichstrommotor ist. Insbesondere kann der Elektromotor auch elektronisch kommutiert sein.

Grundsätzlich wäre es denkbar, auf eine Bestimmung der Rotorposition des Rotors des Elektromotors zu verzichten. Um jedoch insbesondere das Anlaufen des Elektromotors unter Last zu optimieren, ist es günstig, wenn zur Steuer- und/oder Regelung einer Bestromung der mindestens zwei Rotorwicklungen eine Rotorposition des Elektromotors bestimmbar ist. Durch das Space-Vektor-Puls-Weiten-Modulations-(SVPWM)-Verfahren läßt sich bei Kenntnis der Rotorposition der Feldwinkel des durch die bestromten Motorwicklungen erzeugten Statorfeldes stufenlos so weiterschalten, daß ein optimaler Wirkungsgrad des Motors erreicht wird.

Gemäß einer bevorzugten Ausführungsform der Erfindung kann vorgesehen sein, daß zur Bestimmung der Rotorposition des Elektromotors mindestens eine der mindestens zwei Motorwicklungen für ein Zeitintervall t_{Unterbrechung} von einer Energieversorgung der Maschine trennbar ist, daß während des Zeitintervalls t_{Unterbrechung} eine Gegen-EMK der mindestens einen der mindestens zwei Motorwicklungen meßbar ist, und daß aus der gemessenen Gegen-EMK eine Ist-Position des Rotors berechenbar ist. Mit anderen Worten bedeutet dies, daß die gleichzeitige Bestromung beim Space-Vektor-Puls-Weiten-Modulations-(SVPWM)-Verfahren für ein bestimmtes Zeitintervall gezielt kurz unterbrochen wird, und zwar an einer, mehreren oder allen Motorwicklungen. Während der kurzen Unterbrechung kann dann an einer, mehreren oder allen Motorwicklungen die Gegen-EMK bestimmt werden und aufgrund deren Größe auf eine Stellung des Rotors relativ zu den Motorwicklungen geschlossen werden.

Eine Bestimmung der Rotorposition läßt sich weiter verbessern, wenn alle Motorwicklungen gleichzeitig für das Zeitintervall t_{Unterbrechung} von der Energieversorgung der Maschine trennbar sind. Es läßt sich so gleichzeitig die Gegen-EMK an allen Motorwicklungen bestimmen, sodaß sich eventuelle Ungenauigkeiten bei der Bestimmung der Gegen-EMK an nur einer Motorwicklung folglich weniger stark auswirken.

Damit das Zeitintervall t_{Unterbrechung} möglichst kurz bleibt, ist es günstig, wenn die an den mindestens zwei Motorwicklungen anliegenden Spannungen vor oder zu Beginn des Zeitintervalls t_{Unterbrechung} oder vor der Messung der Gegen-EMK meßbar sind und wenn diejenige Motorwicklung, an welcher die niedrigste Spannung gemessen wird, mit einem vorgegebenen Spannungspotential verbindbar ist. Durch diese Ausgestaltung wird eine Zeit für das Einschwingen des Systems minimiert, das heißt, die Gegen-EMK kann nach einer minimalen Wartezeit gemessen werden.

Besonders einfach wird der Aufbau der chirurgischen Maschine, wenn das vorgegebene Spannungspotential Masse ist.

Um eine Bestimmung der Rotorposition weiter zu optimieren, ist es vorteilhaft, wenn die Motorsteuerung derart ausgebildet ist, daß die Gegen-EMK während des Zeitintervalls t_{Unterbrechung} erst nach einer Einschwingzeit t_{Einschwing} gemessen wird. Dies bedeutet mit anderen Worten, daß beispielsweise die Bestromung an mindestens einer Motorwicklung unterbrochen wird, also das Zeitintervall t_{Unterbrechung} beginnt, und erst nach der Einschwingzeit t_{Einschwing}, welche üblicherweise kürzer als das Zeitintervall t_{Unterbrechung} ist, die Gegen-EMK gemessen wird.

Um die Genauigkeit der Bestimmung der Rotorposition weiter zu verbessern, ist es günstig, wenn die Motorsteuerung derart ausgebildet ist, daß zur Bestimmung der Gegen-EMK ein Spannungsverlauf an der oder den nicht mit dem vorgegebenen Spannungspotential verbundenen Motorwicklungen meßbar ist und wenn die Einschwingzeit t_{Einschwing} mindestens der Zeit tₖₒₙₛₜₐₙₜ entspricht, bis die an der oder den nicht mit dem vorgegebenen Spannungspotential Motorwicklungen anliegenden Spannungen im Zeitverlauf konstant oder nahezu konstant sind. Diese Ausgestaltung gestattet es, die Einschwingzeit t_{Einschwing} je nach Bedarf zu variieren. Durch die Bestimmung der Zeit tₖₒₙₛₜₐₙₜ kann die Einschwingzeit t_{Einschwing} gezielt eingestellt und minimiert werden.

Vorzugsweise ist die Motorsteuerung derart ausgebildet, daß ein konstanter Wert für das Zeitintervall t_{Unterbrechung} vorgegeben ist. Es läßt sich so die Motorsteuerung wesentlich vereinfachen.

Gemäß einer alternativen Ausführungsform der Maschine kann es jedoch vorteilhaft sein, wenn die Motorsteuerung derart ausgebildet ist, daß das Zeitintervall t_{Unterbrechung} veränderbar ist. Dadurch kann insbesondere das Zeitintervall t_{Unterbrechung} vergrößert oder verkleinert werden, falls die Zeit tₖₒₙₛₜₐₙₜ länger als das zunächst vorgegebene Zeitintervall t_{Unterbrechung} ist.

Einen optimierten Betrieb der Maschine kann man erreichen, wenn die Motorsteuerung derart ausgebildet ist, daß die Dauer des Zeitintervalls t_{Unterbrechung} derart vorgebbar ist, daß während des Zeitintervalls t_{Unterbrechung} die an der oder den nicht mit dem vorgegebenen Spannungspotential verbundenen Motorwicklungen anliegenden Spannungen im Zeitverlauf einen konstanten oder nahezu konstanten Spannungswert annehmen. Insbesondere bei kurzen Abklingzeiten, das heißt, wenn die Zeit tₖₒₙₛₜₐₙₜ sehr klein ist, läßt sich das Zeitintervall t_{Unterbrechung} entsprechend anpassen, wodurch die Bestromungsunterbrechung der Motorwicklungen minimal kurz wird. Dadurch wird die Laufruhe des Motors, insbesondere bei niedrigen Drehzahlen und beim Anlaufen, verbessert.

Vorzugsweise ist die Motorsteuerung derart ausgebildet, daß das Zeitintervall t_{Unterbrechung} vergrößerbar ist, wenn die Zeit tₖₒₙₛₜₐₙₜ größer als das Zeitintervall t_{Unterbrechung} ist, und/oder daß das Zeitintervall t_{Unterbrechung} verkleinerbar ist, wenn die Zeit tₖₒₙₛₜₐₙₜ kleiner als das Zeitintervall t_{Unterbrechung} ist. So wird sichergestellt, daß das Zeitintervall t_{Unterbrechung} nie länger als unbedingt erforderlich ist, um die Gegen-EMK für die Ermittlung der Rotorposition möglichst genau zu bestimmen.

Grundsätzlich wäre es denkbar, das Zeitintervall t_{Unterbrechung} periodisch zu variieren. Günstig ist es jedoch, wenn die Motorsteuerung derart ausgebildet ist, daß das Zeitintervall t_{Unterbrechung} pro Umdrehung schrittweise veränderbar ist. Insbesondere ist es günstig, wenn das Zeitintervall t_{Unterbrechung} schrittweise vergrößerbar oder verkleinerbar ist. Auf diese Weise kann das Zeitintervall t_{Unterbre-chung} verändert werden, bis es mindestens der Zeit tₖₒₙₛₜₐₙₜ entspricht, um die Gegen-EMK sicher und genau bestimmen zu können.

Gemäß einer bevorzugten Ausführungsform der Erfindung kann vorgesehen sein, daß die Motorsteuerung derart ausgebildet ist, daß eine Soll-Position mit der aus der Gegen-EMK-Messung bestimmten Ist-Position des Rotors vergleichbar ist und daß ein Feldwinkel der Space-Vektor-Puls-Weiten-Modulation (SVPWM) entsprechend der ermittelten Differenz zwischen Soll- und Ist-Position des Rotors nachregelbar ist. Die Motorsteuerung bestimmt also eine Abweichung der Ist-Position von der Sollposition des Rotors und regelt aufgrund der bestimmten Positionsabweichung den Feldwinkel des durch die Motorwicklungen erzeugten Statorfeldes nach. Es kann so ein optimaler Wirkungsgrad des Motors erreicht werden.

Um die Genauigkeit bei der Bestimmung der Gegen-EMK weiter zu erhöhen, ist es vorteilhaft, wenn die Motorsteuerung derart ausgebildet ist, daß die Gegen-EMK erst meßbar ist, wenn der Motorstrom mindestens einer der mindestens zwei Motorwicklungen auf Null abgefallen ist. Es lassen sich so eventuelle, durch das Fließen eines Motorstroms bedingte Meßfehler bei der Ermittlung einer Gegen-EMK vermeiden.

Günstigerweise bilden die Energieversorgungseinheit und die Motorsteuerung eine Einheit und ist die Einheit mit der Maschine lösbar verbindbar. Dies hat insbesondere den Vorteil, daß zu Reinigungszwecken, beispielsweise zur Sterilisierung der Maschine, alle hitze- und feuchtigkeitsempfindlichen Teile der Maschine entfernt werden können. Die Ausbildung der Motorsteuerung und der netzunabhängigen Energieversorgung als Einheit verkürzt eine Vorbereitungszeit der chirurgischen Maschine für den Einsatz.

Günstig ist es, wenn die Motorsteuerung eine Verbindungsschaltung umfaßt, die einen Prozessor der Motorsteuerung erst mit der Energieversorgungseinheit verbindet, wenn der Elektromotor mit der Motorsteuerung verbunden ist. Dadurch kann eine vorzeitige Entladung, insbesondere eine Selbstentladung einer netzunabhängigen Energieversorgungseinheit vermieden werden. Üblicherweise haben nämlich Prozessoren von Motorsteuerungen einen im Vergleich zu anderen Bauteilen der Steuerung deutlich erhöhten Energieverbrauch. Eine Selbstentladung der netzunabhängigen Energieversorgungseinheit kann dadurch vermieden werden, da eine Aktivierung der Motorsteuerung erst nach Verbinden derselben mit dem Elektromotor möglich wird.

Besonders einfach wird der Aufbau der chirurgischen Maschine, wenn der Elektromotor drei Motorwicklungen aufweist.

Chirurgische Maschinen der eingangs beschriebenen Art sind in zahlreichen Varianten bekannt, insbesondere als Bohr- und Fräsmaschinen oder Sägen.

Sie werden betrieben, indem mit der Motorsteuerung Steuersignale für den Elektromotor erzeugt werden, um ihn mit einer bestimmten Drehzahl zu betreiben. Je nach Art des Elektromotors können Drehzahlen bis zu 70.000 Umdrehungen pro Minute erreicht werden. Konstruktionsbedingt ist jedoch der Wirkungsgrad von Elektromotoren nicht bei allen Drehzahlen gleich, insbesondere nicht immer optimal.

Es ist daher vorteilhaft, eine chirurgische Maschine so zu verbessern, daß insbesondere ein Wirkungsgrad des Elektromotors im wesentlichen über den gesamten Drehzahlbereich optimiert werden kann. Dies kann gemäß einer bevorzugten Ausführungsform der Erfindung beispielsweise dadurch erreicht werden, daß ein Gesamtdrehzahlbereich der chirurgischen Maschine in mindestens einen unteren Drehzahlbereich für niedrige Drehzahlen und in mindestens einen oberen Drehzahlbereich für höhere Drehzahlen als der mindestens eine untere Drehzahlbereich unterteilt ist, daß die Motorsteuerung derart ausgebildet ist, daß in dem mindestens einen unteren Drehzahlbereich ein erstes Steuer- und/oder Regelungsverfahren zum Steuern und/oder Regeln des Elektromotors durchführbar ist und daß in dem mindestens einen oberen Drehzahlbereich ein zweites Steuer- und/oder Regelungsverfahren zum Steuern und/oder Regeln des Elektromotors durchführbar ist. Dies hat den Vorteil, daß jeweils an einen Drehzahlbereich des Elektromotors angepaßte Steuer- und/oder Regelungsverfahren angewandt werden können. Es wäre insbesondere denkbar, daß mehr als zwei Drehzahlbereiche definiert werden, wobei jeweils beim Übergang von einem zum anderen Drehzahlbereich auch das jeweils angewandte Steuer- und/oder Regelungsverfahren umgeschaltet wird. Auf diese Weise läßt sich nicht nur der Wirkungsgrad des Elektromotors im Betrieb optimieren, sondern es läßt beispielsweise auch eine Ist-Drehzahl des Elektromotors während des Betriebs in Abhängigkeit der Drehzahl optimiert bestimmen.

Vorteilhaft ist es, wenn das erste und/oder das zweite Steuer- und/oder Regelungsverfahren ein Puls-Weiten-Modulations-(PWM)-Verfahren ist. Mit diesem Verfahren lassen sich insbesondere Gleichstrommotoren auf einfache Weise und optimiert betreiben. Insbesondere lassen sich sinusartige Strom- und Spannungsverläufe erzeugen durch Überlagerung digitaler Spannungs- bzw. Stromsignale mit einer Trägerfrequenz.

Gemäß einer bevorzugten Ausführungsform der Erfindung kann vorgesehen sein, daß das erste Steuer- und/oder Regelungsverfahren ein Space-Vektor-Puls-Weiten-Modulations-(SVPWM)-Verfahren ist, bei welchem alle Motorwicklungen gleichzeitig bestrombar sind. Das SVPWM-Verfahren hat gegenüber herkömmlichen Puls-Weiten-Modulations-(PWM)-Verfahren den Vorteil, daß alle Motorwicklungen gleichzeitig bestrombar sind, so daß auch bei besonders niedrigen Drehzahlen ein sanfter, ruckelfreier Betrieb des Elektromotors möglich wird. Ferner wird ein Anlaufen des Motors aus dem Stillstand wesentlich dadurch verbessert, daß alle Motorwicklungen gleichzeitig bestrombar sind. Alternativ kann in vorteilhafter Weise vorgesehen sein, daß der Motor Drehzahlerfassungssensoren (nicht Teil der Erfindung) aufweist und daß die Motorsteuerung derart ausgebildet ist, daß das erste Steuer- und/oder Regelungsverfahren ein Verfahren zum Steuern und/oder Regeln der chirurgischen Maschine ist, bei welchem die Motorsteuerung Steuersignale für den Elektromotor in Abhängigkeit einer mit den Drehzahlerfassungssensoren ermittelten Ist-Drehzahl bereitstellt. Die Drehzahlerfassungssensoren können auch dazu dienen, eine Position des Rotors des Elektromotors zu bestimmen. Drehzahlerfassungssensoren insbesondere bei niedrigen Drehzahlen des Elektromotors einzusetzen, hat den Vorteil, daß die Drehzahl wesentlich genauer bestimmt werden kann als beispielsweise durch Ermittlung einer an der oder den Motorwicklungen erzeugten Gegen-EMK (Elektro-Motorische-Kraft). Die Ermittlung der Gegen-EMK ist insbesondere bei höheren Drehzahlen besser geeignet, da in diesem Fall höhere Induktionsspannungen erzeugt werden und sich die ermittelten Signale dadurch besser verarbeiten lassen.

Ein besonders einfacher Aufbau der Maschine ergibt sich, wenn ein Hall-System zur Erfassung einer Ist-Drehzahl des Elektromotors vorgesehen ist und wenn das Hall-System die Drehzahlerfassungssensoren (nicht Teil der Erfindung) umfaßt. Hall-Sensoren als Drehzahlerfassungssensoren können besonders klein ausgebildet und direkt in den Elektromotor integriert werden.

Gemäß einer bevorzugten Ausführungsform der Erfindung kann vorgesehen sein, daß ein Drehzahlgrenzwert zwischen dem mindestens einen unteren und dem mindestens einen oberen Drehzahlbereich unveränderbar ist. In diesem Fall kann ein Umschalten zwischen den mindestens zwei Steuer- und/oder Regelungsverfahren immer bei einem gewünschten Drehzahlgrenzwert stattfinden.

Gemäß einer weiteren bevorzugten Ausführungsform der Erfindung kann auch vorgesehen sein, daß ein Drehzahlgrenzwert zwischen dem mindestens einen unteren und dem mindestens einen oberen Drehzahlbereich veränderbar ist. Je nach Betriebssituation läßt sich dadurch ein Umschalten zwischen den mindestens zwei Steuer- und/oder Regelungsverfahren gezielt verändern. Schaltpunkte können dann in gewünschter Weise variiert werden.

Ein dauerndes Umschalten zwischen den mindestens zwei Steuer- und/oder Regelungsverfahren läßt sich auf einfach Weise dadurch vermeiden, daß die Motorsteuerung derart ausgebildet ist, daß eine Umschaltung vom ersten Steuer- und/oder Regelungsverfahren in das zweite Steuer- und/oder Regelungsverfahren bei einer ersten Umschaltdrehzahl erfolgt und daß eine Umschaltung vom zweiten Steuer- und/oder Regelungsverfahren in das erste Steuer- und/oder Regelungsverfahren bei einer zweiten Umschaltdrehzahl erfolgt. Es lassen sich so zwei Schaltpunkte definieren, und zwar beim Übergang vom niedrigen Drehzahlbereich in den höheren Drehzahlbereich und umgekehrt. Es ist so möglich, die Umschaltzeitpunkte zu trennen, das heißt eine kleine Variation der Ist-Drehzahl des Motors führt nicht zwangsläufig sofort zum Umschalten in das andere Steuer- und/oder Regelungsverfahren.

Grundsätzlich wäre es denkbar, daß die erste Umschaltdrehzahl kleiner als die zweite Umschaltdrehzahl ist. Besonders günstig ist es jedoch, wenn die erste Umschaltdrehzahl gleich oder größer als die zweite Umschaltdrehzahl ist. Ein Umschalten in den höheren Drehzahlbereich findet somit bei einer höheren Umschaltdrehzahl statt als das Umschalten vom höheren Drehzahlbereich in den niedrigeren Drehzahlbereich. Es ergibt sich somit quasi ein Hysteresekurve mit einem Bereich, in welchem für bestimmte Drehzahlen sowohl das eine als auch das andere Steuer- und/oder Regelungsverfahren angewandt wird, allerdings in Abhängigkeit davon, ob die Drehzahl des Elektromotors zu- oder abnimmt.

Prinzipiell wäre es möglich, die Maschine so auszubilden, daß eine Bedienperson einen gewünschten Drehzahlbereich vorgibt und das entsprechende Steuer- und/oder Regelungsverfahren aktiviert. Gemäß einer bevorzugten Ausführungsform der Erfindung kann jedoch vorgesehen sein, daß die Motorsteuerung derart ausgebildet ist, daß die Umschaltung vom ersten Steuer- und/oder Regelungsverfahren in das zweite Steuer- und/oder Regelungsverfahren beim Übergang vom mindestens einen unteren in den mindestens einen oberen Drehzahlbereich, und umgekehrt, automatisch erfolgt. Die Bedienperson muß bei dieser Ausgestaltung der Maschine lediglich die Drehzahl vorgeben, bei welcher sie die Maschine betreiben möchte.

Günstig ist es, wenn die Maschine ein erstes und ein zweites Betätigungsglied aufweist und wenn die Motorsteuerung derart ausgebildet ist, daß durch Betätigen des ersten Betätigungsglieds der Motorsteuerung eine Drehzahl des Antriebs vorgebbar ist. Beispielsweise kann das zweite Betätigungsglied zum Umschalten der Maschine von einem ersten Betriebsmodus in einen zweiten Betriebsmodus dienen. Vorzugsweise könnte jedoch auch das erste Betätigungsglied derart ausgebildet sein, daß es eine Umschaltung von einem ersten in einen zweiten Betriebsmodus ermöglicht. Dies könnte beispielsweise dadurch erreicht werden, daß das Betätigungsglied um seine Längsachse verdrehbar ist. Ein Betriebsmodus könnte dabei auch einem ausgeschalteten Zustand der Maschine entsprechen, der zweite Betriebsmodus oder die zweite Stellung des Betätigungsglieds einer Betriebsstellung der Maschine, in welcher sie eingeschaltet ist, wobei es auf einen speziellen Betriebsmodus nicht ankommt. Dadurch wäre es möglich, mit dem ersten Betätigungsglied einerseits eine Drehzahl des Antriebs vorzugeben, andererseits auch die Maschine dauerhaft auszuschalten, um eine Verletzungsgefahr durch eine abgelegte, nicht ordnungsgemäß gesicherte Maschine zu minimieren.

Ferner kann es vorteilhaft sein, wenn die Motorsteuerung derart ausgebildet ist, daß durch Betätigen des zweiten Betätigungsglieds von einem ersten Betriebsmodus in einen zweiten Betriebsmodus des Antriebs umgeschaltet werden kann. Vorzugsweise ist der erste Betriebsmodus ein Rechtslaufbetrieb des Antriebs, der zweite Betriebsmodus ein Linkslaufbetrieb des Antriebs. Diese Ausgestaltung erlaubt es, das erste Betätigungsglied zum Vorgeben einer Drehzahl zu betätigen, wobei bei Betätigung des zweiten Betätigungsglieds der Antrieb beispielsweise von einem Rechtslauf in einen Linkslauf umschaltet. Dabei kann die Motorsteuerung ferner derart ausgebildet sein, daß eine Umschaltung von einem Betriebsmodus in einen anderen Betriebsmodus durch einmaliges Betätigen des zweiten Betätigungsglieds bewirkt wird oder daß der Antrieb nur so lange im anderen Betriebsmodus betrieben wird wie das zweite Betätigungsglied betätigt wird.

Gemäß einer weiteren bevorzugten Ausführungsform der Erfindung kann vorgesehen sein, daß die Motorsteuerung derart ausgebildet ist, daß der Antrieb in einem Rechts- und/oder Linkslaufbetrieb und/oder in einem oszillierendem Betrieb, bei welchem der Antrieb abwechselnd und jeweils gleichlange in unterschiedlichen Drehrichtungen läuft, und/oder in einem Pilgerschrittbetrieb betreibbar ist, wobei im Pilgerschrittbetrieb der Antrieb abwechselnd in unterschiedlichen Drehrichtungen betrieben wird, wobei der Antrieb jeweils etwas länger in einer ersten Drehrichtung als in einer zweiten Drehrichtung betrieben wird. Im Pilgerschrittbetrieb kann beispielsweise auf einfache Weise ein

Gewinde geschnitten werden, wobei der Antrieb hierfür immer etwas länger in Vorwärtsrichtung zum Schneiden des Gewindes betrieben wird als in Rückwärtsrichtung. Ein oszillierender Betrieb kann insbesondere beim Bearbeiten von Oberflächen vorteilhaft sein, um keine fortschreitende Bewegung des mit der Maschine verbundenen Instruments oder Werkzeugs zu erlauben.

Vorteilhaft kann es sein, wenn zum Umschalten des Betriebs der Maschinen von einem Rechts- oder Linkslaufbetrieb auf den oszillierenden Betrieb und/oder den Pilgerschrittbetrieb das zweite Betätigungsglied mindestens während einer vorgegebenen Umschaltzeit dauerhaft betätigbar ist. Eine derartige Motorsteuerung macht kein Betätigungsglied erforderlich, das von einer ersten Stellung in eine zweite Stellung mechanisch umgestellt werden muß. Insbesondere kann das Betätigungsglied ein druckempfindlicher Sensor (nicht Teil der Erfindung) sein.

Wird das Betätigungsglied länger als die vorgegebene Umschaltzeit betätigt, dann kann die Maschine zum Beispiel vom Rechts- oder Linkslaufbetrieb in den oszillierenden Betrieb oder in den Pilgerschrittbetrieb übergehen.

Damit nicht aus einem laufenden Betrieb, also aus einem Rechts- oder Linkslaufbetrieb in den oszillierenden Betrieb oder den Pilgerschrittbetrieb umgeschaltet werden kann, kann die Motorsteuerung vorteilhaft derart ausgebildet sein, daß zum Umschalten vom Rechts- oder Linkslaufbetrieb auf den oszillierenden Betrieb oder den Pilgerschrittbetrieb das zweite Betätigungsglied betätigbar ist, während das erste Betätigungsglied unbetätigt ist. Ein Umschalten zwischen unterschiedlichen Betriebsmodi kann so vorzugsweise nur dann erfolgen, wenn das erste Betätigungsglied unbetätigt ist, also insbesondere keine Drehzahlanforderung für den Antrieb vorliegt. Dies stellt sicher, daß ein Umschalten nur bei stillstehendem Antrieb erfolgen kann.

Günstigerweise ist die Motorsteuerung derart ausgebildet, daß als Umschaltzeit eine Zeitdauer in einem Bereich von 2 bis 5 Sekunden vorgebbar ist. Vorzugsweise ist eine Umschaltzeit von etwa 3 Sekunden vorgebbar. Durch das Vorgeben einer Umschaltzeit im angegebenen Zeitbereich wird sichergestellt, daß nicht durch unbeabsichtigtes Betätigen des zweiten Betätigungsglieds ein unerwünschtes Umschalten von einem ersten in einen zweiten Betriebsmodus erfolgt. Ein Umschalten muß durch eine Bedienperson daher bewußt vorgenommen werden.

Vorteilhafterweise ist die Motorsteuerung derart ausgebildet, daß der Oszillationsbetrieb und/oder der Pilgerschrittbetrieb beibehaltbar sind, auch wenn das zweite Betätigungsglied nach Betätigen während der vorgegebenen Umschaltzeit nicht mehr betätigt wird. Dies erlaubt es, den Antrieb dauerhaft in einen anderen Betriebsmodus umzuschalten. Erforderlich ist hierfür nur die einmalige Betätigung des zweiten Betätigungsglieds für mindestens die Umschaltzeit.

Gemäß einer weiteren bevorzugten Ausführungsform der Erfindung kann vorgesehen sein, daß die Motorsteuerung derart ausgebildet ist, daß der Antrieb vom Oszillationsbetrieb und/oder dem Pilgerschrittbetrieb in den Rechts- oder Linkskaufbetrieb umschaltbar ist durch Betätigen des zweiten Betätigungsglieds für eine Zeitdauer, die kürzer als die vorgegebene Umschaltdauer ist. Dadurch kann durch gezieltes Betätigen des zweiten Betätigungsglieds zwischen zwei Betriebsmodi umgeschaltet werden.

Günstig ist es, wenn die Motorsteuerung derart ausgebildet ist, daß der Antrieb vom Oszillationsbetrieb oder dem Pilgerschrittbetrieb in den Linkslaufbetrieb umschaltbar ist durch Betätigen des zweiten Betätigungsglieds für eine Zeitdauer, die kürzer als die vorgegebene Umschaltdauer ist. Dies ermöglicht es, gezielt vom Oszillationsbetrieb und/oder dem Pilgerschrittbetrieb in den Linkslaufbetrieb umzuschalten.

Gemäß einer bevorzugten Ausführungsform der Erfindung kann vorgesehen sein, daß die Motorsteuerung derart ausgebildet ist, daß nach dem Umschalten vom Rechts- oder Linkslaufbetrieb in den oszillierenden Betrieb oder den Pilgerschrittbetrieb nach Betätigen des ersten Betätigungsglieds der Antrieb zuerst in den Pilgerschrittbetrieb umschaltbar ist. Steht der Antrieb beispielsweise still, so kann nach Betätigen des ersten Betätigungsglieds, zum Beispiel zur Anforderung einer bestimmten Drehzahl des Antriebs, zunächst nur in den Pilgerschrittbetrieb umgeschaltet werden. Denkbar wäre es auch, die Motorsteuerung derart auszubilden, daß zuerst ein oszillierender Betrieb nach Betätigen der Drehzahlanforderung vorgenommen wird. Eine Umschaltung zwischen Pilgerschrittbetrieb und oszillierendem Betrieb kann in Abhängigkeit einer Betätigungsdauer des ersten Betätigungsglieds oder einer angeforderten Drehzahl erfolgen.

Vorzugsweise ist die Motorsteuerung derart ausgebildet, daß die Maschine im Pilgerschrittbetrieb betreibbar ist, solange mit dem ersten Betätigungsglied eine Drehzahl vorgegeben wird, die unterhalb einer Umschaltdrehzahl liegt. Auf diese Weise kann mit dem ersten Betätigungsglied durch Vorgabe der gewünschten Drehzahl des Antriebs zwischen dem Pilgerschrittbetrieb und einem weiteren Betriebsmodus, beispielsweise einem oszillierenden Betrieb, umgeschaltet werden.

Günstig ist es, wenn die Motorsteuerung derart ausgebildet ist, daß die Maschine vom Pilgerschrittbetrieb in den oszillierenden Betrieb umschaltbar ist, wenn mit dem ersten Betätigungsglied eine Drehzahl vorgegeben wird, die oberhalb der Umschaltdrehzahl liegt. Ein Umschalten vom Pilgerschrittbetrieb in den oszillierenden Betrieb erfolgt somit einfach durch Erhöhen der Drehzahlanforderung über eine bestimmte Drehzahl hinaus.

Vorteilhaft ist es bei einem Verfahren der eingangs beschriebenen Art, daß mit der Motorsteuerung ein Space-Vektor-Puls-Weiten-Modulations-(SVPWM)-Verfahren durchgeführt wird.

Das eingangs beschriebene Verfahren derart weiterzubilden hat den Vorteil, daß alle Motorwicklungen gleichzeitig bestromt werden können, wodurch sich ein Feldwinkel des durch die bestromten Motorwicklungen vorgebbaren Statorfeldes in gewünschter Weise stufenlos einstellen und variieren läßt. Insbesondere beim Anlaufen des Elektromotors und bei niedrigen Drehzahlen kann dadurch ein optimaler Betrieb und ein ruhiger Lauf des Elektromotors erreicht werden.

Vorteilhaft ist es, wenn die Motorsteuerung eine Steuerungseinheit und eine Leistungseinheit umfaßt.

Günstig ist es, wenn die Leistungseinheit jeweils zwei Leistungstransistoren für jede der mindestens zwei Motorwicklungen umfaßt. Dies gestattet es insbesondere, eine Gleichspannungsquelle als Energieversorgung zu verwenden.

Vorzugsweise kann vorgesehen sein, daß der Elektromotor ein bürstenloser Gleichstrommotor ist. Es wird so die Wartungsfreundlichkeit der chirurgischen Maschine verbessert.

Um eine Bestromung des Elektromotors in optimaler Weise vornehmen zu können, ist es vorteilhaft, wenn zur Steuer- und/oder Regelung einer Bestromung der mindestens zwei Motorwicklungen eine Rotorposition des Elektromotors bestimmt wird. Dies erlaubt es, in Abhängigkeit der Rotorposition die Motorwicklungen zu bestromen. So kann ein Feldwinkel des durch die bestromten Motorwicklungen erzeugten Statorfeldes optimal an die Rotorposition angepaßt werden, so daß ein ruhiger Betrieb und ein sanftes Anlaufen des Elektromotors sichergestellt werden können.

Günstig ist es, wenn zur Bestimmung der Rotorposition des Elektromotors mindestens eine der mindestens zwei Motorwicklungen für ein Zeitintervall t_{Un-terbrechung} von einer Energieversorgung der Maschine getrennt wird, wenn während des Zeitintervalls t_{Unterbrechung} die Gegen-EMK der mindestens einen der mindestens zwei Motorwicklungen gemessen wird und wenn aus der gemessenen Gegen-EMK eine Ist-Position des Rotors berechnet wird. Da beim Space-Vektor-Puls-Weiten-Modulations-(SVPWM)-Verfahren normalerweise alle Motorwicklungen gleichzeitig bestromt werden, kann eine Gegen-EMK nur bestimmt werden, wenn die Bestromung mindestens einer Motorwicklung unterbrochen wird. Dann kann die aufgrund elektromagnetischer Induktion an der kurzzeitig unbestromten Motorwicklung abfallende Spannung als Gegen-EMK gemessen und aus deren Wert die Ist-Position des Rotors berechnet werden.

Vorzugsweise werden alle Motorwicklungen gleichzeitig für das Zeitintervall t_{Unterbrechung} von der Energieversorgung der Maschine getrennt. Dies eröffnet die Möglichkeit, die Gegen-EMK an allen Motorwicklungen gleichzeitig zu bestimmen und auf diese Weise die Genauigkeit bei der Bestimmung der Ist-Position des Rotors zu verbessern.

Gemäß einer bevorzugten Variante des Verfahrens kann vorgesehen sein, daß die an den mindestens zwei Motorwicklungen anliegenden Spannungen vor oder zu Beginn des Zeitintervalls t_{Unterbrechung} oder vor der Messung der Gegen-EMK gemessen werden und daß diejenige Motorwicklung, an welcher die niedrigste Spannung gemessen wird, mit einem vorgegebenen Spannungspotential verbunden wird. Da grundsätzlich nicht die Gegen-EMK aller Motorwicklungen gemessen werden muß, um die Ist-Position des Rotors zu bestimmen, läßt sich durch die vorgeschlagene Weiterbildung des Verfahrens die Gegen-EMK besonders schnell bestimmen. Insbesondere wird so nämlich ein Einschwingvorgang optimiert und verkürzt.

Vorzugsweise ist das vorgegebene Spannungspotential Masse.

Um die Rotorposition noch genauer bestimmen zu können, ist es günstig, wenn die Gegen-EMK während des Zeitintervalls t_{Unterbrechung} erst nach einer Einschwingzeit t_{Einschwing} gemessen wird. Beispielsweise kann die Einschwingzeit direkt ab Beginn des Zeitintervalls t_{Unterbrechung} abgewartet werden. Durch das Abwarten der Einschwingzeit t_{Einschwing} kann vermieden werden, einen falschen Wert für die Gegen-EMK zu ermitteln, aus welchem sich wiederum eine falsche Ist-Position des Rotors ableiten würde.

Gemäß einer weiteren bevorzugten Variante des Verfahrens kann vorgesehen sein, daß zur Bestimmung der Gegen-EMK ein Spannungsverlauf an der oder den nicht mit dem vorgegebenen Spannungspotential verbundenen Motorwicklungen gemessen wird und daß die Einschwingzeit t_{Einschwing} mindestens einer Zeit tₖₒₙₛₜₐₙₜ entspricht, bis die an der oder den nicht mit dem vorgegebenen Spannungspotential verbundenen Motorwicklungen anliegenden Spannungen im Zeitverlauf konstant oder nahezu konstant sind. Die Bestimmung des Spannungsverlaufs an der oder den Motorwicklungen macht es möglich, die Gegen-EMK direkt nach dem Einschwingen zu bestimmen. Das Zeitintervall t_{Unterbrechung} kann auf diese Weise minimiert werden, was zu einer verbesserten Laufruhe und einer verbesserten Anlaufeigenschaft des Motors beiträgt.

Besonders einfach wird das Verfahren, wenn ein konstanter Wert für das Zeitintervall t_{Unterbrechung} vorgegeben wird. Allerdings kann die Ist-Position wesentlich genauer bestimmt und zudem die Laufruhe des Elektromotors weiter verbessert werden, wenn das Zeitintervall t_{Unterbrechung} während des Betriebs der Maschine verändert wird. Insbesondere dann, wenn die Einschwingzeit t_{Einschwing} länger als das Zeitintervall t_{Unterbrechung} dauert, kann das Zeitintervall entsprechend an die Einschwingzeit angepaßt werden, so daß die Gegen-EMK innerhalb des Zeitintervalls t_{Unterbrechung} bestimmt werden kann.

Vorteilhaft ist es, wenn die Dauer des Zeitintervalls t_{Unterbrechung} derart vorgegeben wird, daß während des Zeitintervalls t_{Unterbrechung} die an der oder den nicht mit dem vorgegebenen Spannungspotential verbundenen Motorwicklungen anliegenden Spannungen im Zeitverlauf einen konstanten oder nahezu konstanten Spannungswert annehmen. So ist es möglich, die Gegen-EMK an jeder gewünschten Motorwicklung dann zu bestimmen, wenn nach Einschwingen des Systems induzierte Spannungen an der oder den Motorwicklungen konstant oder im wesentlichen konstant sind. Dies erhöht die Genauigkeit bei der Bestimmung der Rotorposition.

Vorzugsweise kann vorgesehen sein, daß das Zeitintervall t_{Unterbrechung} vergrößert wird, wenn die Zeit tₖₒₙₛₜₐₙₜ größer als das Zeitintervall t_{Unterbrechung} ist, und/oder daß das Zeitintervall t_{Unterbrechung} verkleinert wird, wenn die Zeit tₖₒₙₛₜₐₙₜ kleiner als das Zeitintervall t_{Unterbrechung} ist. Durch diese Vorgehensweise wird sichergestellt, daß das Zeitintervall t_{Unterbrechung} nie länger als benötigt ist. Dies stellt einen besonders ruhigen Motorlauf sicher.

Günstig ist es, wenn das Zeitintervall t_{Unterbrechung} periodisch schrittweise verändert wird. Insbesondere kann dies pro Umdrehung des Rotors durchgeführt werden oder mit einer Abtastfrequenz, die kleiner als die Modulationsfrequenz des Space-Vektor-Puls-Weiten-Modulations-(SVPWM)-Verfahren oder des Puls-Weiten-Modulations-(PWM)-Verfahren ist. Die schrittweise Veränderung kann insbesondere eine Vergrößerung oder Verkleinerung sein. Das Verfahren so weiterzubilden ermöglicht eine kontinuierliche Anpassung des Zeitintervalls t_{Unterbrechung} an die tatsächlich erforderliche Unterbrechungszeit, um die Gegen-EMK an mindestens einer Motorwicklung in erforderlicher Weise zu messen.

Vorzugsweise werden nach Messung der Gegen-EMK alle Motorwicklungen wieder an die Energieversorgung der Maschine angeschlossen. Dies kann direkt oder mit Zeitverzögerung geschehen. Je schneller die Motorleitungen wieder an die Energieversorgung der Maschine angeschlossen werden, umso kürzer wird das Zeitintervall t_{Unterbrechung} und um so ruhiger ein Motorlauf.

Ferner kann es vorteilhaft sein, wenn eine Soll-Position des Rotors mit der aus der Gegen-EMK-Messung bestimmten Ist-Position des Rotors verglichen wird und wenn ein Feldwinkel der Space-Vektor-Puls-Weiten-Modulation (SVPWM) entsprechend der ermittelten Differenz zwischen Soll- und Ist-Position des Rotors nachgeregelt wird. Durch diese Nachregelung wird sichergestellt, daß die Bestromung des Elektromotors mit optimalem Wirkungsgrad geschieht.

Vorzugsweise wird die Gegen-EMK erst gemessen, wenn der Motorstrom mindestens einer der mindestens zwei Motorwicklungen auf Null abgefallen ist. Es lassen sich so Meßfehler bei der Bestimmung der Gegen-EMK auf einfache Weise vermeiden. Dies erhöht die Genauigkeit bei der Bestimmung der Rotorposition.

Vorteilhafterweise wird zur Energieversorgung der Maschine eine netzunabhängige Energieversorgung verwendet. Dies kann insbesondere eine Batterie oder ein Akkumulator sein. So läßt sich die Maschine kabellos betreiben.

Gemäß einer bevorzugten Variante des Verfahrens kann vorgesehen sein, daß die netzunabhängige Energieversorgung und die Motorsteuerung eine Einheit bilden und daß die Einheit vor Inbetriebnahme der Maschine mit der Maschine verbunden wird. Auf diese Weise ist es möglich, die chirurgische Maschine von der Energieversorgung und der Motorsteuerung getrennt zu reinigen. Eine Einheit vorzusehen erleichtert zudem das Zusammenfügen und Vorbereiten der Maschine für einen chirurgischen Einsatz.

Um eine vorzeitige Entleerung, insbesondere eine Selbstentladung, der netzunabhängigen Energieversorgung zu vermeiden, ist es vorteilhaft, wenn ein Prozessor der Motorsteuerung erst mit der netzunabhängigen Energieversorgung verbunden wird, wenn der Elektromotor mit der Motorsteuerung verbunden ist. Üblicherweise haben Prozessoren von Motorsteuerungen einen im Vergleich zu anderen Bauteilen der Steuerung deutlich erhöhten Energieverbrauch. Eine Selbstentladung der netzunabhängigen Energieversorgung kann dadurch vermieden werden, da eine Aktivierung der Motorsteuerung erst nach Verbinden derselben mit dem Elektromotor möglich wird.

Besonders einfach läßt sich das Verfahren durchführen, wenn ein Elektromotor mit drei Motorwicklungen verwendet wird.

Vorteilhafterweise kann vorgesehen sein, daß ein Gesamtdrehzahlbereich der chirurgischen Maschine in mindestens einen unteren Bereich für niedrige Drehzahlen und in mindestens einen oberen Drehzahlbereich für höhere Drehzahlen als der mindestens eine untere Drehzahlbereich unterteilt wird, und daß in dem mindestens einen unteren Drehzahlbereich das Space-Vektor-Puls-Weiten-Modulations-(SVPWM)-Verfahren durchgeführt wird. Das Space-Vektor-Puls-Weiten-Modulations-(SVPWM)-Verfahren ist besonders vorteilhaft für niedrige Drehzahlen, kann jedoch bei höheren Drehzahlen zu einer Dämpfung des Motors und damit zu einer Absenkung des Wirkungsgrads des Motors führen. Daher ist es vorteilhaft, im oberen Drehzahlbereich ein anderes Steuer- und/oder Regelungsverfahren zur Steuerung des Elektromotors vorzusehen.

Besonders vorteilhaft ist es, wenn im mindestens einen oberen Drehzahlbereich ein zweites Verfahren zum Steuern- und/oder Regeln der chirurgischen Maschine durchgeführt wird, welches ein Puls-Weiten-Modulations-(PWM)-Verfahren ist. Dieses Verfahren im oberen Drehzahlbereich durchzuführen, hat den Vorteil, daß ein unerwünschtes Dämpfen des Motors durch Anwendung des Space-Vektor-Puls-Weiten-Modulations-(SVPWM)-Verfahrens nicht auftreten kann. Mit anderen Worten wird durch die Durchführung von zwei unterschiedlichen Steuer- und/oder Regelungsverfahren der Wirkungsgrad des Elektromotors nahezu über den gesamten Drehzahlbereich verbessert.

Gemäß einer weiteren bevorzugten Variante des eingangs beschriebenen Verfahrens kann ferner vorgesehen sein, daß ein Gesamtdrehzahlbereich der chirurgischen Maschine im mindestens einen unteren Drehzahlbereich für niedrige Drehzahlen und im mindestens einen oberen Drehzahlbereich für höhere Drehzahlen als im mindestens einen unteren Drehzahlbereich unterteilt wird, daß in dem mindestens einen unteren Drehzahlbereich ein erstes Steuer- und/oder Regelungsverfahren zum Steuern und/oder Regeln des Elektromotors durchgeführt wird und daß in dem mindestens einen oberen Drehzahlbereich ein zweites Steuer- und/oder Regelungsverfahren zum Steuern und/oder Regeln des Elektromotors durchgeführt wird. Auf diese Weise kann eine chirurgische Maschine vorteilhaft betrieben werden, insbesondere läßt sich so ihr Wirkungsgrad insgesamt steigern, denn in Abhängigkeit der Drehzahl des Elektromotors kann das jeweils am besten geeignete Steuer- und Regelungsverfahren ausgewählt werden.

Auf besonders einfache Weise läßt sich ein Gleichstrommotor ansteuern, wenn das erste und/oder das zweite Steuer- und/oder Regelungsverfahren Puls-Weiten-Modulations-(PWM)-Verfahren sind.

Insbesondere bei niedrigen Drehzahlen und beim Anlaufen des Elektromotors ist es besonders günstig, wenn das erste Steuer- und/oder Regelungsverfahren ein Space-Vektor-Puls-Weiten-Modulations-(SVPWM)-Verfahren ist, bei welchem alle Motorwicklungen gleichzeitig bestromt werden. Dieses Verfahren gestattet es, insbesondere bei niedrigen Drehzahlen, einen verbesserten Wirkungsgrad des Motors zu erreichen, da in Abhängigkeit der Rotorposition im Vergleich zu herkömmlich bestromten Elektromotoren auf den Rotor von den bestromten Motorwicklungen ausgeübte Kräfte optimiert werden können.

Die Zahl von Kontakten an der chirurgischen Maschine läßt sich verringern, wenn ein Elektromotor verwendet wird, der ein sensorloser Elektromotor ist. Ferner sind derartige Motoren wesentlich kostengünstiger in der Herstellung. Denkbar wäre es, daß der Motor Drehzahlerfassungssensoren (nicht Teil der Erfindung) aufweist und daß das erste Steuer- und/oder Regelungsverfahren ein Verfahren zum Steuern und/oder Regeln der chirurgischen Maschine ist, bei welchem die Motorsteuerung Steuersignale für den Elektromotor in Abhängigkeit einer mit den Drehzahlerfassungssensoren ermittelten Ist-Drehzahl bereitstellt. Insbesondere bei niedrigen Drehzahlen läßt sich mit Drehzahlerfassungssensoren eine Ist-Drehzahl des Elektromotors einfach und genau bestimmten.

Besonders einfach wird die Durchführung des Verfahrens, wenn ein Hall-System (nicht Teil der Erfindung) zur Erfassung einer Ist-Drehzahl des Elektromotors vorgesehen ist und wenn das Hall-System die Drehzahlerfassungssensoren umfaßt. Hall-Systeme haben sich in der Praxis in zahlreichen Anwendungen bewährt. Insbesondere können Hall-Sensoren besonders klein ausgebildet und direkt in den Elektromotor integriert werden.

Vorzugsweise bleibt ein Drehzahlgrenzwert zwischen dem mindestens einen unteren und dem mindestens einen oberen Drehzahlbereich beim Betrieb der Maschine unverändert. Das Verfahren wird auf diese Weise maximal vereinfacht.

Gemäß einer weiteren bevorzugten Variante des Verfahrens kann jedoch vorgesehen sein, daß ein Drehzahlgrenzwert zwischen dem mindestens einen unteren und dem mindestens einen oberen Drehzahlbereich im Betrieb der Maschine verändert wird. Dieses Verfahren ermöglicht es, einen Umschaltdrehzahlwert entsprechend einer aktuellen Betriebssituation festzusetzen.

Günstig ist es, wenn eine Umschaltung vom ersten Steuer- und/oder Regelungsverfahren in das zweite Steuer- und/oder Regelungsverfahren bei einer ersten Umschaltdrehzahl erfolgt und wenn eine Umschaltung vom zweiten Steuer- und/oder Regelungsverfahren in das erste Steuer- und/oder Regelungsverfahren bei einer zweiten Umschaltdrehzahl erfolgt. Dadurch lassen sich Umschaltpunkte beim Übergang vom unteren in den oberen Drehzahlbereich, und umgekehrt, in gewünschter Weise einstellen. Insbesondere kann so ein häufiges Umschalten vermieden werden, wenn die Maschine mit Drehzahlen im Bereich der Umschaltdrehzahl betrieben wird. Ein ständiges Umschalten würde den Betrieb der chirurgischen Maschine negativ beeinflussen, insbesondere eine Laufruhe stören.

Vorzugsweise ist die erste Umschaltdrehzahl gleich oder größer als die zweite Umschaltdrehzahl. Damit erfolgt eine Umschaltung vom niedrigen Drehzahlbereich in den hohen Drehzahlbereich vorzugsweise bei einer höheren Drehzahl als umgekehrt.

Vorteilhaft ist es, wenn die Umschaltung vom ersten Steuer- und/oder Regelungsverfahren in das zweite Steuer- und/oder Regelungsverfahren beim Übergang vom mindestens einen unteren in den mindestens einen oberen Drehzahlbereich, oder umgekehrt, automatisch erfolgt. Eine Bedienperson muß in diesem Fall lediglich eine gewünschte Drehzahl für die chirurgische Maschine vorgeben, sich jedoch nicht um eine mögliche Umschaltung zwischen unterschiedlichen Steuer- und/oder Regelungsverfahren kümmern.

Vorteilhaft ist es, wenn bei einem Verfahren zum Steuern einer chirurgischen Maschine mit einem Elektromotor die Maschine ein erstes und ein zweites Betätigungsglied aufweist und wenn durch Betätigen des ersten Betätigungsglieds eine Drehzahl des Elektromotors vorgegeben wird. Unabhängig von einem sonst verwendeten Verfahren zur Ansteuerung des Motors kann so mit dem ersten Betätigungsglied eine Drehzahl zum Betreiben des Antriebs vorgegeben werden.

Besonders einfach wird die Handhabung der chirurgischen Maschine, wenn durch Betätigen des zweiten Betätigungsglieds von einem Rechtslauf des Elektromotors in einen Linkslauf des Elektromotors umgeschaltet wird. Das Umschalten kann erfolgen, wenn das zweite Betätigungsglied dauerhaft gedrückt wird oder solange es gedrückt bleibt oder auch nur durch einmaliges Betätigen des zweiten Betätigungsglieds.

Vorteilhaft ist es, wenn die Maschine in einem Rechts- und/oder Linkslaufbetrieb und/oder einem oszillierenden Betrieb, bei welchem der Elektromotor abwechselnd und jeweils gleichlang in unterschiedlichen Drehrichtungen läuft und/oder einem Pilgerschrittbetrieb betrieben wird, bei welchem der Elektromotor abwechselnd in unterschiedlichen Drehrichtungen betrieben wird, wobei der Elektromotor jeweils etwas länger im Rechtslauf als im Linkslauf betrieben wird. Je nach gewünschtem Einsatzzweck der Maschine kann diese in dem jeweils dafür am besten geeigneten Betriebsmodus betrieben werden. Zum Bohren von Löchern beispielsweise in einem Rechtslaufbetrieb, zum Zurückziehen eines Bohrers beispielsweise in einem Linkslaufbetrieb. Ein oszillierender Betrieb kann beispielsweise bevorzugt angewandt werden beim Bearbeiten von Bohrungen oder beim Einsatz der Maschine als Trephine.. Der beschriebene Pilgerschrittbetrieb ist vorteilhaft, wenn Bohrungen mit Gewinde versehen werden sollen. Es ist so ein schrittweises Schneiden des Gewindes möglich.

Günstig ist es, wenn zum Umschalten des Betriebs der Maschine von einem Rechts- oder Linkslaufbetrieb auf einen oszillierenden Betrieb oder einen Pilgerschrittbetrieb das zweite Betätigungsglied mindestens während einer vorgegebenen Umschaltzeit dauerhaft betätigt wird. Ein Umschalten kann dadurch nicht versehentlich erfolgen, sondern nur gezielt dann, wenn eine Bedienperson das zweite Betätigungsglied mindestens während einer vorgegebenen Umschaltzeit dauerhaft betätigt.

Vorzugsweise wird zum Umschalten vom Rechts- oder Linkslaufbetrieb auf den oszillierenden Betrieb und/oder den Pilgerschrittbetrieb das zweite Betätigungsglied betätigt, während das erste Betätigungsglied unbetätigt ist. Es wird so sichergestellt, daß der Antrieb beim Umschalten stillsteht, das heißt nicht aus vollem Betrieb eine Umschaltung in einen anderen Betriebsmodus erfolgen kann.

Damit sichergestellt werden kann, daß eine Umschaltung zwischen zwei Betriebsmodi nicht versehentlich erfolgt, ist es günstig, wenn als Umschaltzeit eine Zeitdauer in einem Bereich von 2 bis 5 Sekunden vorgegeben wird. Besonders vorteilhaft ist es, wenn eine Umschaltzeit von etwa 3 Sekunden vorgeben wird.

Günstig ist es, wenn der Oszillationsbetrieb oder der Pilgerschrittbetrieb beibehalten wird, auch wenn das zweite Betätigungsglied nach Betätigen während der vorgegebenen Umschaltzeit nicht mehr betätigt wird. Dies erlaubt es, daß sich eine Bedienperson voll auf die Handhabung der Maschine konzentriert, insbesondere auf das Vorgeben einer gewünschten Drehzahl mit einem anderen Betätigungsglied. Vorteilhafterweise wird vom Oszillationsbetrieb oder dem Pilgerschrittbetrieb in den Rechts- oder Linkslaufbetrieb umgeschaltet durch Betätigen des zweiten Betätigungsglieds für eine Zeitdauer, die kürzer als die vorgegebene Umschaltzeit ist. Auf diese Weise kann zwischen verschiedenen Betriebsmodi der Maschine umgeschaltet werden durch nochmaliges kurzes Betätigen des zweiten Betätigungsglieds.

Ferner ist es vorteilhaft, wenn vom Oszillationsbetrieb oder dem Pilgerschrittbetrieb in den Linkslauf umgeschaltet wird durch Betätigen des zweiten Betätigungsglieds für eine Zeitdauer, die kürzer als die vorgegebene Umschaltzeit ist. Frißt sich beispielsweise ein mit der Maschine verbundenes Instrument beim Einsatz der Maschine fest, so kann auf einfache Weise zwischen den beschriebenen Betriebsmodi umgeschaltet werden.

Günstigerweise wird nach dem Umschalten vom Rechts- oder Linkslaufbetrieb in den oszillierenden Betrieb oder den Pilgerschrittbetrieb nach Betätigen des ersten Betätigungsglieds zuerst in den Pilgerschrittbetrieb umgeschaltet. Auf diese Weise kann beispielsweise mit der Maschine ein Loch im Rechtslaufbetrieb gebohrt und der Bohrer im Linkslaufbetrieb zurückgezogen werden. Ferner kann anschließend nach Umschalten in den Pilgerschrittbetrieb beziehungsweise Oszillationsbetriebmodus ein Gewinde in das gebohrte Loch geschnitten werden.

Vorteilhaft ist es, wenn die Maschine im Pilgerschrittbetrieb betrieben wird, solange mit dem ersten Betätigungsglied eine Drehzahl vorgegeben wird, die unterhalb einer Umschaltdrehzahl liegt. Denkbar wäre es auch, die Maschine im Pilgerschrittbetrieb zu betreiben, wenn eine angeforderte Drehzahl oberhalb einer Umschaltdrehzahl liegt.

Ferner kann es günstig sein, wenn die Maschine vom Pilgerschrittbetrieb auf den oszillierenden Betrieb umgeschaltet wird, wenn mit dem ersten Betätigungsglied eine Drehzahl vorgegeben wird, die oberhalb der Umschaltdrehzahl liegt. Dadurch kann ein Umschalten vom Pilgerschrittbetrieb auf den oszillierenden Betrieb einfach durch Vorgeben einer gewünschten Drehzahl erfolgen.

Die nachfolgende Beschreibung bevorzugter Ausführungsformen der Erfindung dient im Zusammenhang mit der Zeichnung der näheren Erläuterung. Es zeigen:
- Figur 1:: eine Explosionsdarstellung eines ersten Ausführungsbeispiels einer chirurgischen Maschine;
- Figur 2:: eine perspektivische Ansicht einer in Figur 1 dargestellten Kupplungsvorrichtung;
- Figur 2a:: eine Draufsicht auf die Kupplungsvorrichtung in Figur 2;
- Figur 3:: eine Schnittansicht längs Linie 3-3 in Figur 2a;
- Figur 4a:: eine Seitenansicht einer Energie- und Steuereinheit;
- Figur 4b:: eine Schnittansicht längs Linie 4-4 in Figur 4a;
- Figur 5a:: eine perspektivische Ansicht eines zweiten Ausführungsbeispiels einer chirurgischen Maschine;
- Figur 5b:: eine Explosionsdarstellung der in Figur 5a dargestellten Maschine;
- Figur 6:: eine Vorderansicht der Maschine in Figur 5a;
- Figur 7:: eine Schnittansicht längs Linie 7-7 in Figur 6;
- Figur 8:: eine Schnittansicht ähnlich Figur 7 der in Figur 5b zerlegten Maschine;
- Figur 8a:: eine vergrößerte Ansicht von in Figur 8 rechts dargestellten Baugruppen;
- Figur 9:: eine Seitenansicht eines dritten Ausführungsbeispiels einer chirurgischen Maschine;
- Figur 10:: eine Stirnansicht der in Figur 9 dargestellten Maschine;
- Figur 11:: eine Explosionsdarstellung der in Figur 9 dargestellten Maschine;
- Figur 12:: eine Schnittansicht längs Linie 12-12 in Figur 10;
- Figur 13:: eine Schnittansicht längs Linie 13-13 in Figur 12;
- Figur 14:: eine Schnittansicht längs Linie 14-14 in Figur 12;
- Figur 15:: eine Schnittansicht längs Linie 15-15 in Figur 12;
- Figur 16:: ein Prinzipschaltbild einer chirurgischen Maschine;
- Figur 17:: eine schematische Darstellung eines zeitlichen Verlaufs des Betriebs einer chirurgischen Maschine;
- Figur 18:: eine weitere Darstellung eines zeitlichen Verlaufs eines Betriebs einer chirurgischen Maschine;
- Figur 19:: eine weitere Darstellung eines zeitlichen Verlaufs eines Betriebs einer chirurgischen Maschine;
- Figur 20:: eine schematische Darstellung einer Drücker-/Sensoranordnung;
- Figur 21:: eine schematische Darstellung eines relativ zum Betätigungssensor bewegten Feldänderungsglieds.
- Figur 22:: eine schematische Darstellung einer chirurgischen Akkumaschine;
- Figur 23:: ein Prinzipschaltbild eines Drei-Phasen-Leistungsinverters;
- Figur 24:: ein Funktionsschema eines Drei-Phasen-Pulsweisten-Modulationsinverters;
- Figur 25:: eine Prinzipdarstellung einer trägerbasierten Pulsweiten-Modulation;
- Figur 26:: eine schematische Darstellung von acht Schaltzuständen bei der Space-Vektor-Puls-Weiten-Modulation;
- Figur 27:: eine schematische Darstellung des Spannungsvektorraums bei der Space-Vektor-Puls-Weiten-Modulations;
- Figur 28:: ein Ablaufdiagramm des Space-Vektor-Puls-Weiten-Modulations-(SVPWM)-Verfahrens zur Steuer- und/oder Regelung eines bürstenlosen Gleichstrommotors;
- Figur 29:: eine schematische Darstellung des Stromverlaufs in einer Motorwicklung in Abhängigkeit der Zeit;
- Figur 30:: eine Darstellung des Stromverlaufs in Abschnitt A in Figur 8 mit zehnfach höherer zeitlicher Auflösung;
- Figur 31:: schematische Darstellung des Spannungsverlaufs einer Motorwicklung bezogen auf Masse der Energieversorgung;
- Figur 32:: der Spannungsverlauf aus Figur 31, jedoch mit zehnfach höherer zeitlicher Auflösung;
- Figur 33:: eine Prinzipskizze einer Akku-/Motorsteuerungseinheit;
- Figur 34:: ein Schaltdiagramm einer Motorsteuerung der in Figur 22 dargestellten Akkumaschine; und
- Figur 35:: ein zum Schaltdiagramm in Figur 13 korrespondierender Ablaufplan für einen Betrieb der in Figur 22 dargestellten Akkumaschine.

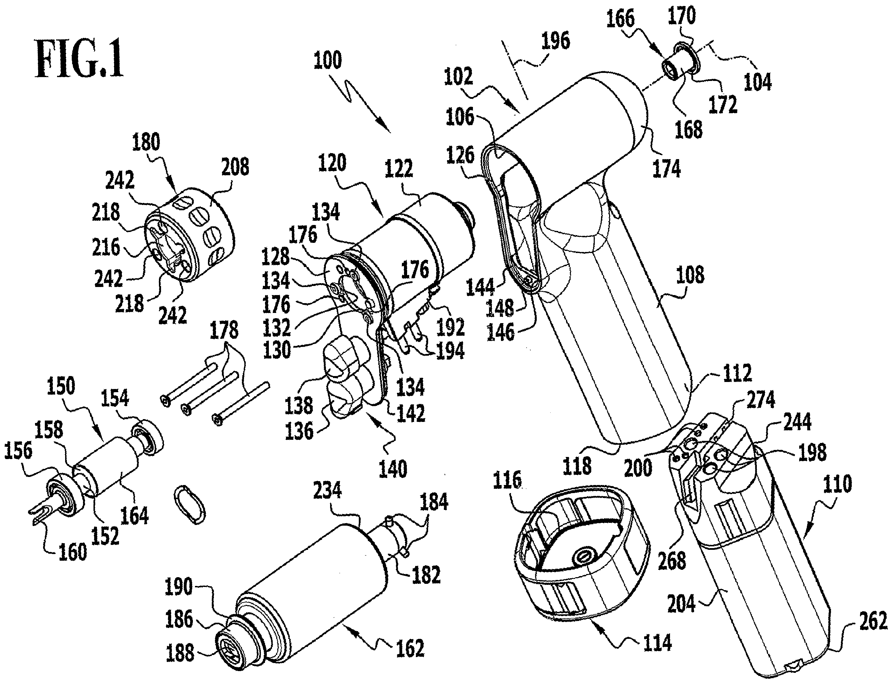

In Figur 1 ist eine insgesamt mit dem Bezugszeichen 100 versehene, in nachfolgend näher beschriebene Baugruppen zerlegte Akkumaschine in Form einer chirurgischen Bohr- oder Fräsmaschine dargestellt. Die Akkumaschine 100 umfaßt ein Gehäuse 102 mit einem eine Längsachse 104 definierenden Motorträger 106 und einem im wesentlichem quer von diesem abstehenden, einstückig angeformten Griff 108, der zur Aufnahme einer Energie- und Steuereinheit 110 dient, welche durch eine vom Motorträger 106 weg weisende Öffnung 112 des Griffs 108 eingeschoben werden kann. Zum Verschließen der Öffnung 112 und Sichern der Energie- und Steuereinheit 110 im Griff 108 dient ein Deckel 114, welcher eine innenliegende Dichtung 116 aufweist, welche bei auf den Griff 108 aufgesetztem Deckel 114 gegen einen die Öffnung 112 umgebenden Rand 118 des Griffs 108 drückt. Das Gehäuse 102 weist im Bereich des Griffs 108 einen im wesentlichen rautenförmigen Querschnitt mit abgerundeten Gehäusekanten auf. Nicht dargestellt ist eine optional im Griff 108 vorgesehene Feder zur Unterstützung des Entfernens der Energie- und Steuereinheit 110 nach Entfernen des Deckels 114.

In den im wesentlichen hohlzylindrischen Motorträger 106 ist von vorne kommend, parallel zur Längsachse 104 eine Antriebseinheit 120 einführbar. Die Antriebseinheit 120 umfaßt einen elektrischen Antrieb in Form eines von einem Motorgehäuse 122 umgebenen, elektrisch kommutierten sensorlosen Gleichstrommotors. Eine stirnseitige, schlüssellochartig geformte Öffnung 126 des Motorträgers 106 sowie des Griffs 108 im Übergangsbereich vom Motorträger 106 zum Griff 108 kann mit einer als Rahmen dienenden, im wesentlichen schlüssellochartig geformten Platte 128 verschlossen werden. An der Platte 128 ist konzentrisch zur Längsachse 104 eine Bohrung 130 vorgesehen. Die Bohrung 130 symmetrisch umgebend sind drei nach vorne weisende Aussparungen 132 an die Bohrung 130 angrenzend vorgesehen, die zur Aufnahme von Köpfen von Schrauben 134 dienen, mit denen die Antriebseinheit 120 koaxial zur Längsachse 104 an der Platte 128 festlegbar ist.

Ferner sind an der Platte 128 zwei Betätigungsglieder in Form eines Gasdrückers 136 und eines Betriebsmodiwahlschalters 138 angeordnet, welche beide parallel zur Längsachse 104 in Richtung auf die Platte 128 hin bewegt werden können und mittels nicht dargestellter Rückstellfedern in ihre von der Platte 128 abstehende Grundstellung überführt werden. Die beiden Betätigungsglieder bilden zusammen mit der Platte 128 eine Drückereinheit 140.

Die Platte 128 weist einen umlaufenden, quer zur Längsachse 104 vorspringenden Rand 142 auf, welcher korrespondierend zu einem einstufig vorspringenden Rand 144 der Öffnung 126 ausgebildet ist. Eine nicht dargestellte, zwischen die Ränder 142 und 144 einzulegende Dichtung stellt einen keimfreien Verschluß der Öffnung 126 mit der ein Verschlußelement bildenden Platte 128 sicher. Ferner ist in der Nähe des Rands 144 von einer Anschlagplatte 146 in Richtung auf die Platte 128 abstehend ein kurzer zylindrischer Zapfen 148 vorgesehen, der in eine nicht dargestellte Ausnehmung der Platte 128 eintaucht um eine Bewegung der Platte 128 quer zur Längsachse 104 zu verhindern.

Ein Rotor 150 des Elektromotors kann aus der Antriebseinheit 120 auch dann entfernt werden, wenn diese an der Platte 128 festgelegt ist, und zwar durch die Bohrung 130 hindurch. Der Rotor 150 umfaßt eine langgestreckte, im wesentlichen zylindrische Lagerwelle 152, auf welcher ein endseitiges Kugellager 154 sowie ein vorderes Kugellager 156 befestigt sind zur drehbaren Lagerung des Rotors 150 im Motorgehäuse 122. Zwischen den Kugellagern 154 und 156 ist auf der Lagerwelle 152 ferner ein Dauermagnet 158 festgelegt, welcher zu diesem Zweck mit einer sich parallel zur Längsachse 104 erstreckenden Längsbohrung versehen ist. Eventuell erforderliche Wuchtelemente zum Auswuchten des Rotors 150 können ebenfalls auf der Lagerwelle 152 befestigt sein. An ihrem vom vorderen Kugellager 156 vorstehenden Ende ist die Lagerwelle 152 in Form eines gabelförmigen Kupplungsstückes 160 ausgebildet, welches formschlüssig in ein identisch ausgebildetes, nicht dargestelltes Kupplungsstück einer mit diesem verbundenen, weiteren Welle eingreifen und diese in Rotation versetzen kann.

Der Dauermagnet 158 kann einteilig oder aus Einzelscheiben zusammengesetzt sein. Auf diese Weise lassen sich Wirbelstromverluste verringern. Durch das Auffädeln des durchbohrten Dauermagneten 158 auf die Lagerwelle 152 wird sowohl die Festigkeit als auch die Biegesteifigkeit des Rotors 150 erhöht. Zum Schutz des Dauermagneten 158 ist eine Rotorarmierung 164 vorgesehen, und zwar in Form einer dünnen, den Dauermagneten 158 umgebenden Hülse aus einem nichtrostenden und nichtmagnetischem Material. Die Rotorarmierung 164 dient zur Erhöhung der mechanischen Festigkeit des Rotors 150, was insbesondere bei sehr hohen Drehzahlen wichtig ist. Ferner bildet die Rotorarmierung 164 einen Korrosionsschutz für den Dauermagneten 158, so daß insbesondere auch korrosionsanfällige Neodym-Eisen-Bor-Magnete verwendet werden können.

Die Kugellager 154 und 156 weisen nicht dargestellte Radialnute am Außenring auf, auf die Dämpfungselemente montiert werden können, wodurch die so entstehende Lagerung zu Wartungszwecken einfacher demontierbar und wieder montierbar ist als ein schwer zugängliches, im Motorgehäuse 122 montiertes Dämpfungselement. Durch Verwendung von Keramik-Kugellagern oder Kugellagern mit einzelnen Keramikbauteilen verbessern sich Notlaufeigenschaften des Motors.

Zum Festlegen der Antriebseinheit 120 im Motorträger 106 dient eine durch eine koaxial zur Längsachse an einem halbkugelig geformten, der Öffnung 126 gegenüberliegenden Ende des Motorträgers 106 vorgesehene Bohrung, durch die eine Befestigungsschraube 166 mit ihrem Außengewindeabschnitt 168 hindurchführbar ist. Ein Kopf 170 der Befestigungsschraube 166 ist mittels eines Dichtrings 172 gegenüber dem Gehäuse 102 fluiddicht abdichtbar.

Der Außengewindeabschnitt 168 ist mit einem nicht dargestellten Innengewindeabschnitt am hinteren Ende des Motorgehäuses 122 verschraubbar. Wird die an der Platte 128 festgelegte Antriebseinheit 120 in den Motorträger 106 hineingeschoben, dann wird sie durch Verschrauben mit der Befestigungsschraube 166 gegen das hintere Ende 174 des Motorträgers 106 gezogen und damit der Rand 142 gegen den Rand 144. So kann allein durch Lösen der Befestigungsschraube 166 die Platte 128 mit der Antriebseinheit 120 vom Gehäuse 102 gelöst und aus diesem herausgenommen werden.

Benachbart der Aussparungen 132 sind an der Platte 128 drei Gewindebohrungen 176 vorgesehen, deren Innengewinde zu Außengewindeabschnitten von langschäftigen Schrauben 178 korrespondieren, mit denen eine Kupplungsvorrichtung 180 an der Platte 128 festlegbar ist. Die Kupplungsvorrichtung 180 dient zur Verbindung eines Kupplungsstücks 182 mit drei von diesem radial abstehenden Kupplungselementen 184 in Form kurzer zylindrischer Stifte. Das Kupplungsstück 182 bildet ein hinteres Ende eines Getriebeblocks 162, an dessen vorderem Ende ein Kupplungsstück 186 mit einer Werkzeugaufnahme 188 zum Aufnehmen eines Instruments oder Werkzeugs zum Verbinden mit dem Getriebeblock 162 vorgesehen ist, wobei durch einen nicht näher beschriebener Schnellspannmechanismus durch Zurückziehen einer äußeren Betätigungshülse 190 mit Ringflansch in Richtung auf die Kupplungsvorrichtung 180 hin ein Werkzeug aus der Werkzeugaufnahme 188 gelöst werden kann.

Vom Motorgehäuse 122 steht ein Kontaktblock 192 in Richtung auf den Griff 108 ab, von welchem wiederum drei parallel zueinander ausgerichtete Motorkontakte 194 abstehen, die jeweils mit zwei von insgesamt drei Motorwicklungen 199 verbunden sind. Die Motorkontakte 194 sind in Form hohler Kontaktstifte realisiert, die Querbohrungen aufweisen, durch die die Motorwicklungen hindurchgefädelt und verlötet werden. Der Kontaktblock 192 dient als Träger für die Motorkontakte 194 und ist aus einem hochtemperaturbeständigen Kunststoff hergestellt. Er ist ferner relativ zum Motorgehäuse 122 abgedichtet. Die Motorkontakte 194 sind parallel zu einer Längsachse 196 des Griffs 108 ausgerichtet.

Die entsprechend dem innen unrunden Griff 108 geformte Energie- und Steuereinheit 110 weist drei zu den Motorkontakten 194 korrespondierende Verbindungskontakte 198 auf, die die abstehenden Motorkontakte 194 beim Einschieben der Energie- und Steuereinheit 110 in den Griff 108 eintauchen. Ein sicherer Kontakt wird erreicht, indem die Verbindungskontakte 198 beschränkt beweglich, d. h. schwimmend, in der Energie- und Steuereinheit 110 eingebaut sind. Damit können Maß-, Form- und Lagetoleranzen der Verbindungskontakte 198 relativ zu den Motorkontakten 194 ausgeglichen werden. Übergangswiderstände zwischen den Verbindungskontakten 198 und den Motorkontakten 194 werden dadurch definiert vorgegeben. Des weiteren kann die Energie- und Steuereinheit 110 leicht in den Griff 108 eingeschoben und auch wieder aus diesem gelöst werden. Ferner sind an der Energie- und Steuereinheit 110 vier Ladekontakte 200 vorgesehen, über die die Energie- und Steuereinheit 110 mittels Anschlußkabeln oder über eine Direktverbindung mit einem Ladegerät 202 verbunden werden kann zum Laden von in einem hülsenartigen Gehäuse 204 der Energie- und Steuereinheit 110 angeordneten Akkuzellen 206.

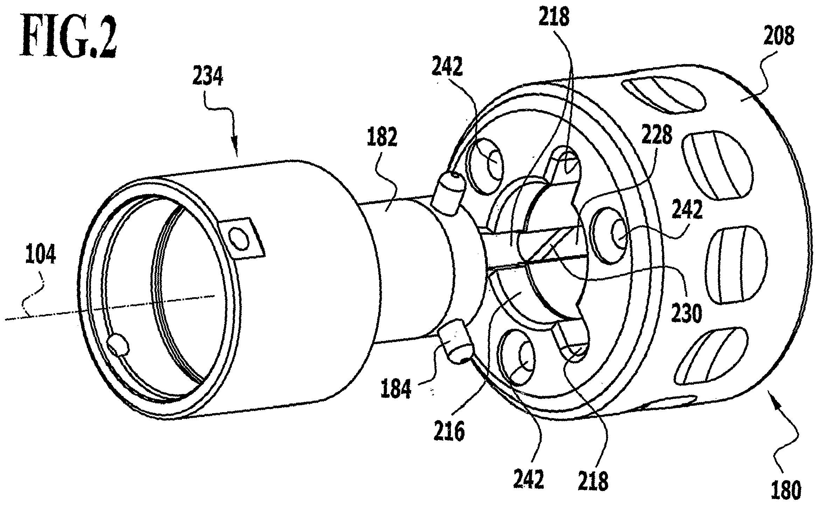

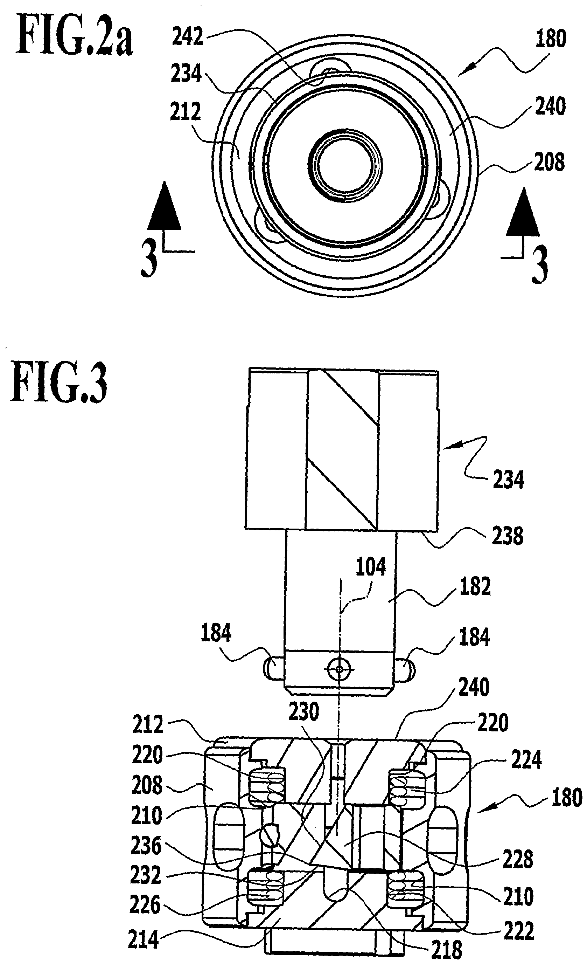

Die Funktion der Kupplungsvorrichtung 180 wird im Zusammenhang mit den Figuren 2, 2a und 3 näher erläutert.

Die Kupplungsvorrichtung 180 umfaßt einen Drehring 208, welcher zwei symmetrisch angeordnete, in Richtung auf die Längsachse 104 geöffnete Ringnute 210 aufweist. In den Drehring 208 sind von beiden Seiten rotationssymmetrische Kupplungskörper 212 und 214 eingesetzt, die eine die Kupplungsvorrichtung 180 koaxial zur Längsachse 104 durchsetzende hohlzylindrische Kupplungsaufnahme 216 definieren und drehfest miteinander verbunden sind. Die Kupplungsaufnahme 216 dient zur Aufnahme des Kupplungsstücks 182. Drei sich ausgehend von der Kupplungsaufnahme 216 sich parallel zur Längsachse 104 erstreckende Kupplungsaufnahmen 218 dienen zur Aufnahme der Kupplungselemente 184, sodaß diese von vorn kommend seitlich in die Kupplungsaufnahme 218 eintauchen. Ferner sind an den Kupplungskörpern 212 und 214 den Ringnuten 210 gegenüberliegende Aussparungen 220 und 222 vorgesehen, die mit diesen zusammen zwei Ringräume definieren, in denen jeweils eine Spiralfeder 224 und 226 gelagert ist. Jeweils ein Ende der Spiralfedern 224 und 226 ist drehfest mit dem Drehring 208 verbunden, das jeweils andere Ende der Spiralfedern 224 und 226 mit den Kupplungskörpern 212 und 214. Die Kupplungsaufnahmen 218 durchsetzen nicht die gesamte Kupplungsvorrichtung 180, sondern enden blind im Kupplungskörper 214.

Mit dem Drehring 208 sind drehfest drei Verriegelungselemente 228 verbunden, die bei einer Verdrehung des Drehrings 208 relativ zu den Kupplungskörpern 212 in Umfangsrichtung bewegt werden. Sie weisen zwei jeweils eine Kupplungsaufnahme 218 quer durchsetzende Kanten auf, nämlich eine erste Aufgleitkante 230 und eine zweite Aufgleitkante 232. Die erste Aufgleitkante 230 ist relativ zur Längsachse 104 um einen Winkel von etwa 30° gedreht, die zweite Aufgleitkante 232, die in Richtung auf die Platte 128 hinweist, um einen Winkel von annähernd 90°.

Wird das Kupplungsstück 182, welches an einer Endhülse 234 des Getriebeblocks 162 angeordnet ist, so in die Kupplungsaufnahme 216 eingeführt, daß die Kupplungselemente 184 in die Kupplungsaufnahme 218 eintauchen, dann stoßen die Kupplungselemente 184 zunächst an der ersten Aufgleitkante 230 an. Wird das Kupplungsstück 182 weiter in die Kupplungsaufnahme 216 hineinbewegt, dann gleitet die erste Aufgleitkante 230 an den Kupplungselementen 184 auf, wodurch diese entgegen den Federkräften der Spiralfedern 224 und 226 in Umfangsrichtung zusammen mit dem Drehring 208 gegen die Kupplungskörper 212 und 214 verschoben werden. Sobald die Kupplungselemente 184 über eine die beiden Aufgleitkanten 230 und 232 miteinander verbindende Kante 236 hinweggeschoben werden, zwingen die Spiralfedern 224 und 226 die Verriegelungselemente 228 wieder in Umfangsrichtung in ihre Grundstellung zurück. Die zweite Aufgleitkante 232 wird durch die Federkräfte an den Kupplungselementen 184 entlangbewegt und zwingt diese noch weiter in die Kupplungsaufnahme 218. Eine auf die Kupplungsvorrichtung 180 hin weisende Ringkante 238 der Endhülse 234 schlägt an einer Stirnfläche 240 der Kupplungsvorrichtung 180 an und die zweite Aufgleitkante 232 hält die Kupplungselemente 184 derart unter Zug parallel zur Längsachse 104, daß die Ringkante 238 spielfrei an der Stirnfläche 240 gehalten wird.

Zum Lösen des Getriebeblocks 162 muß nur der Drehring 208 entgegen den Federkräften der Spiralfedern 224 und 226 relativ zu den Kupplungskörpern 212 und 214 verdreht werden, bis die Verriegelungselemente 228 die Kupplungsaufnahmen 218 freigeben, sodaß die Kupplungselemente 184 wieder parallel zur Längsachse 104 aus diesen herausgezogen werden können.

Die Kupplungskörper 212 und 214 sind mit drei parallel zur Längsachse 104 verlaufenden Durchgangsbohrungen 242 versehen, durch die die Schrauben 178 hindurchgesteckt und mit den Gewindebohrungen 176 der Platte 128 verschraubt werden können. Die Kupplungsvorrichtung 180 läßt sich auf diese Weise auch dann von der Platte 128 lösen, wenn diese mit dem Gehäuse 102 verbunden ist. Wird die Kupplungsvorrichtung 180 von der Platte 128 gelöst, dann kann der Rotor 150 aus der Antriebseinheit 120 herausgezogen werden. Zur Wartung der Kugellager 154 und 156 muß daher nicht die Platte 128 gelöst werden, das Entfernen der Kupplungsvorrichtung 180 genügt.

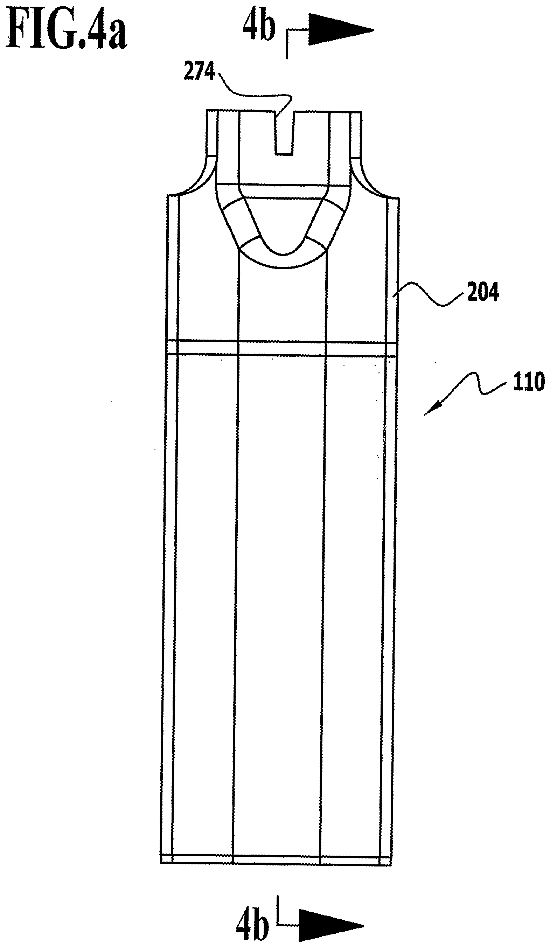

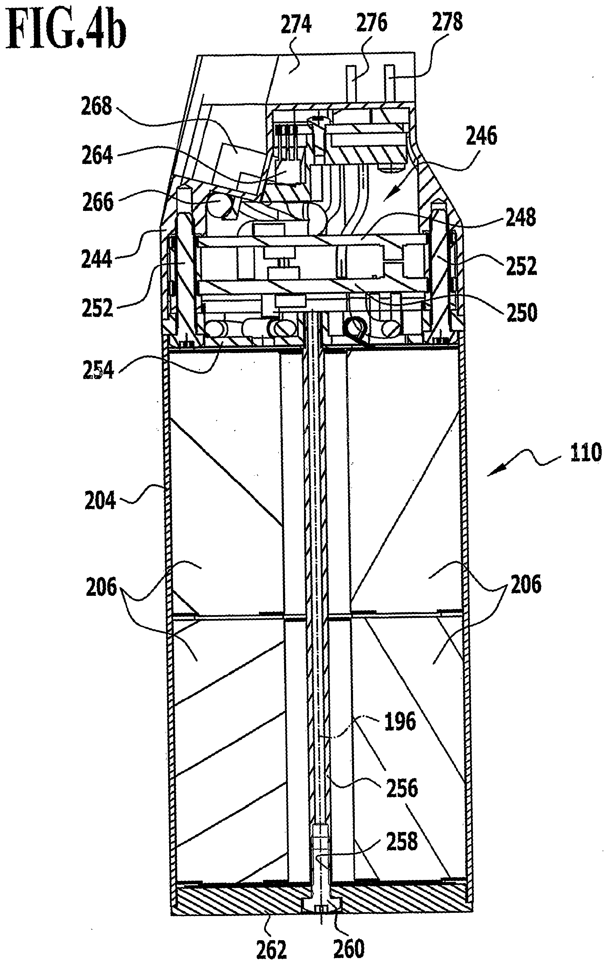

Der Aufbau der Energie- und Steuereinheit 110 wird in Verbindung mit den Figuren 4a und 4b näher erläutert.

In einem in Richtung auf den Motorträger 106 hin weisenden Endbereich des Gehäuses 204, dem sogenannten Steuerungsgehäuse 244, ist eine Motorsteuerung 246 angeordnet, die insbesondere die in Figur 4b dargestellten Leiterplatten 248 und 250 umfaßt, auf denen die zur Steuerung der Akkumaschine 100 erforderlichen elektronischen Bauelemente angeordnet sind. Das Steuerungsgehäuse 244 ist mit dem Gehäuse 204 fest verbunden. Zur Sicherung der Motorsteuerung 246 im Steuerungsgehäuse 244 dienen Schrauben 252, mit denen eine die Motorsteuerung 246 von den Akkuzellen 206 trennende Trennplatte 254 mit dem Steuerungsgehäuse 244 verschraubt werden kann. Von der Trennplatte 254 steht parallel zur Längsachse 196, das Gehäuse 204 bis zu dessen Ende durchsetzend ein hohler Stab 256 ab, welcher an seinem von der Motorsteuerung 246 weg weisenden Ende mit einem Innengewindeabschnitt 258 versehen ist. Mittels einer Schraube 260 kann ein zum Verschließen des Gehäuses 204 passender Gehäusedeckel 262 mit dem Gehäuse 204 verbunden werden, welches an seinem anderen Ende mit dem Steuerungsgehäuse 244 verbunden ist. Die Energie- und Steuereinheit 110 ist bis auf die herausgeführten Verbindungskontakte 198 und Ladekontakte 200 vollständig gekapselt.

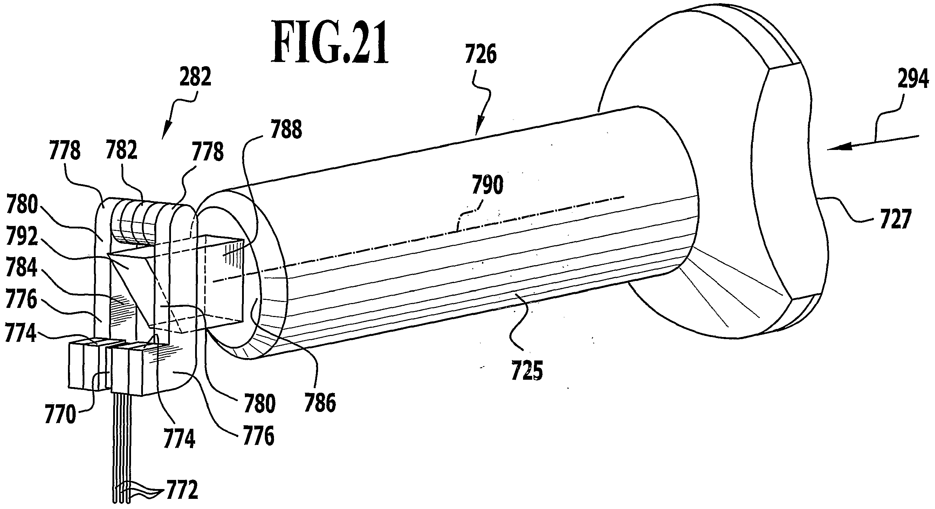

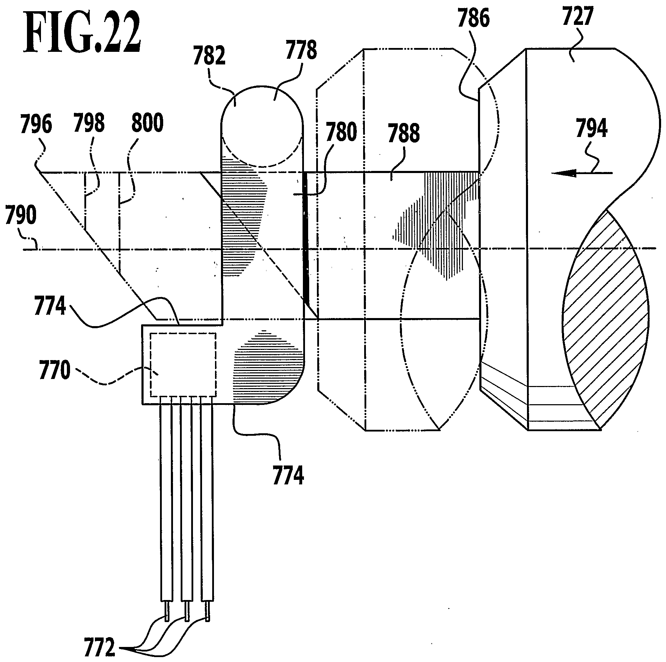

Insbesondere sind innerhalb des Steuerungsgehäuses 244 ein Hallsensor 264 (nicht Teil der Erfindung) sowie ein mit diesem über ein weiter unten im Zusammenhang mit den Figuren 21 und 22 näher beschriebenes Rückschlußsystem gekoppelter Stabmagnet 266 angeordnet. Das Rückschlußsystem ist beidseitig eines Spalts 268 im Steuerungsgehäuse 244 angeordnet, sodaß ein am Gasdrücker 136 angeordneter magnetischer Weicheisenquader 270 in eine vom Rückschlußsystem gebildete Öffnung 272 eintauchen und einen magnetischen Fluß im Rückschlußsystem ändern kann. Auf diese Weise kann ohne direkte elektrische Verbindung zwischen dem Gasdrücker 136 und der Energie- und Steuereinheit 110 beispielsweise ein Drehzahlvorgabesignal an die Energie- und Steuereinheit 110 übermittelt werden.

Des weiteren ist an einer Oberseite des Steuerungsgehäuses 244 ein parallel zum Spalt 268 angeordneter schmaler Schlitz 274 vorgesehen. Beidseits des Schlitzes sind zwei diesen durchsetzende Lichtschranken 276 und 278 vorgesehen, die entweder mittels sichtbarem Licht betrieben werden, wobei dann entsprechende Aussparungen im Steuerungsgehäuse 244 vorgesehen sind, oder die durch Infrarotlichtschranken gebildet werden, wobei dann keine Durchbrechungen des Steuerungsgehäuses 244 erforderlich sind. Die Lichtschranke 276 dient als Betriebsmodusumschaltsensor, (nicht Teil der Erfindung), welcher von einem in das Gehäuse 102 hineinragenden Ende des Betriebsmodiwahlschalters 138 berührungslos betätigt werden kann, nämlich wenn das Ende so weit in den Schlitz 274 hineinbewegt wird, daß die Lichtschranke 276 unterbrochen wird.

Die Lichtschranke 278 dient als Betriebsmodusaktivierungssensor, (nicht Teil der Erfindung), welcher von einem nicht dargestellten Betriebsmodusaktivierungsbetätigungsglied berührunglos betätigbar ist. Dieses kann beispielsweise an der Antriebseinheit 120 angeordnet sein oder auch nicht, wobei es bei Vorhandensein in den Schlitz 274 eintaucht und die Lichtschranke 278 unterbricht. Damit kann zum Beispiel ein bestimmter Betriebsmodus gezielt aktiviert oder deaktiviert werden, insbesondere ein Oszillationsbetrieb der Akkumaschine 100.

Die beschriebene Energie- und Steuereinheit 110 kann für unterschiedliche Maschinen verwendet werden, insbesondere auch für die nachfolgend beschriebenen Ausführungsbeispiele und nicht nur für die Akkumaschine 100. Insbesondere kann die Energie- und Steuereinheit 110 kürzer als in Figur 4b dargestellt ausgebildet werden, insbesondere dann, wenn nur zwei Akkuzellen 206 unterhalb der Trennplatte 254 angeordnet sind. Das Gehäuse 204 kann dann entsprechend gekürzt werden. Ebenso kann dann auch der Griff 108 des Gehäuses 102 entsprechend verkürzt werden. Insbesondere wenn Anwender leichtere Maschinen wünschen oder geringere Kapazitäten zum Betrieb der Akkumaschine 100 erforderlich sind, bietet sich eine Verkürzung des Griffs 108 in der beschriebenen Weise an.

Durch das Vorsehen des Gehäuses 204 in Verbindung mit dem lösbaren Gehäusedeckel 262 sind die Akkuzellen 206 direkt zugänglich und damit leicht austauschbar, was aufgrund der in der Regel begrenzten Lebensdauer der Akkuzellen 206 regelmäßig erforderlich ist.

Wie bereits angedeutet, sind sämtliche elektronischen Bauelemente der Akkumaschine 100, insbesondere die Motorsteuerung 246, in der Energie- und Steuereinheit 110 untergebracht, die nicht sterilisiert wird. Die empfindliche Elektronik ist daher keinen schädlichen Umgebungsbedingungen, insbesondere Hitze und Feuchte in einem Sterilisator, ausgesetzt. Des weiteren ist es nicht erforderlich, diese über die Maßen vor Verschmutzung zu schützen oder zusätzlich zu kapseln. Die Elektronik der Akkumaschine 100 kann neben der Motorsteuerung 246 ferner die beschriebene Sensorik (nicht Teil der Erfindung) zur Drehzahl-, Drehrichtungs- und Betriebsmodussteuerung enthalten. Ferner können Überwachungselemente für die Akkuzellen 206 sowie Kommunikationselemente zum Ladegerät 202 vorgesehen sein. Da die gesamte Sensorik der Akkumaschine 100 berührungslos ausgeführt ist, sind an der Energie- und Steuereinheit 110 keine beweglichen mechanischen Elemente vorgesehen. Insbesondere trägt die Energie- und Steuereinheit 110 keinerlei mechanische Elemente für die Rückstellung der Betätigungsglieder, also insbesondere des Gasdrückers 136 und des Betriebsmodiwahlschalters 138, wodurch sich die Betätigungskräfte für die Betätigungsglieder minimieren lassen. Die Motorsteuerung 246 umfaßt programmierbare Hall-IC's wodurch sich bei einem leichten Drücken, beispielsweise des Gasdrückers 136, zunächst ein Anstieg der Drehzahl proportional zum Drückerweg des Gasdrückers 136 ergibt und erst danach ein überproportionaler Anstieg der Motordrehzahl bis zum Drehzahlmaximum.

Um den Stromverbrauch der Akkuzellen 206, insbesondere bei einer nicht benutzten Maschine, zu minimieren, also den Ruhestromverbrauch möglichst gering zu halten und damit einer schleichenden Entladung entgegenzuwirken, wird die Energie- und Steuereinheit 110 nur unter bestimmten Bedingungen in einen betriebsfähigen Zustand versetzt. Dies kann beispielsweise der Fall sein, wenn die Energie- und Steuereinheit 110 in den Griff 108 eingeschoben ist und die Verbindungskontakte 198 in Kontakt mit den Motorkontakten 194 stehen. Um diese Bedingung zu überprüfen, wird die Motorsteuerung 146 periodisch, beispielsweise in einem Abstand von einer Sekunde bis zu zehn Sekunden, vorzugsweise 6 Sekunden, für wenige Millisekunden aktiviert und fragt die vorgegebenen Aktivierungsbedingungen ab. Treffen diese nicht zu, wird die Motorsteuerung 246 wieder deaktiviert.

Als Akkuzellen 206 werden Zellenblöcke verschiedenster Technologie verwendet. Durch Auswahl von Akkuzellen 206 geeigneter Größe sowie Reihenschaltung von einzelnen Akkuzellen 206 werden Akkupacks geschaffen, die sowohl die erforderlichen Abmessungen aufweisen als auch eine vorgegebene Nominalspannung von vorzugsweise 14,4 Volt. Dies ermöglicht es, je nach Einsatzprofil, Zellen mit Lithium-Ionen, Lithium-Polymer, NiMH und NiCd-Technologie einzusetzen. Beispielsweise können zwölf NiMH-Zellen mit je 1,2 Volt und damit einer Gesamtspannung von 14,4 Volt in Fällen eingesetzt werden, bei denen es auf eine Hohe Kapazität ankommt. Dagegen können alternativ vier Lithium-Ionen-Zellen mit einer Nominalspannung von jeweils 3,6 Volt also zusammen ebenfalls 14,4 Volt, für Einsätze zur Anwendung kommen, bei denen es auf ein besonders niedriges Gewicht der Akkumaschine 100 ankommt. Ferner können auch Lithium-Ionen-Zellen auf Mangan-Basis verwendet werden, wodurch auf eine externe Schutzbeschaltung der Energie- und Steuereinheit 110 verzichtet werden kann.

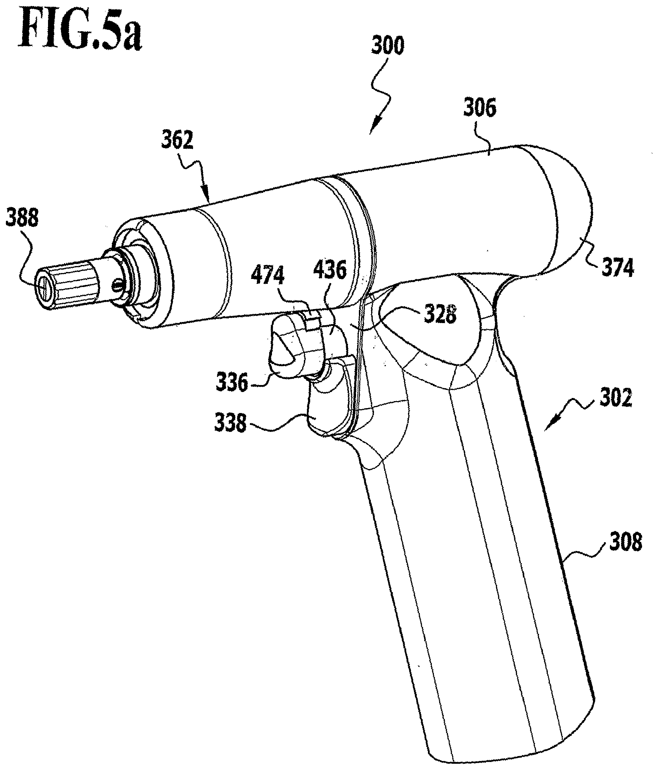

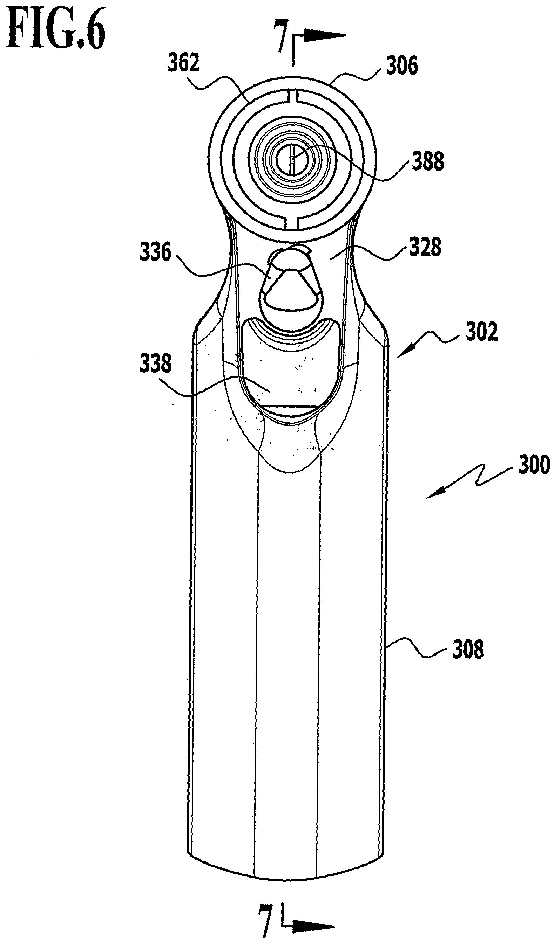

Ein zweites Ausführungsbeispiel einer chirurgischen Maschine in Form einer Stichsäge 300 ist in den Figuren 5a bis 8a dargestellt. Ein Großteil der Bauelemente und Baugruppen der Stichsäge 300 stimmt mit Bauelementen oder Baugruppen der Akkumaschine 100 überein, was leicht durch Vergleich der Figuren erkennbar ist. Insbesondere identisch sind die Gehäuse 102 und 302 sowie die in den Figuren 5a bis 8a nicht dargestellte Energie- und Steuereinheit 110, die in analoger Weise wie bei der Akkumaschine 100 ausgebildet ist und mit einem nicht dargestellten, zum Deckel 114 identischen Deckel im Griff 308 des Gehäuses 302 gehalten werden kann.

Das Gehäuse 302 umfaßt einen eine Längsachse 304 definierenden Motorträger 306 sowie einen von diesem im wesentlichen quer abstehenden, eine Längsachse 396 definierenden Griff 308. In eine auf der Vorderseite des Gehäuses 302 im Bereich des Motorträgers 306 sowie des Übergangsbereichs zum Griff 308 definierten Öffnung 326 ist eine Antriebseinheit 320 parallel zur Längsachse 304 einschiebbar. Sie umfaßt ein Motorgehäuse 322, welches an einer zur Öffnung 326 korrespondierend ausgebildeten Platte 328 lösbar festlegbar ist. Das Motorgehäuse 322 ragt durch eine kreisförmige Durchbrechung der Platte 328 mit einem hülsenförmigen Abschnitt 324 etwas hindurch. Ein Gasdrücker 336 ist an der Platte 328 beweglich gelagert und an einer Position angeordnet, an der bei der Akkumaschine 100 der Betriebsmodiwahlschalter 138 vorgesehen ist. Ferner ist an der Position, an der bei der Akkumaschine 100 der Gasdrücker 136 vorgesehen ist, eine Verkleidung 338 vorgesehen, sodaß im wesentlichen die Platten 128 und 328 identisch sind. Die Platte 328 mit dem Gasdrücker 326 bildet ein Drückereinheit 340. Ein Rand 342 der Platte 328 wird mittels einer nicht dargestellten Dichtung gegen den Rand 344 der Öffnung 326 keimdicht abgedichtet. Eine Anschlagplatte 346 entsprechend der Anschlagplatte 126 am unteren Ende der Öffnung 326 begrenzt eine Bewegung der Platte 328 auf das Gehäuse 302 hin.

Die in den Motorträger 306 eingeschobene Antriebseinheit 320 kann mit einer der Befestigungsschraube 166 entsprechenden Befestigungsschraube 366 festgelegt werden, die hierzu einen Außengewindeabschnitt 368 und einen Kopf 370 aufweist, der als Anschlag für ein hinteres Ende 374 des Motorträgers 306 dient und gegenüber dem hinteren Ende 374 mit einem Dichtring 372 abgedichtet ist zum keimfreien Verschließen einer im hinteren Ende 374 vorgesehenen Öffnung 375.

Vom Motorgehäuse 322 steht in Richtung auf den Griff 308 weisend ein Kontaktblock 392 ab, von dem wiederum drei, den Motorkontakten 194 identische Motorkontakte 394 parallel zur Längsachse 396 in Form von vergoldeten Kontaktstiften in Richtung auf den Griff 308 abstehen.

Ein Rotor 350 ist Teil der Antriebseinheit 320 und aus dieser herausnehmbar, wenn ein Getriebeblock 362, der den zylindrischen Abschnitt 324 umgreifend an der Platte 328 festlegbar ist, von der Platte 328 entfernt wird. Der Rotor 350 ist im wesentlichen identisch mit dem Rotor 150 aufgebaut und umfaßt eine Lagerwelle 352, einen auf diese aufgesteckten, zylindrischen und mit einer zur Lagerwelle 352 korrespondierenden Bohrung 359 versehenen Dauermagneten 358. An einem in Richtung auf das hintere Ende 374 weisenden Ende der Lagerwelle 352 ist ein Kugellager 354 in Form eines Ringlagers festgelegt. Vor und hinter dem Dauermagnet 358 sind manschettenartige Klemmelemente 400 und 402 vorgesehen, die einerseits einen Korrosionsschutz zusammen mit einer dünnen, hülsenartigen Rotorarmierung 364 aus amagnetischem Material bilden, zum anderen den Dauermagneten 358 auf der Lagerwelle 352 halten. Ein vorderes Ende der Lagerwelle 352 bildet ein Kupplungsstück 360, welches eine quer zur Längsachse 304 angeordnete Aussparung 361 aufweist, in die ein Klemmkörper 363 eingesetzt ist und der in eine Nut eines Antriebsrads 404 des Getriebeblocks 362 einschiebbbar ist und als Mitnehmer für das Antriebsrad 404 dient.

Am vorderen Ende des Getriebeblocks 362 ist eine Werkzeugaufnahme 388 koaxial zur Längsachse 304 vorgesehen, in welche zum Beispiel ein Sägeblatt einschiebbar und mit einer Schraube 406 festlegbar ist.

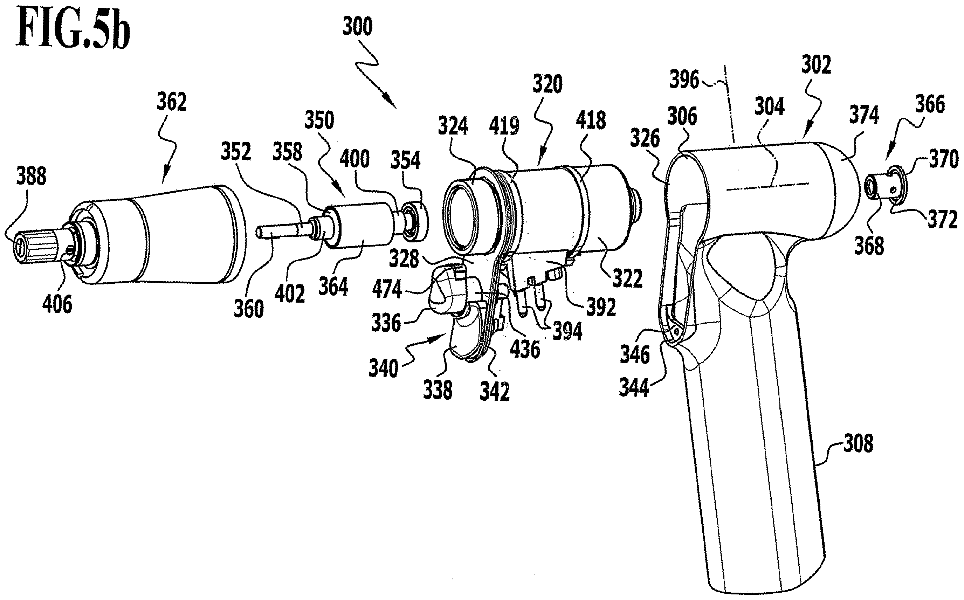

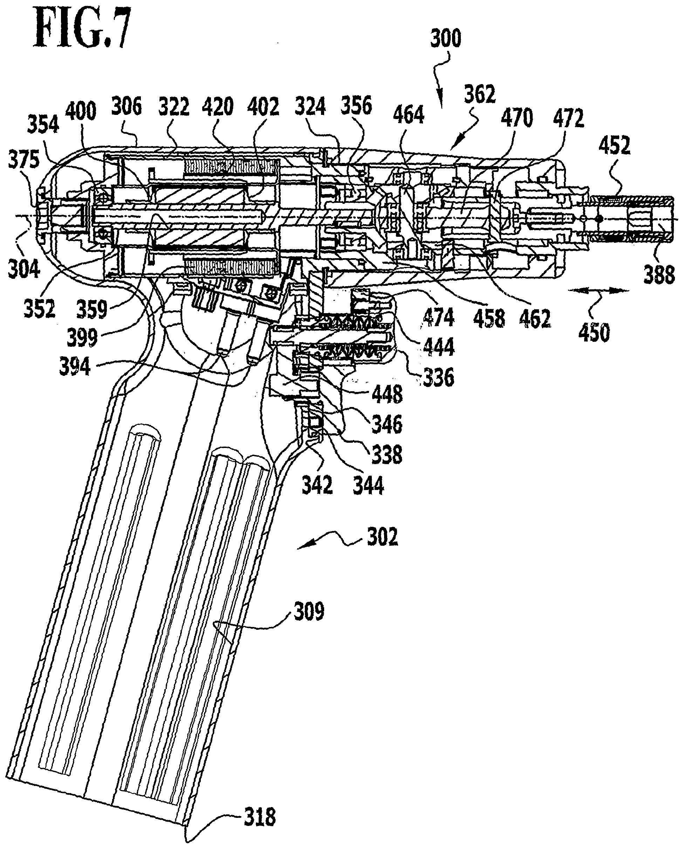

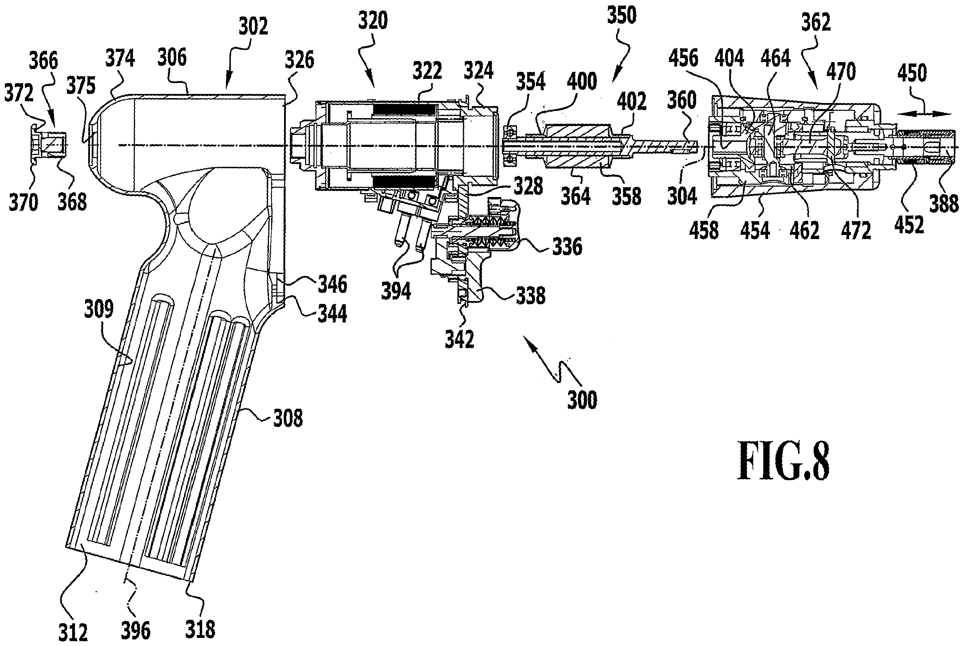

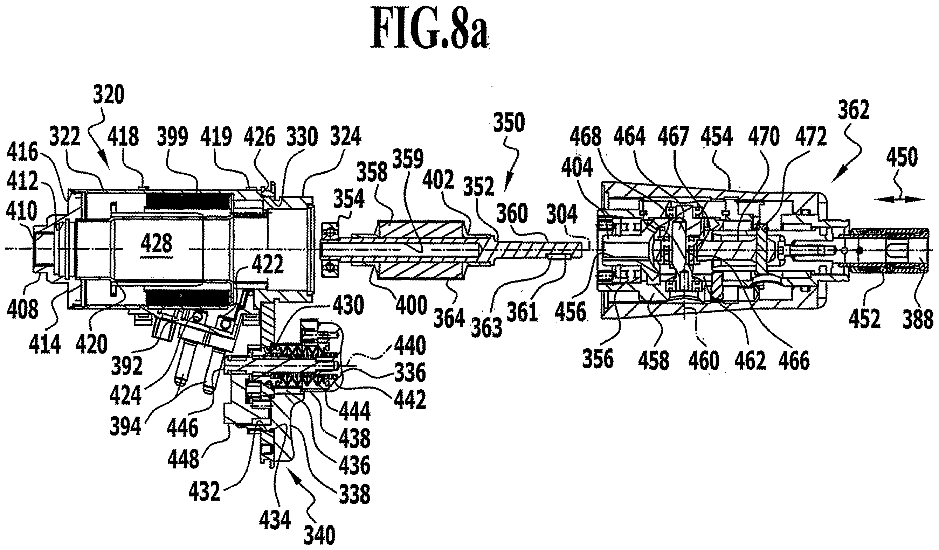

Ein innerer Aufbau der in den Figuren 5a und 5b beschriebenen Bauelemente und Baugruppen der Stichsäge 300 wird nachfolgend im Zusammenhang mit den Figuren 7, 8 und 8a näher erläutert.

Ein im Griff 308 gebildeter Aufnahmeraum 309, in den die Energie- und Steuereinheit 110 einschiebbar ist, ist, wie bereits erwähnt mit einem dem Deckel 114 entsprechenden Deckel verschließbar, wobei die in den Deckel 114 eingelegte umlaufende Dichtung 116 gegen einen unteren Rand 318 des Gehäuses 302 drückt und diesen so keimdicht verschließt. Insgesamt weist das Gehäuse drei Öffnungen auf, nämlich eine Öffnung 312 zum Einschieben der Energie- und Steuereinheit 110 in den Griff 308, die Öffnung 375 im hinteren Ende 374 des Motorträgers 306 zum Einsetzen der Befestigungsschraube 366 sowie die Öffnung 326 zum Einführen der Antriebseinheit 320 in das Gehäuse 302. Verschlußelemente für die Öffnungen bilden somit die Befestigungsschraube 366, der nicht dargestellte Deckel 114 sowie die Platte 328. Jeweils sind Dichtungselemente vorgesehen, um die Öffnungen 312, 374 und 326 mit den beschriebenen Verschlußelementen keimdicht verschließen zu können und die Stichsäge 300, wie auch die Akkumaschine 100, nach Einsetzen der nicht sterilen Energie- und Steuereinheit 110 für den Einsatz in einem sterilen Bereich, beispielsweise einem Operationssaal, vorzubereiten.

Am hinteren Ende der Antriebseinheit 320 ist ein hülsenförmiger Verbindungsstutzen 408 angeordnet, der ein Innengewinde 410 aufweist, welches zum Außengewinde des Außengewindeabschnitts 368 korrespondierend ausgebildet ist. Der Verbindungsstutzen 408 erweitert sich zweimal jeweils einstufig, wobei die zweite Erweiterung 412 zur Aufnahme des Kugellagers 354 dient. An die Erweiterung 412 schließt sich ein radial nach außen abstehender Ringflansch 414 an, welcher in ein hinteres Ende des Motorgehäuses 322 eingesetzt und mit einer Ringdichtung 416 abgedichtet ist. Am Motorgehäuse 322 sind dieses umgebend außen zwei Abstandsringe 418 und 419 vorgesehen, die bei eingesetzter Antriebseinheit 320 innen am Motorträger 306 anliegen. Sie sind vorzugsweise aus einem dämpfenden Material hergestellt. Sie dienen ferner dazu, den Kontaktblock 392 unten am Motorgehäuse 322 zu halten.

Koaxial zur Längsachse 304 ist ein hülsenartiger Wickelkörper in das Motorgehäuse 322 eingesetzt, auf den drei Motorwicklungen 399 aufgewickelt sind. Ein in das Motorgehäuse 322 hineinragendes Ende der Motorkontakte 394 ist mit einer Querbohrung 424 versehen, durch die die Motorwicklungen hindurchgeführt und verlötet sind. Ein Anschlußbereich der Motorwicklungen 399 kann durch das Durchfädeln daher sehr kurz gehalten werden. Der vordere Abschnitt 324 des Motorgehäuses 322 ragt durch eine dafür vorgesehene Bohrung 330 der Platte 328 hindurch. Ein ringförmiger Anschlag 426 am Motorgehäuse 322, gebildet durch eine einstufige Verringerung des Außendurchmessers beim Übergang zum Abschnitt 324 liegt direkt an der Platte 328 an.

Alle elektrisch leitfähigen Elemente des Motorgehäuses 322 sind vollständig vergossen, insbesondere die zwischen dem Wickelkörper 420 und dem Motorgehäuse 322 angeordneten Motorwicklungen 399 sowie die in dem Kontaktblock 392 gehaltenen Motorkontakte 394. Ein rotationssymmetrischer Aufnahmeraum 428 im Inneren der Antriebseinheit 320 dient zur Aufnahme des bereits näher beschriebenen Rotors 350.

Zur Aufnahme des Gasdrückers 336 und der Verkleidung 338 sind übereinander zwei Bohrungen 430 und 432 parallel zur Längsachse 304 an der Platte 328 vorgesehen. Die in die Bohrung 432 eingesetzte Verkleidung 338 ist mit einer Ringdichtung 434 relativ zur Platte 328 keimdicht abgedichtet. Sie weist außen eine ergonomisch geformte Außenfläche auf, auf welcher eine Bedienperson vorzugsweise ihren Mittelfinger und/oder Ringfinger abstützen kann.

Der Gasdrücker 336 umfaßt einen hülsenförmigen, durch die Bohrung 430 hindurchgeführten feststehenden Lagerkörper 436, an dem ein koaxial zu einer Längsachse 440 der Bohrung 430 bewegliches Drückerelement 438 vorgesehen ist, welches durch eine Schraubenfeder 442 in einer von der Platte 328 abstehenden Grundstellung gehalten wird. Eine einerseits am Lagerkörper 436 und andererseits am Drückerelement 438 festgelegte Faltenbalgdichtung 444 stellt eine keimfreie Abdichtung des Gasdrückers 336 relativ zur Platte 328 sicher.

Das Drückerelement 438 umfaßt einen die Bohrung 430 durchsetzenden Stift, an dessen in das Gehäuse 302 hineinragendem Ende nach unten ein Plättchen 448 absteht, das eine Breite aufweist, damit es in den Spalt 268 der Energie- und Steuereinheit 110 geschoben werden kann. Es dient zum Betätigen des Hall-Sensors 264 (nicht Teil der Erfindung) des Drehzahlvorgabesensors 282 (nicht Teil der Erfindung) der Energie- und Steuereinheit 110. Das Weicheisenplättchen 448 ist bei der Stichsäge 300 so weit nach unten geführt, daß es an der gleichen Position in den Spalt 268 eintauchen kann, wie ein am Gasdrücker 136 der Akkumaschine 100 angeordnetes Weicheisenplättchen, das in den Figuren jedoch nicht dargestellt ist. Durch die besondere Form des Weicheisenplättchens 448 sind keine Veränderungen an der Energie- und Steuereinheit 110 erforderlich, um diese sowohl mit der Akkumaschine 100 als auch mit der Stichsäge 300 betreiben zu können, obwohl bei der Stichsäge 300 der Gasdrücker 336 an der Position angeordnet ist, an der bei der Akkumaschine 100 der Betriebsmodiwahlschalter 138 vorgesehen ist. Diese vertauschte Anordnung der Gasdrücker 136 beziehungsweise 336 hat den Vorteil, daß eine Bedienung sowohl der Akkumaschine 100 als auch der Stichsäge 300 ergonomisch besonders günstig ist.

Sowohl der Gasdrücker 136 und der Betriebsmodiwahlschalter 138 als auch der Gasdrücker 336 können bei mit dem Gehäuse 302 verbundener Platte 328 von der Platte 328 zu Wartungszwecken entfernt werden.

Die auf den Wickelkörper 420 aufgewickelten Motorwicklungen 399 werden mit einem Kaltverguß unter Vakuum oder in Normal-Atmosphäre mit Epoxydharz vergossen, sodaß keine Hohlräume entstehen in denen sich aufgrund der offenen Bauweise sowohl der Akkumaschine 100 als auch der Stichsäge 300 Keime einnisten können. Ferner dient das Vergießen als Vibrationsschutz sowie als Schutz vor Heißdampf bei der Sterilisation der Maschinen. Vorteilhafterweise kann die Kaltvergußmasse mit Füllstoffen gefüllt sein, die einen Wärmetransport von den Motorwicklungen 399 weg verbessern.

Insgesamt umfaßt die Antriebseinheit 320 somit einen dreiphasigen elektronisch kommutierten Motor, welcher in allen Ausführungsbeispielen identisch ausgebildet und sensorlos ist, das heißt es ist insbesondere kein Hall-System mit Hall-Sensoren für die zur Bestromung der Motorwicklungen 399 notwendige Kenntnis einer Lage oder Orientierung des Rotors 350 vorhanden. Eine Rotorlage wird mittels sensorlosem Verfahren, wie weiter unten beschrieben, bestimmt. Grundsätzlich wäre es jedoch auch denkbar, Sensoren zur Erfassung einer Rotorlage in der Antriebseinheit 320 vorzusehen.

Der Getriebeblock 362 dient zur Umsetzung einer Rotation des Rotors 350 in eine durch den Pfeil 450 angedeutete oszillierende Bewegung koaxial zur Längsachse 304 eines die Kupplungsaufnahme 388 umfassenden Kupplungsteils 452 zur Aufnahme eines Sägeblatts. Der Getriebeblock 362 weist ein im wesentlichen konisches Gehäuse auf, in dessen hinterem Ende ein durch das Kugellager 356 gelagertes Antriebsrad 404 gelagert ist, in dessen zylindrische Aufnahme 456 das Kupplungsstück 360 des Rotors 350 einführbar ist. Das Kugellager 356 ist in einem Lagerkörper 458 gehalten, der sich weiter in Richtung auf das vordere Ende des Getriebeblocks 362 erstreckt. Um eine Längsachse 460 quer zur Längsachse 304 ist am Lagerkörper 458 ein Antriebsrad 462 gelagert, welches vom Antriebsrad 404 angetrieben wird. Die Antriebsräder 404 und 462 können entweder als Stirn- und Zahnrad oder als zwei Kegelräder ausgebildet sein. Exzentrisch zur Längsachse 460 trägt das Antriebsrad 462 parallel zur Längsachse 460 eine Pleuelstange 464 auf der zwei Kugellager 466 und 467 gelagert sind, die in einer Bohrung 468 einer koaxial zur Längsachse 304 angeordneten Antriebsstange 470 gefaßt sind. Das Kupplungsteil 452 ist über eine Welle 472 mit der Pleuelstange 464 verbunden, sodaß infolge einer Rotation des Antriebsrads 462 die Pleuelstange 464 die Antriebsstange 470 periodisch bewegt, wobei die Antriebsstange 470 die Welle 472 parallel zu dem über Gleitlager im Gehäuse 454 gelagerten Kupplungsteil 452, wie durch den Pfeil 450 angedeutet, hin und her bewegt.

Der Getriebeblock 362 wird mittels eines Schnellverschlusses mit dem vorderen Abschnitt 324 des Motorgehäuses 322 verbunden. Wird der Getriebeblock 362 abgenommen, kann der Rotor 350 aus der Antriebseinheit 320 entfernt werden, ohne daß diese aus dem Gehäuse 302 herausgenommen werden muß.

Die Antriebsräder 404 und 462 bilden eine Untersetzungsstufe, sodaß der Motor in einem Bereich mit höherem Wirkungsgrad betrieben werden kann. Hierfür muß die Energie- und Steuereinheit 110 nur einen relativ niedrigen Motorstrom bereitstellen, wodurch eine hohe Motordrehzahl erreicht werden kann. Dadurch sinkt bei einer Sägebelastung die Motordrehzahl nicht in einen Bereich unterhalb des Leistungsmaximums des Motors ab, wobei der Motorstrom trotzdem nicht stark ansteigt. Die Untersetzungsstufe ist trotz zusätzlicher Reibungsverluste und Verlusten aufgrund der höheren Motordrehzahl für die Energiebilanz der Maschine positiv, mit anderen Worten kann so bei gleicher zur Verfügung gestellter Energie durch die Energie- und Steuereinheit 110 die Maschine länger betrieben werden. Allerdings ist die Betriebsdauer der Maschine stark von der Höhe des entnommenen Motorstroms abhängig.

Am Gasdrücker 336 ist, wie auch beim Gasdrücker 136, ein verschwenkbar gelagertes Verriegelungselement 474 vorgesehen. Es ist um eine Achse parallel zur Längsachse 440 verschwenkbar am Drückerelement 438 gelagert. In einer ersten extremen Schwenkstellung ermöglicht es eine Bewegung des Drückerelements 438 parallel zur Längsachse 440, in einer anderen extremen Schwenkstellung ist eine Bewegung des Drückerelements 438 in Richtung auf die Platte 328 hin verriegelt. Das Verriegelungselement 474 dient damit als Sicherung der Maschine gegen unbeabsichtigtes Betreiben derselben.

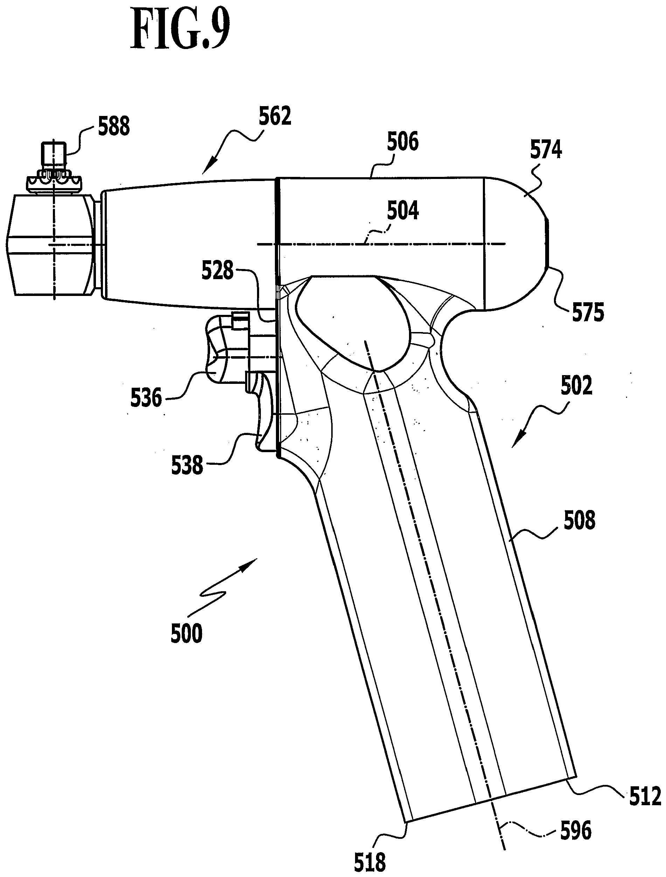

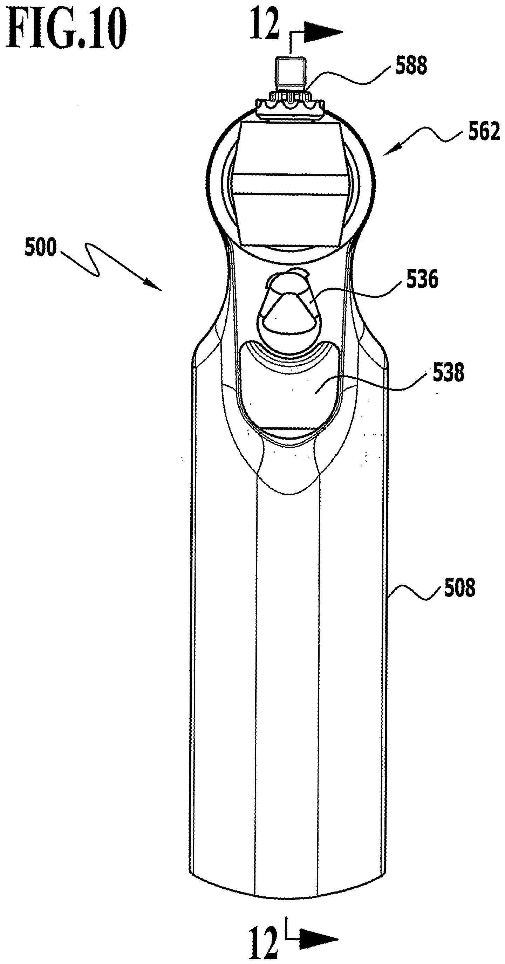

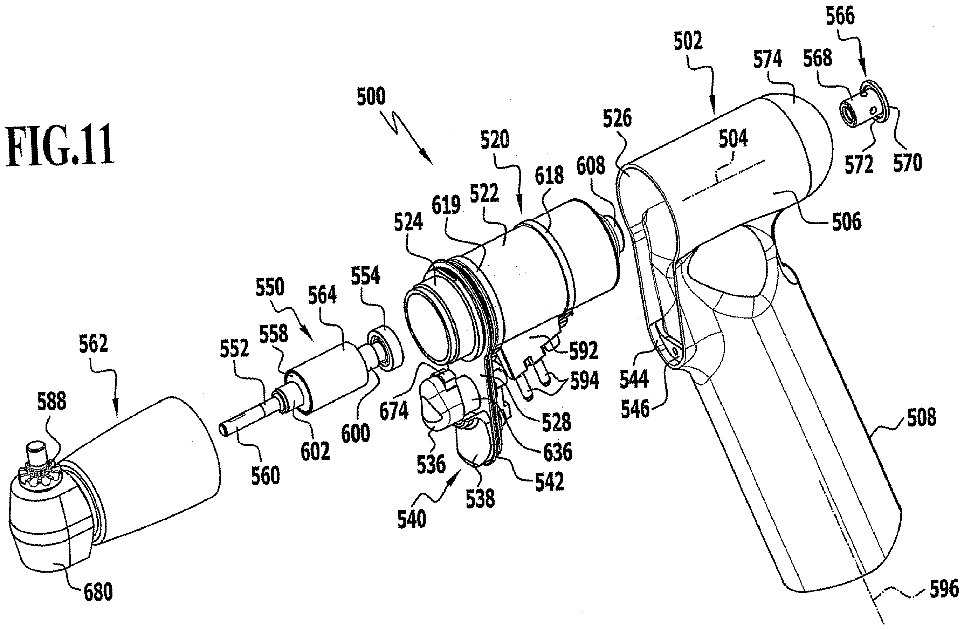

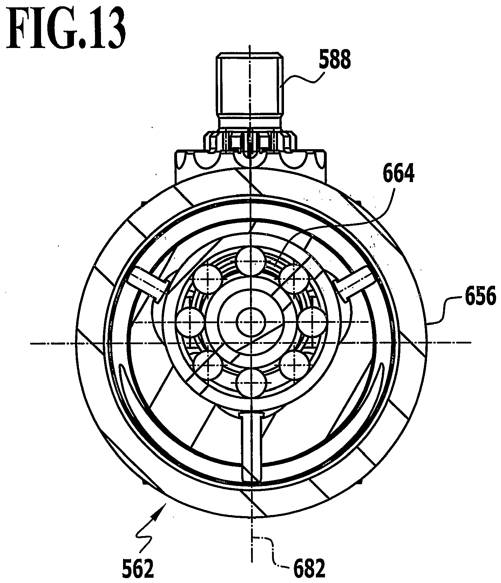

In den Figuren 9 bis 15 ist ein drittes Ausführungsbeispiel einer erfindungsgemäßen chirurgischen Maschine in Form einer insgesamt mit dem Bezugszeichen 500 versehenen oszillierenden Säge dargestellt. Der Aufbau der Säge 500 entspricht im wesentlichen der Stichsäge 300, sodaß identische Teile oder Bauelemente mit Bezugsziffern zwischen 500 und 699 versehen sind, die analog der zur Beschreibung der Stichsäge 300 verwendeten Bezugszeichen 300 bis 499 verwendet wurden.

Zur Energieversorgung dient die die Motorsteuerung 246 umfassende Energie- und Steuereinheit 110, die in einen Aufnahmeraum 509 eines Griffs 508 eines Gehäuses 502 eingeschoben und mittels eines dem Deckel 114 entsprechenden Deckels durch Verschließen der Öffnung 512 gesichert werden kann. Der Griff 508 steht im wesentlichen quer von einem Motorträger 506 ab, welcher eine Längsachse 504 definiert. Der Deckel zum Verschließen der Öffnung 512 weist eine Dichtung auf, die gegen einen unteren Rand des Griffs 518 drückt und damit den Aufnahmeraum 509 keimdicht verschließt.

Eine Antriebseinheit 520 ist auf einer Platte 528 montiert und umfaßt ein Motorgehäuse 522 von welchem ein mit Abstandsringen 618 und 619 zusätzlich gesicherter Kontaktbock 592 in Richtung auf den Griff 508 hin absteht. Der Kontaktbock 592 trägt drei stiftartige Motorkontakte 594, die parallel zur Längsachse 596 abstehen und leitend mit drei Motorwicklungen 599 verbunden sind.

Zwei an der Platte 528 übereinander angeordnete, parallel zur Längsachse 504 orientierte Bohrungen 630 und 632 dienen zur Aufnahme eines Gasdrückers 536 beziehungsweise einer Verkleidung 538, die zusammen mit der Platte 528 eine Drückereinheit 540 bilden. Eine im wesentlichen schlüssellochartige Öffnung des Gehäuses 502 im Bereich des Motorträgers 506 sowie im Übergangsbereich zum Griff 508 dient zum Einführen der Antriebseinheit 520 in das Gehäuse 502. Ein Rand 542 der Platte wird gegen einen Rand 544 der Öffnung 526 mittels einer nicht dargestellten Dichtung abgedichtet. Damit die Platte 528 in einer definierten Position gehalten wird, ist im unteren Bereich der Öffnung 526 eine Anschlagplatte 546 vorgesehen.

Die ins Gehäuse 502 eingeschobene Antriebseinheit 520 wird gesichert, indem eine Befestigungsschraube 566 mit einem Außengewindeabschnitt 568 mit einem Innengewinde 610 eines Verbindungsstutzens 608 am hinteren Ende der Antriebseinheit 520 verschraubt wird. Zu diesem Zweck ist am hinteren Ende 574 des Motorträgers 506 eine Öffnung 575 vorgesehen, an deren Rand sich ein Kopf 570 der Befestigungsschraube 566 abstützt. Mittels einer Dichtung 572 zwischen dem Kopf 570 und dem Rand der Öffnung 575 wird diese keimdicht abgedichtet.

Ein hülsenförmiger Abschnitt 524 am vorderen Ende der Antriebseinheit 520 ragt durch eine Bohrung 530 der Platte 528 hindurch und ist mit einem Getriebeblock 562 verbindbar, welcher an seinem vorderen Ende ein Winkelstück 680 trägt, welches mit einer zapfenförmigen Werkzeugaufnahme 588 versehen ist, auf welche zum Beispiel ein Sägeblatt mit korrespondierender Bohrung aufgesteckt und festgelegt werden kann.

Wird der Getriebeblock 562 von der Platte 528 abgenommen, kann ein Rotor 550 aus der Antriebseinheit 520 entnommen werden. Er umfaßt eine im wesentlichen zylindrische Lagerwelle 552, an deren hinterem Ende ein Kugellager 554 festgelegt ist. Ein zylindrischer, in Richtung der Längsachse 504 durchbohrter Dauermagnet 558 ist auf die Lagerwelle 552 aufgeschoben und in Umfangsrichtung durch eine dünne, hülsenartige Rotorarmierung und an seinen Stirnflächen durch manschettenartige Klemmelemente 600 und 602 gleichzeitig geschützt und gehalten. Ein vorderes Ende der Lagerwelle 552 bildet ein Kupplungsstück 560, welches mit einem Antriebsrad 604 drehfest in Eingriff bringbar ist.

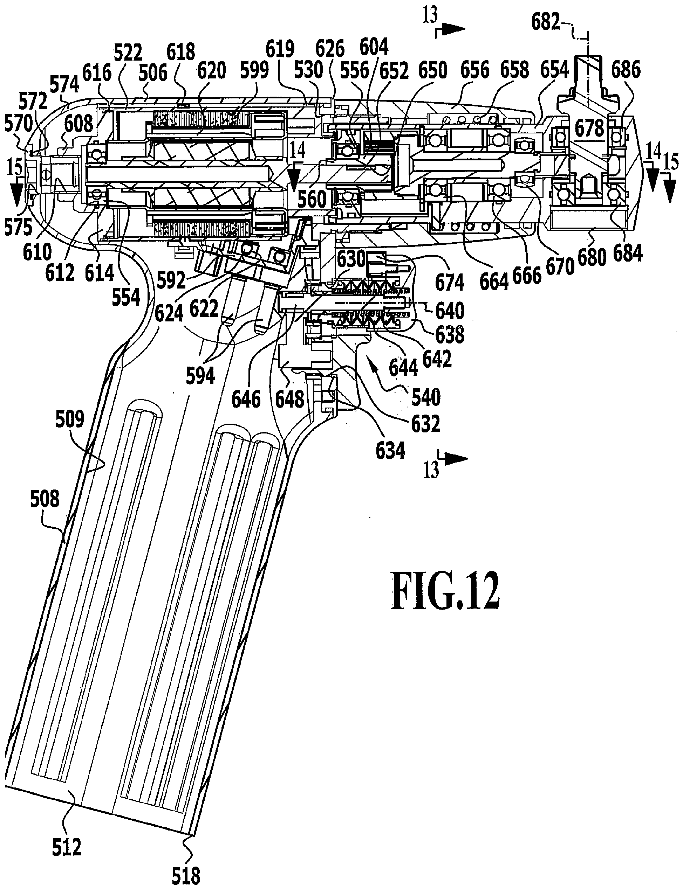

Ein innerer Aufbau der Säge 500, insbesondere des Getriebeblocks 562, wird nachfolgend im Zusammenhang mit den Figuren 12 bis 15 näher erläutert.

Der Verbindungsstutzen 608 erweitert sich zweimal einstufig im Innendurchmesser, wobei im Bereich seiner zweiten Erweiterung 612 das Kugellager 554 gehalten ist. Ein radial nach außen vom Verbindungsstutzen 608 abstehender Ringflansch 614 dient in Verbindung mit einer Ringdichtung 616 zum Verschließen eines hinteren Endes des Motorgehäuses 522. In das Motorgehäuse 522 ist ein Wickelkörper 620 eingesetzt, auf welchem die Motorwicklungen 599 aufgewickelt sind. Wie im Zusammenhang mit dem Aufbau der Stichsäge 300 beschrieben, sind auch die Motorwicklungen 599 mit einem Kaltverguß vollständig vergossen. Im Kontaktblock 592 in Richtung auf die Längsachse hin weisende Enden 622 der Motorkontakte 594 sind mit Querbohrungen 624 versehen, durch welche die Motorwicklungen 599 durchgefädelt und verlötet sind. Ein ringförmiger Anschlag 626 im Übergangsbereich des Motorgehäuses 522 im vorderen Abschnitt 527 liegt direkt an der Platte 528 an.

Die Drückereinheit 540 ist identisch mit der Drückereinheit 340 aufgebaut. Der Gasdrücker 536 umfaßt einen in die Bohrung 630 eingesetzten Lagerkörper 636, der gegenüber der Platte 528 keimdicht abgedichtet ist. Die ergonomisch zur Abstützung eines Mittel- oder Ringfingers geformte Verkleidung 538 ist mittels einer die Bohrung 632 umgebenden Ringdichtung gegenüber der Platte 528 abgedichtet. Koaxial zu einer Längsachse 640 ist im Lagerkörper 636 ein Drückerelement 638 verschiebbar gelagert. Mittels einer Schraubenfeder 642 wird das Drückerelement, wenn es nicht betätigt ist, in einer Grundstellung gehalten, in der es maximal von der Platte 528 vorsteht. Eine einerseits am Lagerkörper 636 und andererseits am Drückerelement 638 festgelegte Faltenbalgdichtung 644 dient zur Abdichtung des Gasdrückers 536 relativ zur Platte 528. Das einen in das Gehäuse 502 hineinragenden Stift 646 umfassende Drückerelement 638 trägt am vorstehenden Ende des Stifts 646 ein nach unten weisendes Weicheisenplättchen 648, welches Abmessungen aufweist, damit es in den Spalt 268 der Energie- und Steuereinheit 110 eingeschoben werden kann. Durch Betätigen des Gasdrückers 536 wird der Drehzahlvorgabesensor 282 (nicht Teil der Erfindung) betätigt, woraufhin die Motorsteuerung 246 die Antriebseinheit 220 mit einem für die gewünschte Drehzahl erforderlichen Strom beaufschlagt. Der Getriebeblock 562 weist an seinem hinteren, auf die Platte 528 hin weisenden Ende eine Aufnahme 650 für das Kupplungsstück 560 auf. An diesem ist eine Aussparung 652 vorgesehen, in welche ein Vorsprung in der Aufnahme 650 eintauchen kann, wodurch eine drehfeste Verbindung zwischen dem Antriebsrad 604 und der Lagerwelle 552 hergestellt wird. Das Antriebsrad 604 ist mittels des Kugellagers 556 an einem Gehäuseinnenteil gelagert. Das Gehäuseinnenteil ist relativ zu einem Außengehäuse 656 um die Längsachse 504 verdrehbar gelagert und wird mittels einer Spiralfeder in einer Grundstellung gehalten, in der der Getriebeblock 562 an der Platte 528 verriegelt ist.

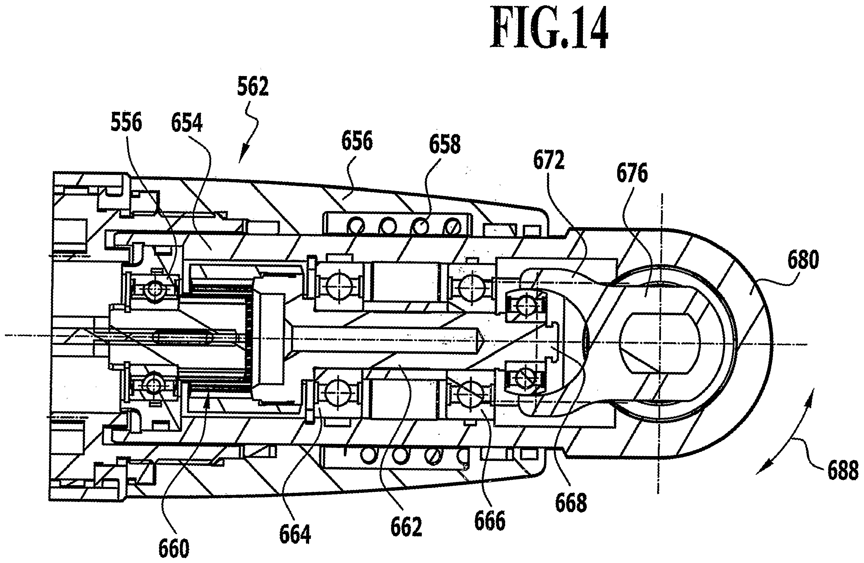

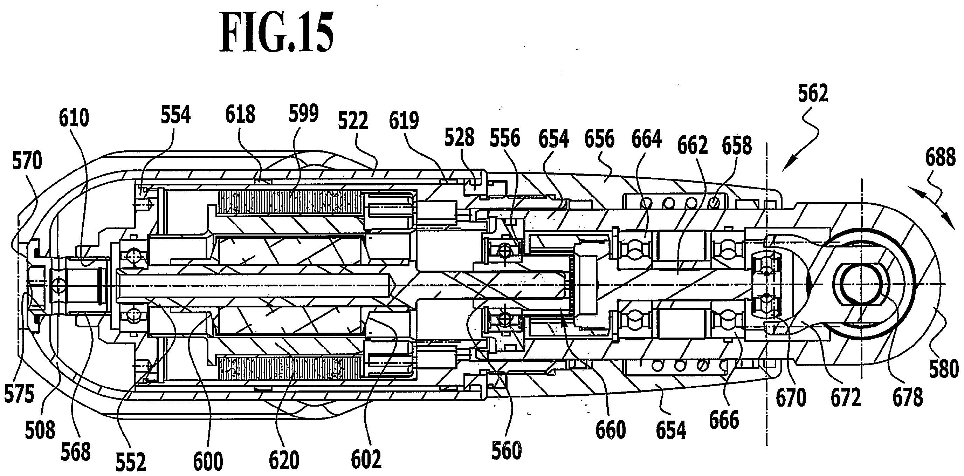

Das Antriebsrad 604 ist Teil einer Untersetzungseinheit 660, mit der eine Drehzahl der Antriebseinheit 520 auf einen gewünschten Wert herabgesetzt werden kann. Von der Untersetzungseinheit 660 angetrieben wird eine Antriebswelle 662, die mittels zweier Kugellager 664 und 666 im Gehäuseinnenteil 654 parallel, aber etwas versetzt zur Längsachse 504 gelagert ist. Ein vorderes Ende der Antriebswelle 662 ist in Form eines exzentrischen Zapfens 668 ausgebildet, welcher ein Kugellager 670 mit einer sphärischen Außenfläche trägt, die in einem gabelförmigen Ende 672 eines Schwinghebels 676 gehalten ist. Der Schwinghebel ist an einer um eine Drehachse 682 drehbar gelagerten Welle 678 festgelegt, die im Winkelstück 680 gelagert ist und deren aus dem Winkelstück 680 vorstehendes Ende die Werkzeugaufnahme 588 bildet. Die Drehachse 682 steht senkrecht auf der Längsachse 504 und schneidet diese. Die Welle 678 ist mittels zweier Kugellager 684 und 686 im Winkelstück 680 gelagert. Infolge einer Rotation der Antriebswelle 662 führt der Zapfen 668 eine exzentrische Bewegung aus und zwingt dem Ende 672 und damit dem Schwinghebel 676 eine oszillierende Bewegung auf, wobei das Kugellager 670 im gabelförmigen Ende 672 gleiten kann. Die oszillierende Bewegung der Welle 678 ist durch einen Pfeil 688 in den Figuren 14 und 15 angedeutet.

Durch die parallel zur Lagerwelle 552 angeordnete Antriebswelle 662 kann ein besonders geringer Außendurchmesser des Außengehäuses 656 sowie ein symmetrischer Aufbau desselben realisiert werden. Es könnte aber auch bei Einsatz einer alternativen Untersetzungseinheit die Antriebswelle 662 koaxial zur Lagerwelle 552 angeordnet sein.

Allen drei beschriebenen Maschinen, also der Akkumaschine 100, der Stichsäge 300 und der oszillierenden Säge 500 ist gemein, daß die Antriebseinheiten 120, 320 und 520 sowie die Kupplungsvorrichtung 180 und die Getriebeblöcke 362 und 562 nicht abgedichtet sind. Dies hat den Vorteil, daß diese Maschinenbereiche bei der Sterilisation nach innen keimfrei werden. Die genannten Elemente können zur Schmierung der Lager mit Öl durchgesprüht werden. Alternativ könnten die Antriebseinheiten auch abgedichtet sein und die Lager für die gesamte Lebensdauer geschmiert werden. Der Innenraum könnte dann trotzdem durch Sterilisation keimfrei gemacht werden, wenn ein in der internationalen Patentanmeldung PCT/EP03/00911 beschriebenes Membranventil vorgesehen ist.

Im An- und Abtriebsbereich der Maschinen können zusätzlich Anschlußmöglichkeiten für eine Waschmaschine vorgesehen sein, sodaß verschmutzte Kanulierungen von rotatorischen Antrieben manuell gereinigt werden können. Ebenso verschmutzte Werkzeugaufnahmen 588 für Sägeblätter.

Durch die Möglichkeit, die Antriebseinheiten 120, 320 und 520 vom jeweiligen Gehäuse 102, 302 und 502 zu lösen, besteht die Möglichkeit, die Antriebseinheiten 120, 320 und 520 auch außerhalb des Gehäuses zu testen und zu betreiben.

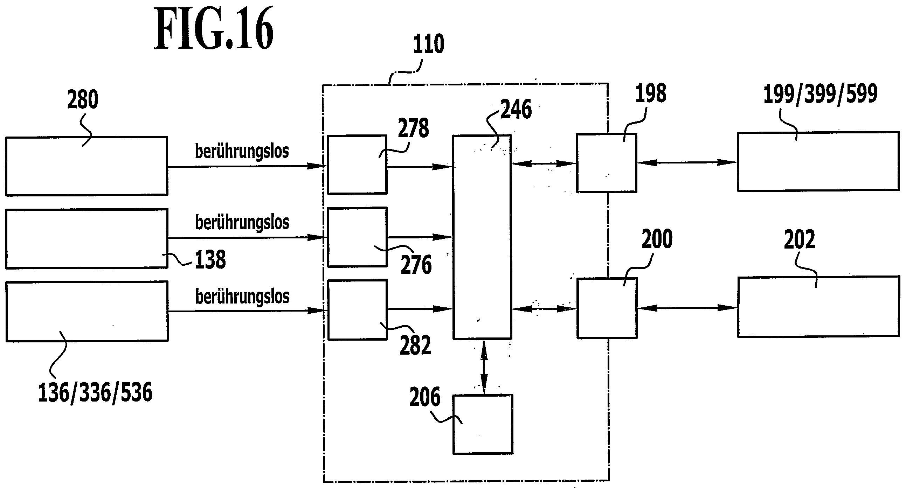

Im Zusammenhang mit den Figuren 16 bis 19 wird die Funktionsweise der Energie- und Steuereinheit 110 in Verbindung mit den Drückereinheiten 140, 340 und 540 näher erläutert. Mit den Gasdrückern 136, 336 und 536 kann berührungslos der Drehzahlvorgabesensor 282 (nicht Teil der Erfindung) der Energie- und Steuereinheit 110 betätigt werden. Entsprechende, bei der Motorsteuerung 246 ankommende Signale des Drehzahlvorgabesensors werden verarbeitet und über die Verbindungskontakte 198 die Motorkontakte 194 und die damit verbundenen Motorwicklungen 199, 399 bzw. 599 entsprechend mit Strom beaufschlagt, um die Antriebseinheiten 120, 320 und 520 mit einer entsprechenden Drehzahl zu betreiben. Der Betriebsmodiwahlschalter 138 dient zur berührungslosen Betätigung der Lichtschranke 276, die bei Unterbrechung die Akkumaschine 10 von einem Rechtslaufbetrieb in einen Linkslaufbetrieb umschaltet.

Ferner kann mit dem Vorsprung 280 die Lichtschranke 278 berührungslos betätigt werden. Tritt dieser Fall ein, aktiviert oder deaktiviert die Motorsteuerung 246 die grundsätzliche Möglichkeit, die Akkumaschine 110 in einen Oszillationsbetrieb oder einen Pilgerschrittbetrieb umzuschalten.

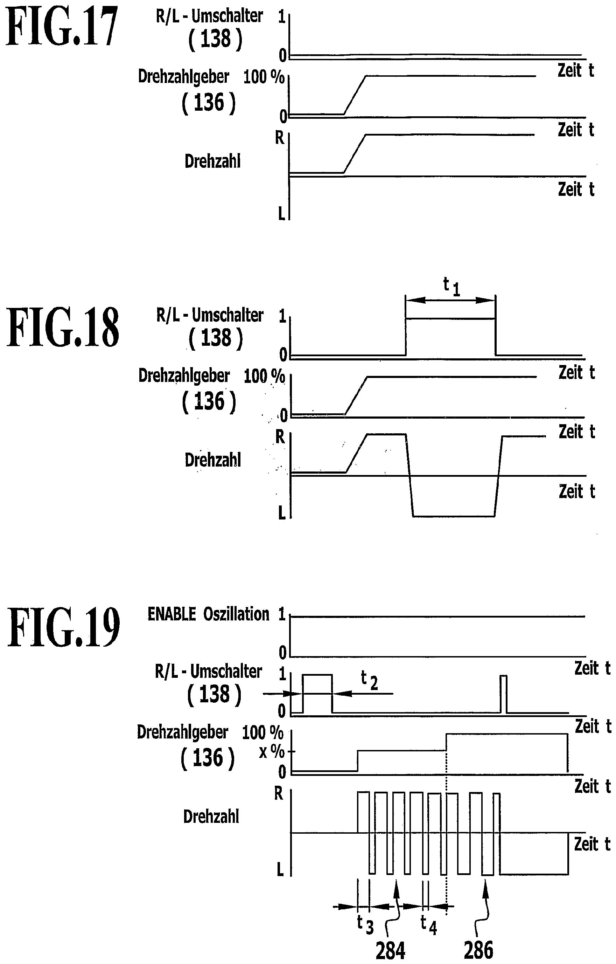

In Figur 17 sind zeitliche Verläufe einer Drehzahl der Lagerwelle 152 sowie einer prozentualen Betätigung des als Drehzahlgeber dienenden Gasdrückers 136 dargestellt. Der als Rechts(R)/Links(L)-Umschalter dienende Betriebsmodiwahlschalter 138 ist bei den in Figur 17 dargestellten zeitlichen Verläufen nicht betätigt. Je weiter der Gasdrücker 136 eingedrückt wird, maximal bis zum Anschlag, was im Diagramm dem Wert 100 % entspricht, umso mehr nimmt die Drehzahl der Antriebseinheit 120 zu.

In Figur 18 ist im Unterschied zu Figur 17 dargestellt, daß während des Betriebs der Akkumaschine 100 im Rechtslauf der Betriebsmodiwahlschalter 138 für eine Zeitdauer t₁ gedrückt wird. Folge hiervon ist, daß die Antriebseinheit vom Rechtslaufbetrieb auf den Linkslaufbetrieb umschaltet, was im zeitlichen Verlauf der Drehzahl zu erkennen ist.

Unter der Voraussetzung, daß von der Lichtschranke 278 ein entsprechendes Signal geliefert wird, in Figur 19 mit "ENABLE Oszillation" bezeichnet, kann die Akkumaschine 100 nicht nur in einem Rechtslaufbetrieb oder einem Linkslaufbetrieb, wie im Zusammenhang mit den Figuren 17 und 18 beschrieben, betrieben werden, sondern auch in einem Oszillationsbetrieb oder in einem Pilgerschrittbetrieb. Voraussetzung hierfür ist, daß die Akkumaschine 100 zunächst stillsteht. Wird der Betriebsmodiwahlschalter 138 für eine Zeit t₂ gedrückt, die größer ist als eine vorgegebene Umschaltzeit, die beispielsweise vorgegeben werden kann in einem Bereich von einer Sekunde bis zehn Sekunden, dann kann zum Beispiel durch ein von der Energie- und Steuereinheit 110 erzeugtes akkustisches Signal signalisiert werden, daß die Akkumaschine 100 in den Oszillationsmodus umgeschaltet wurde. Wird nach dem Umschalten der Gasdrücker 136 nur bis maximal X-% durchgedrückt, so aktiviert die Motorsteuerung 246 die Antriebseinheit 120 im sogenannten Pilgerschrittbetrieb, wie in Figur 19 mit dem Pfeil 284 angedeutet. Die Antriebseinheit wechselt dabei periodisch die Drehrichtung, wobei jedoch die Antriebseinheit 120 jeweils während einer Zeitdauer t₃ im Rechtslaufbetrieb und während einer Zeitdauer t₄ im Linkslaufbetrieb betrieben wird und wobei die Zeitdauer t₃ etwas größer ist als die Zeitdauer t₄. So ergibt sich im Ergebnis ein "stotternder" Rechtslaufbetrieb. In den eigentlichen Oszillationsbetrieb, der in Figur 19 mit Pfeil 286 gekennzeichnet ist, und bei dem die Antriebseinheit 120 jeweils gleich lange im Rechtslauf und im Linkslauf betrieben wird, wird erst umgeschaltet, wenn der Gasdrücker 136 mehr als die voreingestellten X-% durchgedrückt wird. Wird der Betriebsmodiwahlschalter 138 kurzzeitig betätigt, schaltet die Motorsteuerung 246 die Akkumaschine 100 in den Linkslaufbetrieb um. Ein Umschalten in den Oszillationsbetrieb erfordert wiederum die dauerhafte Betätigung des Betriebsmodiwahlschalters 138 bei stillstehender Antriebseinheit 120 für mindestens die vorgegebene Umschaltzeit.

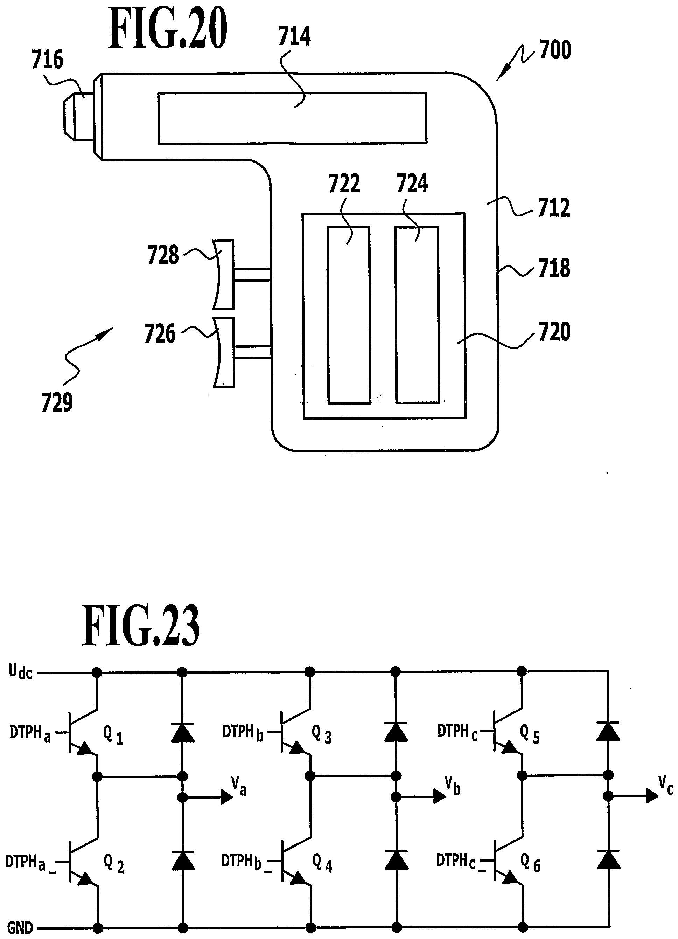

Nachfolgend wird im Zusammenhang mit den Figuren 20 bis 22 der Aufbau und die Funktionsweise einer Motorsteuerung für eine netzabhängig oder netzunabhängig betreibbare chirurgische Maschine näher erläutert.

In Figur 20 ist eine insgesamt mit dem Bezugszeichen 700 versehene chirurgische Akkumaschine rein schematisch dargestellt, bei der es sich beispielsweise auch um die oben beschriebene Akkumaschine 100, die Stichsäge 300 oder die oszillierende Säge 500 handeln kann, die in ihrem Aufbau von der Akkumaschine 700 im Detail abweichen können. Die Akkumaschine 700 weist ein Gehäuse 712 auf, in dessen einem Teil ein sensorloser Elektromotor 714 parallel zur Längsachse dieses Gehäuseteils angeordnet ist und eine nicht dargestellte Antriebswelle der Akkumaschine 700 antreibt. Am Ende der Antriebswelle ist eine Kupplung 716 angeordnet, mittels welcher die Akkumaschine 700 mit Werkzeugen jedweder Art verbunden werden kann, beispielsweise Bohrern, Fräsen, Meißeln und auch Sägen oder Getriebeeinheiten zum Verbinden mit einem der beispielhaft genannten Werkzeuge.

Von dem den Elektromotor 714 aufnehmenden Gehäuseteil des Gehäuses 712 steht quer ein Handgriff 718 ab, in welchen eine Energie- und Steuereinheit in Form eines Powerpacks 720 einführbar ist. Der Powerpack 720 umfaßt eine wiederaufladbare Batterie oder Akkuzelle 722 sowie eine Motorsteuerung 724. Zur Inbetriebnahme der Akkumaschine 700 sind zwei Drücker, nämlich ein Gasdrücker 726 und ein Betriebsmodiwahlschalter 728 vorgesehen, welche im wesentlichen parallel zu einer Längsachse des Elektromotors 714 in den Handgriff 718 hineingedrückt werden können. Der Gasdrücker 726 und der Betriebsmodiwahlschalter 728 bilden zusammen eine Drückereinheit.

Bei dem Elektromotor 714 handelt es sich erfindungsgemäß um einen sensorlosen bürstenlosen Gleichstrommotor, das heißt, es sind keine Drehzahlerfassungssensoren zur Detektion einer Rotorbewegung und einer Position eines Rotormagneten, nachfolgend mit Rotor bezeichnet, des Elektromotors 714 vorgesehen. Grundsätzlich wäre es aber auch denkbar, einen mit einem Sensorsystem zur Drehzahlerfassung ausgestatteten Elektromotor einzusetzen.

In den Figuren 721 und 722 ist schematisch dargestellt, beispielsweise für den Gasdrücker 726 oder einen der Gasdrücker 136, 336 oder 536, wie mit einem am Powerpack angeordneten Betätigungssensor (nicht Teil der Erfindung), beispielsweise dem Hallsensor 264, berührungslos ein Betätigungssignal durch Bewegen des Gasdrückers 726 erzeugt werden kann.

Ein Hallsensor 770 mit drei Anschlußkontakten 772 dient als Betätigungssensor (nicht Teil der Erfindung). Er ist zwischen kurzen Schenkeln 774 zweier L-förmiger Weicheisenplatten 776 eingebracht, so daß sich eine quaderförmige Struktur aus den Schenkeln 774 und dem dazwischen angeordneten Hallsensor 770 und gegebenenfalls weiterer, einen Zwischenraum zwischen den Schenkeln 774 ausfüllenden Weicheisenelementen ergibt. Zwischen freien Enden 778 zweier langer Schenkel 780 der beiden Weicheisenplatten 776 ist ein zylindrischer Stabmagnet 782 angeordnet, wie er beispielsweise an der Energie- und Steuereinheit 110 in Form des Stabmagneten 266 vorgesehen ist. Insgesamt ergibt sich also eine rahmenförmige Struktur, die eine von den langen Schenkeln 780, dem Stabmagnet 782 und den kurzen Schenkeln 774 mit dem dazwischenliegenden Hallsensor 770 begrenzte Öffnung 784 definiert, wie sie beispielsweise an der Energie- und Steuereinheit 110 in Form der Öffnung 272 vorgesehen ist und welche einen rechteckigen Querschnitt aufweist. An dem in Figur 21 schematisch dargestellten Drücker 726 ist an einer von einem Betätigungsknopf 727 weg weisenden Stirnfläche 786 des Grundkörpers 725 ein Weicheisenquader 788, wie er beispielsweise an der Energie- und Steuereinheit 110 in Form des Weicheisenquaders 270 vorgesehen ist, in Richtung einer Längsachse 790 des Grundkörpers 725 abstehend angeordnet, wobei eine Stirnfläche 792 des Weicheisenquaders 788 relativ zur Stirnfläche 786 des Grundkörpers 725 um 45° geneigt ist.

Die Rahmenstruktur mit dem Hallsensor 770 (nicht Teil der Erfindung) ist relativ zum Drücker 726 so angeordnet, daß infolge einer Betätigung des Drückers 726 in Richtung des Pfeils 794 in Figur 21, also parallel zur Längsachse 790, der Grundkörper 725 mit dem Weicheisenquader 788 in die Öffnung 784 hineinbewegt werden kann. Der ferromagnetische Weicheisenquader 788 weist aufgrund seiner Form ausgehend von einer vorderen Stirnkante 796 parallel zur Stirnfläche 786 zunehmende Querschnitte auf, was in Figur 22 durch die beiden eingezeichneten Schnittebenen 798 und 800 angedeutet ist, welche beide parallel zur Stirnfläche 786 verlaufen.

Wird der Weicheisenquader 788 in die Öffnung 784 hineinbewegt, dann hat dies Auswirkungen auf den magnetischen Fluß innerhalb des durch die beiden Weicheisenplatten 776 gebildeten Rückschlußsystems, welches den Stabmagnet 782 mit dem Hallsensor 770 (nicht Teil der Erfindung) koppelt. Eine Änderung des den Hallsensor 770 durchsetzenden magnetischen Flusses führt zu einer Änderung einer vom Hallsensor 770 erzeugten Hallspannung, welche über die Anschlußkontakte 772 abgegriffen werden kann. Von der Motorsteuerung 724 wird dann die gemessene Hallspannung, welche als Betätigungssignal genutzt wird, in entsprechende Steuersignale für den Motor 714 aufbereitet.

Die beiden Drücker 726 und 728 sind derart angeordnet, daß sie insbesondere bei einer batteriebetriebenen Antriebsmaschine verbleiben, wobei die Energie- und Steuereinheit 720 mit der Motorsteuerung 724 und der Batterie 722 vor einer Sterilisierung der Akkumaschine 700 entnommen werden kann. An der Akkumaschine 700 verbleibt dann also die Drückereinheit 729, welche die Feldänderungsglieder in Form der Weicheisenquader 788 umfaßt. Damit verbleiben keine elektrischen Kontakte innerhalb der Akkumaschine 700, die für die Erzeugung eines Betätigungssignals erforderlich sind, denn mit einer Steuerelektronik, beispielsweise in Form der Motorsteuerung 724, werden auch die Hallsensoren 770 vollständig entfernt. Die besondere Anordnung des Hallsensors 770 relativ zum Stabmagnet 782 gestattet es, deren räumliche Zuordnung eindeutig und dauerhaft festzulegen. Eine Nachjustierung wie bei herkömmlichen Systemen ist nicht erforderlich. Etwaige Fertigungstoleranzen bei der Größe und Form des Weicheisenquaders 788 und der Öffnung 784 wirken sich weit weniger kritisch auf eine Funktion der Drückereinheit 729 aus, als dies bei herkömmlichen Systemen mit einen relativ zu dem Hallsensor 770 bewegten, am Drücker gelagerten Magneten der Fall ist.

In Figur 23 ist eine schematische Darstellung eines Drei-Phasen-Leistungsinverters dargestellt, wobei Vₐ, V_{b} und V_{c} die an die Motorwicklungen angelegten Spannungen repräsentieren. Insgesamt sechs Leistungstransistoren sind paarweise in Serie und als Paare jeweils parallel zueinander geschaltet und mit Q₁ bis Q₆ bezeichnet. In der Schaltungsskizze sind die Schaltzustände der jeweiligen Transistoren Q₁ bis Q₆ mit DTPHₓ bezeichnet, wobei x für a, b oder c steht. Üblicherweise wird der mit dem oberen Transistor in Serie geschaltete untere Transistor ausgeschaltet, wenn der obere Transistor angeschaltet wird, und umgekehrt.

Die in Figur 23 dargestellte Schaltskizze entspricht sowohl der herkömmlichen Puls-Weiten-Modulation (PWM) als auch der Space-Vektor-Puls-Weiten-Modulation (SVPWM). Allerdings unterscheiden sich die Schaltschemata beider Verfahren deutlich.

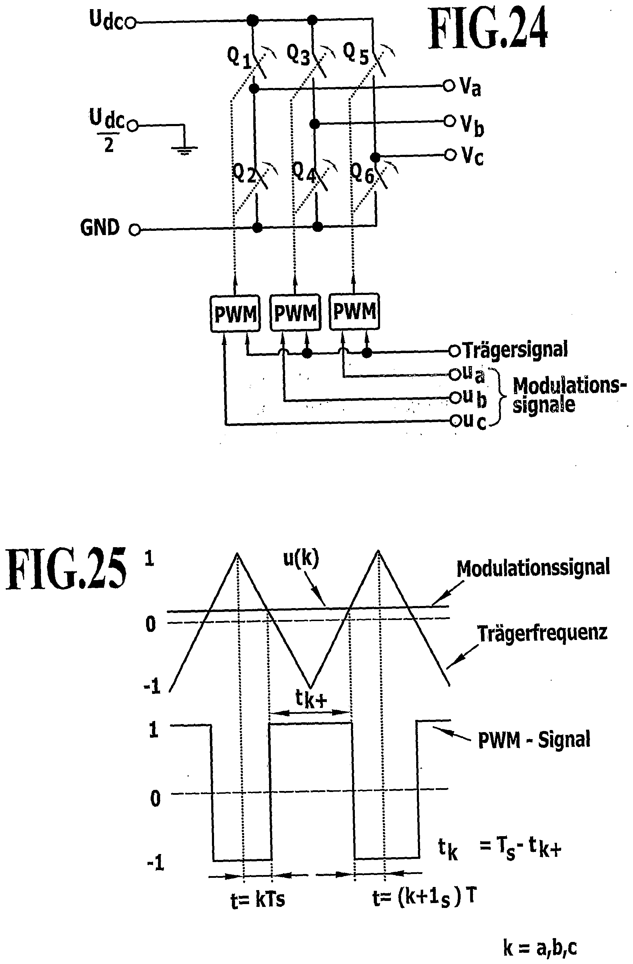

Beim herkömmlichen Puls-Weiten-Modulations-(PWM)-Verfahren, welches nachfolgend mit Bezug zu den Figuren 24 und 25 beschrieben wird, wird ein Modulationssignal jeder Motorwicklung a, b und c auf eine Trägerfrequenz aufmoduliert. Die Trägerfrequenz wird als periodischer Sägezahn gewählt, so daß sich das Puls-Weiten-Modulations-Signal dadurch ergibt, daß der Schaltzustand dann "1" ist, wenn das Trägerfrequenzsignals unterhalb des Modulationssignals liegt. Die Transistoren Q₁ bis Q₆ werden dann entsprechend geschaltet, wobei jeweils entweder der obere Transistor Q₁, Q₃ oder Q₅ oder der untere Transistor Q₂, Q₄ oder Q₆ durchgeschaltet wird. Es ist ohne weiteres verständlich, daß bei der in den Figuren 24 und 25 exemplarisch dargestellten herkömmlichen Puls-Weiten-Modulation (PWM) eines Drei-Phasen-Leistungsinverters stets mindestens eine Motorwicklung unbestromt bleibt. Dies ermöglicht eine Verstellung eines Feldwinkels γ des durch Bestromung der drei Motorwicklungen aufgebauten Statorfeldes nur in 60°-Schritten.

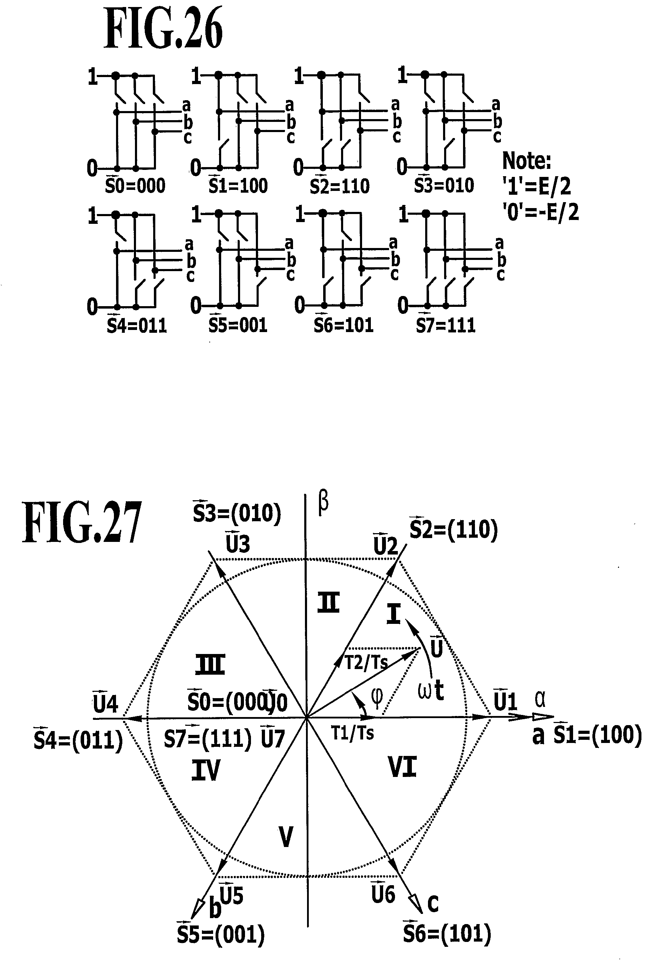

Hiervon unterscheidet sich das Space-Vektor-Puls-Weiten-Modulations-(SVPWM)-Verfahren. In Figur 26 ist schematisch das Schaltschema der sechs Transistoren dargestellt. Jeder Schaltstellung ist ein sogenannter Space-Vektor im Space-Vektor-Raum zugeordnet. So entspricht der Space-Vektor Sₒ einer Schaltstellung 000, bei welcher die drei unteren Transistoren Q₂, Q₄ und Q₆ geschlossen sind. Die in Figur 27 angegebenen Space-Vektoren definieren die Schaltstellung der in Serie geschalteten Transistorenpaare, wobei eine "0" bedeutet, daß der untere Transistor durchgeschaltet ist, und eine "1", daß der obere Transistor durchgeschaltet ist. Insgesamt gibt es acht Schaltzustände, die durch jeweils einen Spannungsvektor U₀ bis U₇ dargestellt werden können, welche jeweils einem der acht Schaltzustände entsprechen. Jeder Spannungsvektor U₁ bis U₆ weist eine Länge auf, die einem Einheitsvektor entspricht, die Länge der Spannungsvektoren U₀ und U₇ dagegen ist Null. Somit unterteilen die sechs Spannungsvektoren U₁ bis U₆ den Spannungsvektorraum in insgesamt sechs Sektoren, wobei die Spannungsvektoren U₁ und U₄, U₂ und U₅ sowie U₃ und U₆ jeweils entgegengesetzt gerichtet sind und sich paarweise zum Nullvektor addieren.

Eine beliebige Ausgangsspannung U läßt sich nunmehr periodisch stufenlos variieren durch entsprechende Bestromung aller drei Motorwicklungen. Dadurch läßt sich also ein beliebiger Winkel γ des Statorfeldes relativ zum Rotor des Elektromotors einstellen, also nicht nur in 60°-Schritten wie bei der oben beschriebenen herkömmlichen Puls-Weiten-Modulation (PWM).

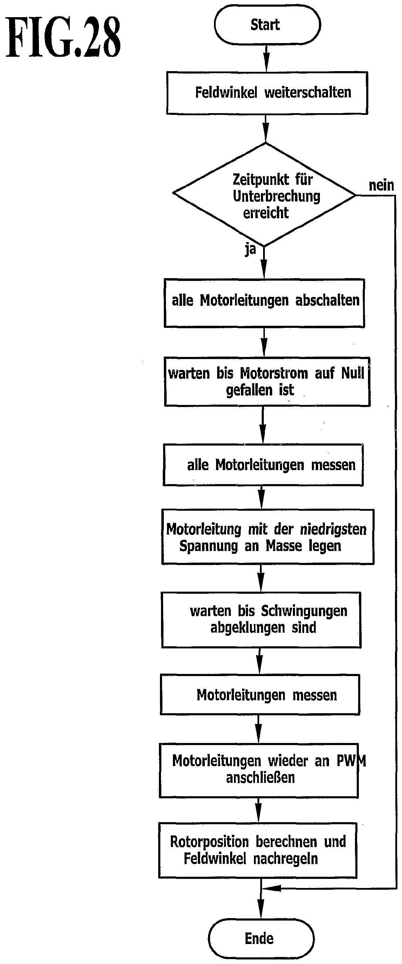

Allerdings kann bei der Space-Vektor-Puls-Weiten-Modulation (SVPWM) eine Gegen-EMK der Motorwicklungen nicht ohne weiteres bestimmt werden, da grundsätzlich alle Motorwicklungen gleichzeitig bestromt werden. Aus diesem Grund wird die Bestromung mindestens einer, vorzugsweise aller Motorleitungen kurzzeitig unterbrochen. Ein entsprechender Ablaufplan ist in Figur 28 dargestellt. Die Motorsteuerung gibt das Schaltschema der Space-Vektor-Puls-Weiten-Modulation (SVPWM) entsprechend einer gewünschten Drehzahlanforderung durch einen Bediener vor. Dies bedeutet, daß nach Aufnahme eines Betriebs der chirurgischen Maschine ein Feldwinkel γ des durch die bestromten Motorwicklungen erzeugten Statorfeldes kontinuierlich weitergeschaltet wird. Die in Figur 7 dargestellte Abfrageroutine wird bei jedem Puls-Weiten-Modulations-Impuls aufgerufen. Nach Start der Routine wird zunächst der Feldwinkel des Statorfeldes weitergeschaltet. Bei dieser Vorgehensweise wird mit zwei unterschiedlichen Frequenzen gearbeitet. Die Frequenz, mit welcher eine Bestromung des Motors unterbrochen wird, ist gleich oder kleiner als die Puls-Weiten-Modulations-Frequenz. Beispielsweise kann die Unterbrechungsfrequenz ein kHz betragen, bei einer Puls-Weiten-Modulations-Frequenz von acht kHz. Oder anders ausgedrückt, beträgt die Puls-Weiten-Modulations-Frequenz vorzugsweise ein Vielfaches, insbesondere ein ganzzahliges Vielfaches, der Unterbrechungs- oder Abtastfrequenz. Es kommt also zu einer Überlagerung von zwei Puls-Weiten-Modulations-Frequenzen. Mit anderen Worten bedeutet dies für das angegebene Zahlenbeispiel, daß alle acht Umdrehungen des Rotors die Motorleitungen kurzzeitig abgeschaltet werden.

Ist, wie in Figur 28 dargestellt, der Zeitpunkt für eine Unterbrechung der Bestromung erreicht, dann werden alle Motorleitungen abgeschaltet. In einem nächsten Schritt wird gewartet, bis der Motorstrom auf Null gefallen ist. Danach werden alle Motorleitungen beziehungsweise Motorwicklungen durchgemessen und diejenige Motorleitung mit der niedrigsten Spannung an Masse gelegt beziehungsweise mit Masse verbunden.

Als nächstes wird abgewartet, bis auftretende Schwingungen aufgrund der Schaltvorgänge abgeklungen sind. Danach werden alle Motorleitungen gemessen, das heißt an allen Motorleitungen wird gleichzeitig die Gegen-EMK bestimmt. Nach Abschluß der Messungen werden die Motorleitungen wieder entsprechend dem Space-Vektor-Puls-Weiten-Modulations-(SVPWM)-Verfahren bestromt. Mit den gemessenen Gegen-EMK-Werten wird die Rotorposition berechnet und der Feldwinkel γ entsprechend nachgeregelt, was durch eine entsprechende Anpassung des Space-Vektor-Puls-Weiten-Modulations-Schaltschemas geschieht.

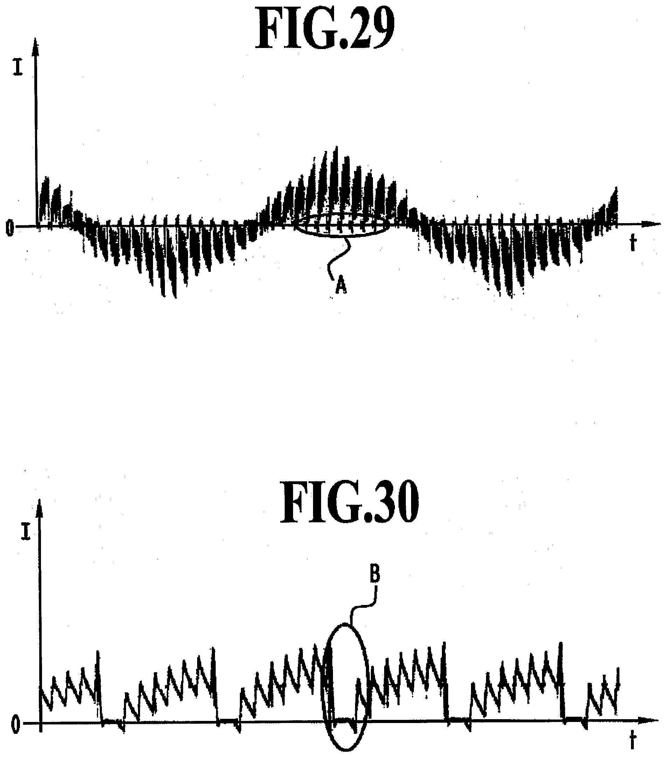

Durch die Space-Vektor-Puls-Weiten-Modulations läßt sich an jeder Motorleitung ein quasi sinusartiger Verlauf des Phasenstroms erzeugen. Der Phasenstrom an einer Motorleitung ist beispielhaft in Figur 29 für etwas mehr als eine Umdrehung des Rotors dargestellt. Die verschmierten Bereiche entstehend durch die Puls-Weiten-Modulation. In dem mit A markierten Bereich erkennt man periodische Unterbrechungen, in denen der Phasenstrom auf Null abfällt. Dies ist in Figur 30 zusätzlich vergrößert dargestellt, und zwar mit einer zehnfach höheren zeitlichen Auflösung. Man erkennt nun die in Figur 29 dargestellten dunklen Bereiche deutlicher. Periodisch, wie oben angegeben mit achtfach höherer Frequenz als die Unterbrechungsfrequenz, wird ein Puls-Weiten-Modulations-Signal erzeugt. Alle acht PMW-Pulse wird jedoch die Motorbestromung unterbrochen, das heißt der Phasenstrom fällt auf Null, was in dem mit B gekennzeichneten Bereich in Figur 30 gut zu erkennen ist.

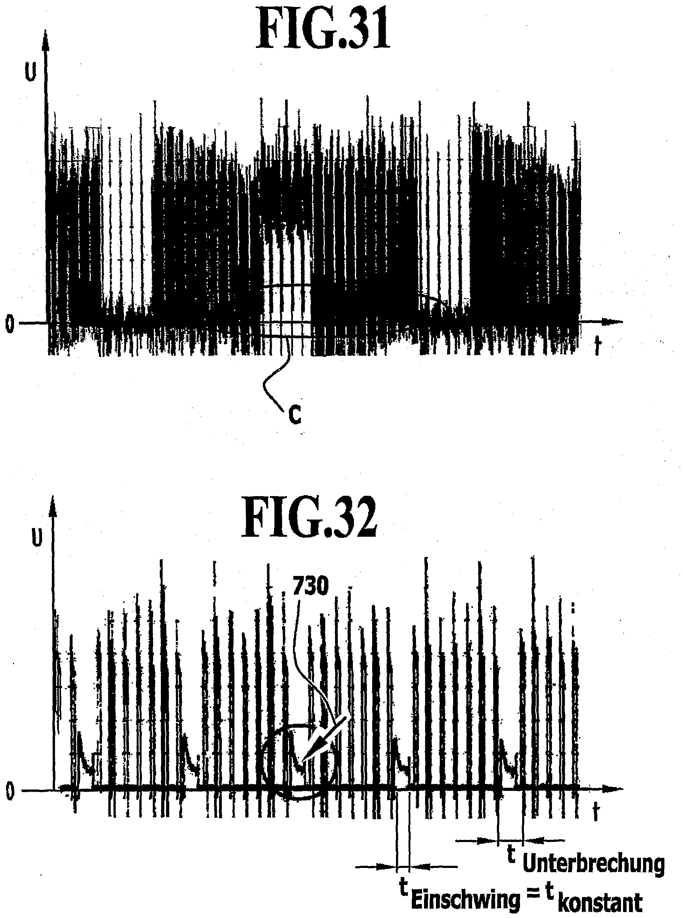

In den Figuren 31 und 32 ist ein Spannungsverlauf einer Motorwicklung bezogen auf die Masse der Batterie 722 dargestellt. Durch die Puls-Weiten-Modulation wird das Bild sehr stark verrauscht. In dem mit C gekennzeichneten Bereich ist in den Unterbrechungen der Puls-Weiten-Modulation eine sinusähnliche Gegen-EMK des Motors zur erkennen. In Figur 32 ist nochmals der Spannungsverlauf aus Figur 31, aber mit zehnfach höherer zeitlicher Auflösung, gezeigt. Man erkennt die durch Störspitzen überlagerte Puls-Weiten-Modulation.

In den bereits beschriebenen Unterbrechungen der Bestromung, also anstelle jedes achten Pulses, ist ein Spannungsverlauf der Gegen-EMK der Motorwicklung zu erkennen. Die Gegen-EMK, also die in der Motorwicklung aufgrund der Drehung des Rotors induzierte Spannung pendelt sich auf einen nach einer Einschwingzeit t_{Einschwing} in etwa konstanten Wert ein, was durch den Pfeil 730 gekennzeichnet ist. Am Ende des Einschwingvorgangs kann die Gegen-EMK gemessen werden. Dies geschieht für alle drei Motorwicklungen in gleicher Weise und gleichzeitig, so daß aus den drei ermittelten Gegen-EMK-Werten sowohl eine Ist-Drehzahl des Elektromotors 714 als auch eine Rotationsstellung des Rotors des Elektromotors 714 berechnet werden kann.

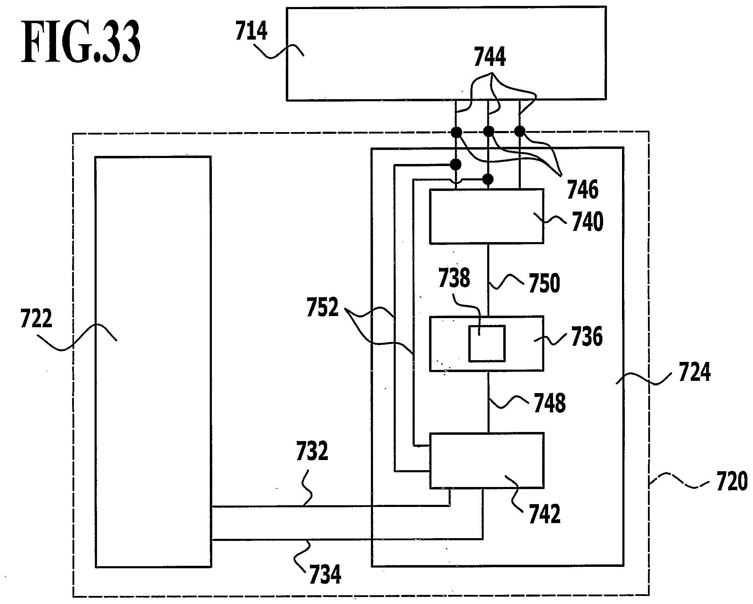

In Figur 33 ist schematisch der Aufbau des als Energie- und Steuereinheit dienenden Batteriepacks 720 dargestellt. Wie bereits eingangs erwähnt, umfaßt er die Batterie 722 sowie die Motorsteuerung 724. Die Motorsteuerung 724 umfaßt unter anderem drei wesentliche Schaltungsbestandteile, nämlich eine Prozessoreinheit 736 mit einem digitalen Signalprozessor 738, eine Leistungsstufe 740 mit den sechs Leistungstransistoren Q₁ bis Q₆ sowie eine Unterbrechungsschalteinheit 742. Über zwei Leitungen 732 und 734 ist die Unterbrechungsschalteinheit 742 mit der Batterie 722 verbunden. Ferner ist die Unterbrechungsschalteinheit mit zwei der drei Anschlußleitungen 744 des Elektromotors 714 oder beispielsweise den Verbindungskontakten 198 der Energie- und Steuereinheit 110 verbunden. Die Anschlußleitungen 744 sind über drei Kontakte oder zum Beispiel über die Motorkontakte 194, 394 oder 594 mit dem Powerpack 720 lösbar verbindbar. Ferner sind die Unterbrechungsschalteinheit 742 und die Prozessoreinheit 736 verbunden, was schematisch durch die Leitung 748 symbolisiert wird. Die Prozessoreinheit 736 ist mit der Leistungsstufe 740 verbunden, was schematisch durch die Leitung 750 symbolisiert wird.

Die Unterbrechungsschalteinheit dient dazu, den digitalen Signalprozessor 738 der Prozessoreinheit 736 von der Batterie 722 zu trennen, wenn der Powerpack 720 nicht mit dem Elektromotor 714 verbunden ist. Zu diesem Zweck ist die Unterbrechungsschalteinheit 742 über zwei Leitungen 752 mit zwei Kontaktstellen 746 des Batteriepacks 720 verbunden, die mit zwei Anschlußleitungen 744 des Elektromotors verbunden sind, wenn der Batteriepack 720 in den Handgriff 718 des Gehäuses 712 der Akkumaschine 710 eingeschoben wird. Erst nach Verbinden des Powerpacks 720 mit dem Elektromotor 714 schaltet die Unterbrechungsschalteinheit 742 die Prozessoreinheit 736 frei, das heißt, diese wird mit der Batterie 722 verbunden. Auf diese Weise wird eine Selbstentladung der Batterie 722 verhindert, denn die Prozessoreinheit 736, welche einen hohen Energieverbrauch hat, ist in einem Lagerzustand, also wenn der Batteriepack 720 nicht mit dem Elektromotor 714 verbunden ist, außer Funktion gesetzt.

Zur Bestimmung einer Ist-Drehzahl des Elektromotors 714 beziehungsweise zur Rotorpositionsdetektion können, wie eingangs beschrieben, prinzipiell Positionssensoren (nicht Teil der Erfindung) verwendet werden. Bei der vorgeschlagenen Akkumaschine 710 wird jedoch gerade auf derartige Sensoren verzichtet. Eine Bestimmung der Drehzahl und der Rotorposition kann jedoch sehr genau mit Identifikationsverfahren ermittelt werden. Beispiele hierfür sind Luenberger-Beobachter oder Kalman-Filter. Da es sich bei dem digitalen Signalprozessor 738 um einen Prozessor mit einer sehr schnellen und hohen Rechenleistung handelt, kann eine Drehzahl beziehungsweise Rotorposition sehr genau detektiert werden.

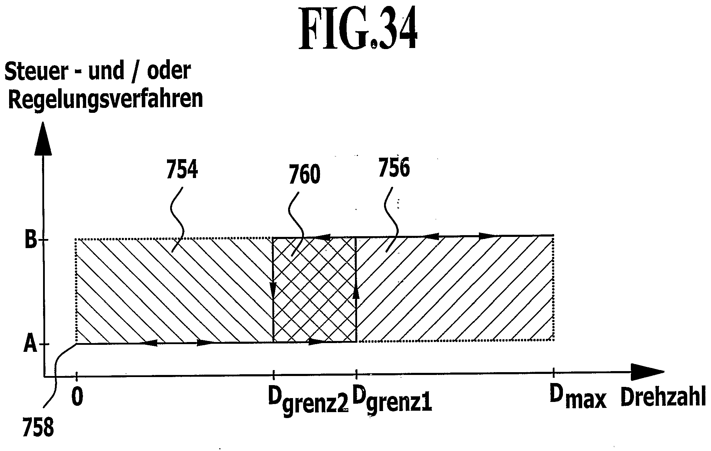

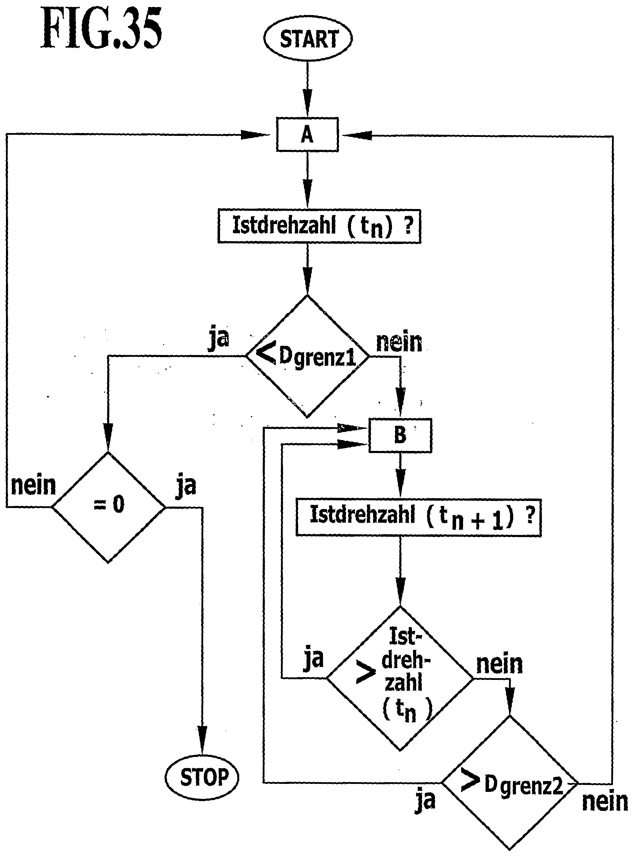

Die Motorsteuerung 724 ist derart ausgebildet, daß ein Drehzahlbereich des Elektromotors 714 in zwei Teilbereiche unterteilt wird, nämlich einen unteren Drehzahlbereich 754 und einen oberen Drehzahlbereich 756, wie dies in Figur 34 schematisch dargestellt ist. Des weiteren gestattet es die Motorsteuerung 724, zwei unterschiedliche Steuer- und/oder Regelungsverfahren zum Betreiben des Elektromotors 714 auszuführen. Dies ist zum einen ein Space-Vektor-Puls-Weiten-Modulations-(SVPWM)-Verfahren, welches in den Figuren 34 und 35 schematisch mit A bezeichnet ist. Zum anderen handelt es sich um ein herkömmliches Puls-Weiten-Moluations-(PWM)-Verfahren, welches in den Figuren 34 und 35 schematisch mit B gekennzeichnet ist.

Im Falle eines Elektromotors 714 mit einem Drehzahlerfassungssystem, welches Positionssensoren (nicht Teil der Erfindung) und Drehzahlerfassungssensoren (nicht Teil der Erfindung) aufweist, könnte das Steuer- und/oder Regelungsverfahren A auch ein Steuer- und/oder Regelungsverfahren sein, bei welchem eine Ist-Drehzahl des Elektromotors 714 mittels der Drehzahlerfassungssensoren (nicht Teil der Erfindung) ermittelt und von der Motorsteuerung 724 verarbeitet wird. Beim Space-Vektor-Puls-Weiten-Molulations-(SVPWM)-Verfahren und auch beim herkömmlichen Puls-Weiten-Modulations-(PWM)-Verfahren wird eine Ist-Drehzahl des Elektromotors 714 durch Bestimmung der Gegen-EMK ermittelt.

Mit Bezug zu den Figuren 34 und 35 wird nachfolgend die Vorgehensweise beim Umschalten vom Steuer- und/oder Regelungsverfahren A auf das Steuer- und/oder Regelungsverfahren B näher erläutert.

Betätigen des Gasdrückers 726 durch eine Bedienperson setzt die Akkumaschine 700 in Betrieb. In Figur 34 sind Start/Stop mit dem Bezugszeichen 758 versehen. Erhöht die Bedienperson die Drehzahl des Elektromotors 714, so führt die Motorsteuerung 724 das Steuer- und/oder Regelungsverfahren A aus bis zum Erreichen der Umschaltdrehzahl D_{grenz1}. Sobald die Umschaltdrehzahl D_{grenz1} erreicht ist, schaltet die Motorsteuerung 724 automatisch auf das Steuer- und/oder Regelungsverfahren B um. Bis zum Erreichen der Maximaldrehzahl Dₘₐₓ des Elektromotors 714 wird der Elektromotor 714 von der Motorsteuerung 724 im Steuer- und/oder Regelungsverfahren B betrieben. Wird die Drehzahlanforderung für den Elektromotor 714 durch die Bedienperson wieder herabgesetzt, so wird auch für Drehzahlen des Elektromotors 714, die kleiner sind als die Umschaltdrehzahl D_{grenz1} das Steuer- und/oder Regelungsverfahren B beibehalten, bis die Umschaltdrehzahl D_{grenz2} erreicht wird. Erst bei Erreichen der Umschaltdrehzahl D_{grenz2} und Unterschreiten derselben schaltet die Motorsteuerung 724 wieder auf das Steuer- und/oder Regelungsverfahren A um. Wird die Drehzahlanforderung wieder erhöht, dann erfolgt jedoch eine Umschaltung auf das Steuer- und/oder Regelungsverfahren B erst wieder nach Überschreiten der Umschaltdrehzahl D_{grenz1}.

Folge dieses Schaltschemas ist, daß in Figur 34 ein Überlappbereich zwischen dem unteren Drehzahlbereich 754 und dem oberen Drehzahlbereich 756 gebildet wird, der insgesamt mit dem Bezugszeichen 760 versehen ist. Im Überlappbereich 760 kann die Motorsteuerung 724 sowohl das Steuer- und/oder Regelungsverfahren A als auch das Steuer- und/oder Regelungsverfahren B ausführen. Welches Verfahren ausgeführt wird, hängt jedoch davon ab, ob die Drehzahlanforderung ausgehend von einer Ist-Drehzahl unterhalb der Umschaltdrehzahl D_{grenz2} erhöht oder von oberhalb der Umschaltdrehzahl D_{grenz1} abgesenkt wird. Insgesamt ergibt sich die in Figur 34 dargestellte hystereseartige Kurve, auf welcher der Überlappbereich 60 im Gegenuhrzeigersinn umwandert werden kann.

Die Funktionsweise der Motorsteuerung 724 zum Umschalten zwischen den beiden Steuer- und/oder Regelungsverfahren A und B wird anhand Figur 35 deutlich. Ausgangspunkt ist ein stillstehender Elektromotor 714. Wird dieser gestartet, so führt die Motorsteuerung 724 das Steuer- und/oder Regelungsverfahren A aus. Die Ist-Drehzahl zum Zeitpunkt tₙ wird in periodischen Abständen ermittelt. Nach Ermittlung der Ist-Drehzahl zum Zeitpunkt tₙ wird abgefragt, ob die Ist-Drehzahl kleiner als die Umschaltdrehzahl D_{grenz1}. Ist die Drehzahl kleiner als die Umschaltdrehzahl D_{grenz1}, dann wird abgefragt, ob die Drehzahl gleich 0 ist. Ist dies der Fall, dann stoppt die Motorsteuerung 724 den Betrieb des Elektromotors 714. Ist die Ist-Drehzahl kleiner als die Umschaltdrehzahl D_{grenz1}, jedoch größer als 0, dann wird weiter das Steuer- und/oder Regelungsverfahren A ausgeführt.

Ist die ermittelte Ist-Drehzahl zum Zeitpunkt tₙ größer als die Umschaltdrehzahl D_{grenz1}, dann schaltet die Motorsteuerung 724 auf das Steuer- und/oder Regelungsverfahren B um. Die Ist-Drehzahl zum Zeitpunkt tₙ₊₁ wird weiterhin in periodischen Abständen bestimmt und anschließend mit der zuvor bestimmten Ist-Drehzahl zum Zeitpunkt tₙ verglichen. Ist die Ist-Drehzahl zum Zeitpunkt tₙ₊₁ größer als die Ist-Drehzahl zum Zeitpunkt tₙ, dann führt die Motorsteuerung 724 weiterhin das Steuer- und/oder Regelungsverfahren B aus. Ist die Ist-Drehzahl zum Zeitpunkt tₙ₊₁ jedoch kleiner als die Ist-Drehzahl zum Zeitpunkt tₙ, dann wird die Ist-Drehzahl mit der Umschaltdrehzahl D_{grenz2} verglichen. Ist die Ist-Drehzahl größer als die Umschaltdrehzahl D_{grenz2} dann führt die Motorsteuerung weiterhin das Steuer- und/oder Regelungsverfahren B aus. Andernfalls schaltet die Motorsteuerung 724 automatisch auf das Steuer- und/oder Regelungsverfahren A um.

Das Umschalten zwischen den beiden Steuer- und/oder Regelungsverfahren A und B hat insbesondere den Vorteil, daß ein bei niedrigen Drehzahlen durchgeführtes Space-Vektor-Puls-Weiten-Modulations-(SVPWM)-Verfahren bei hohen Drehzahlen unerwünschte Dämpfungseffekte zeigt, wodurch sich Motorverluste ergeben und der Wirkungsgrad der Akkumaschine 700 nachteilig beeinflußt wird.

Die beiden Steuer- und/oder Regelungsverfahren A und B können in der Motorsteuerung hardware- oder softwaremäßig implementiert sein.

## Patentansprüche

1. Chirurgische Maschine (100; 300; 500; 700) mit einem eine Längsachse (104; 304; 504) definierenden Motorträger (106; 306; 506) umfassenden Gehäuse (102; 302; 502), einem im wesentlichem quer vom Motorträger (106; 306; 506) abstehenden Griff (108; 308; 508) und einem chirurgischen Antrieb (120; 320; 520), wobei der Antrieb (120; 320; 520; 714) und das Gehäuse (102; 302; 502) direkt oder indirekt lösbar verbindbar sind, wobei der Griff (108; 308; 508) einstückig an den Motorträger (106; 306; 506) angeformt ist, wobei der Motorträger (106; 306; 506) und der Griff (108; 308; 508) stirnseitig eine schlüssellochartig geformte Öffnung im Übergangsbereich vom Motorträger (106; 306; 506) zum Griff (108; 308; 508) aufweisen, welche mit einer als Rahmen dienenden, im wesentlichen schlüssellochartig geformten Platte (128; 328; 528) verschlossen ist, wobei der Antrieb (120; 320; 520) in den im wesentlichen hohlzylindrischen Motorträger (106; 306; 506) von vorne kommend, parallel zur Längsachse (104; 304; 504) eingeführt ist und wobei der Antrieb (120; 320; 520; 714) ein Elektromotor ist, **dadurch gekennzeichnet, daß** der Elektromotor (120; 320; 520; 714) ein sensorloser Motor ist.

2. Maschine nach Anspruch 1, **dadurch gekennzeichnet, daß** das Gehäuse (102; 302; 502; 712) mindestens zwei Öffnungen aufweist.

3. Maschine nach Anspruch 2, **dadurch gekennzeichnet, daß** zum Verschließen der mindestens zwei Öffnungen (112, 126; 312, 326, 375; 512, 526, 575) mindestens zwei Verschlußelemente (114, 128, 166; 328, 366; 528, 566) vorgesehen sind.

4. Maschine nach Anspruch 3, **dadurch gekennzeichnet, daß** der Rahmen (128; 328; 528) ein Verschlußelement bildet.

5. Maschine nach einem der Ansprüche 2 bis 4, **dadurch gekennzeichnet, daß** die mindestens zwei Öffnungen (112, 126; 312, 326, 375; 512, 526, 575) fluiddicht abgedichtet sind.

6. Maschine nach einem der Ansprüche 3 bis 5, **dadurch gekennzeichnet, daß** die mindestens zwei Verschlußelemente (114, 128, 166; 328, 366; 528, 566) die mindestens zwei Öffnungen (112, 126; 312, 326, 375; 512, 526, 575) fluiddicht verschließen.

7. Maschine nach einem der voranstehenden Ansprüche, **dadurch gekennzeichnet, daß** der Elektromotor (120; 320; 520; 714) ein bürstenloser Motor ist.

8. Maschine nach einem der voranstehenden Ansprüche, **dadurch gekennzeichnet, daß** der Elektromotor (120; 320; 520; 714) ein elektronisch kommutierter Gleichstrommotor ist.

9. Maschine nach einem der voranstehenden Ansprüche, **dadurch gekennzeichnet, daß** der Elektromotor (120; 320; 520; 714) ein elektronisch kommutierter bürstenloser Gleichstrommotor ist.

## Claims

1. Surgical machine (100; 300; 500; 700) comprising a housing (102; 302; 502) with a motor mount (106; 306; 506) defining a longitudinal axis (104; 304; 504), a grip (108; 308; 508) protruding substantially transversely from the motor mount (106; 306; 506) and a surgical drive (120; 320; 520; 714), wherein the drive (120; 320; 520; 714) and the housing (102; 302; 502) are directly or indirectly connectable in a detachable manner, wherein the grip (108; 308; 508) is integrally formed with the motor mount (106; 306; 506), wherein the motor mount (106; 306; 506) and the grip (108; 308; 508) comprise a front end opening of keyhole-like shape in the area of transition from the motor mount (106; 306; 506) to the grip (108; 308; 508) which is closed with a plate (128; 328; 528) of substantially keyhole-like shape serving as frame, wherein coming from the front, the drive (120; 320; 520) is inserted parallel to the longitudinal axis (104; 304; 504) into the substantially hollow-cylindrical motor mount (106; 306; 506) and wherein the drive (120; 320; 520; 714) is an electric motor **characterized in that** the electric motor (120; 320; 520; 714) is a sensorless motor.

2. Machine in accordance with claim 1, **characterized in that** the housing (102; 302; 502; 712) comprises at least two openings.

3. Machine in accordance with claim 2, **characterized in that** at least two closure elements (114, 128, 166; 328, 366; 528, 566) are provided for closing the at least two openings (112, 126; 312, 326, 375; 512, 526, 575).

4. Machine in accordance with claim 3, **characterized in that** the frame (128; 328; 528) forms a closure element.

5. Machine in accordance with claims 2 to 4, **characterized in that** the at least two openings (112, 126; 312, 326, 375; 512, 526, 575) are sealed in a fluid-tight manner.

6. Machine in accordance with claims 3 to 5, **characterized in that** the at least two closure elements (114, 128, 166; 328, 366; 528, 566) close the at least two openings (112, 126; 312, 326, 375; 512, 526, 575) in a fluid-tight manner.

7. Machine in accordance with any one of the preceding claims, **characterized in that** the electric motor (120; 320; 520; 714) is a brushless motor.

8. Machine in accordance with any one of the preceding claims, **characterized in that** the electric motor (120; 320; 520; 714) is an electronically commutated DC motor.

9. Machine in accordance with any one of the preceding claims, **characterized in that** the electric motor (120; 320; 520; 714) is an electronically commutated brushless DC motor.

## Revendications

1. Machine chirurgicale (100 ; 300 ; 500 ; 700) dotée d'un boîtier (102 ; 302 ; 502) comprenant un support de moteur (106 ; 306 ; 506) définissant un axe longitudinal (104 ; 304 ; 504), d'un manche (108 ; 308 ; 508) faisant saillie de manière essentiellement transversale depuis le support de moteur (106 ; 306 ; 506), et d'une commande chirurgicale (120 ; 320 ; 520), dans laquelle la commande (120 ; 320 ; 520 ; 714) et le boîtier (102 ; 302 ; 502) peuvent être reliés de façon amovible directement ou indirectement, dans laquelle le manche (108 ; 308 ; 508) est formé d'un seul tenant contre le support de moteur (106 ; 306 ; 506), dans laquelle le support de moteur (106 ; 306 ; 506) et le manche (108 ; 308 ; 508) présentent frontalement une ouverture en forme de trou de serrure dans la zone de transition allant du support de moteur (106 ; 306 ; 506) au manche (108 ; 308 ; 508), laquelle ouverture est fermée avec une plaque (128 ; 328 ; 528) servant de cadre et sensiblement en forme de trou de serrure, dans laquelle la commande (120 ; 320 ; 520) est engagée dans le support de moteur (106 ; 306 ; 506) essentiellement cylindrique creux en venant de l'avant, parallèlement à l'axe longitudinal (104 ; 304 ; 504), et dans laquelle la commande (120 ; 320 ; 520 ; 714) est un moteur électrique, **caractérisée en ce que** le moteur électrique (120 ; 320 ; 520 ; 714) est un moteur sans capteur.

2. Machine selon la revendication 1, **caractérisée en ce que** le boîtier (102 ; 302 ; 502 ; 712) présente au moins deux ouvertures.

3. Machine selon la revendication 2, **caractérisée en ce que** pour fermer lesdites au moins deux ouvertures (112, 126 ; 312, 326, 375 ; 512, 526, 575) sont prévus au moins deux éléments de fermeture (114, 128, 166 ; 328, 366 ; 528, 566).

4. Machine selon la revendication 3, **caractérisée en ce que** le cadre (128 ; 328 ; 528) forme un élément de fermeture.

5. Machine selon l'une quelconque des revendications 2 à 4, **caractérisée en ce que** lesdites au moins deux ouvertures (112, 126 ; 312, 326, 375 ; 512, 526, 575) sont rendues étanches aux fluides.

6. Machine selon l'une quelconque des revendications 3 à 5, **caractérisée en ce que** lesdits au moins deux éléments de fermeture (114, 128, 166 ; 328, 366 ; 528, 566) ferment lesdites au moins deux ouvertures (112, 126 ; 312, 326, 375 ; 512, 526, 575) de façon étanche.

7. Machine selon l'une quelconque des revendications précédentes, **caractérisée en ce que** le moteur électrique (120 ; 320 ; 520 ; 714) est un moteur sans balai.

8. Machine selon l'une quelconque des revendications précédentes, **caractérisée en ce que** le moteur électrique (120 ; 320 ; 520 ; 714) est un moteur à courant continu à commutation électronique.

9. Machine selon l'une quelconque des revendications précédentes, **caractérisée en ce que** le moteur électrique (120 ; 320 ; 520 ; 714) est un moteur à courant continu sans balai à commutation électronique.
